(19) Europäisches Patentamt
European Patent Office
Office européen des brevets

(11) **EP 2 540 827 A1**

## EUROPEAN PATENT APPLICATION
published in accordance with Art. 153(4) EPC

(43) Date of publication:
**02.01.2013 Bulletin 2013/01**

(21) Application number: **11747529.3**

(22) Date of filing: **25.02.2011**

(51) Int Cl.:
*C12N 15/09* [(2006.01)]    *A61K 39/395* [(2006.01)]
*A61P 35/00* [(2006.01)]    *A61P 35/02* [(2006.01)]
*C07K 16/28* [(2006.01)]    *C12Q 1/68* [(2006.01)]
*G01N 33/53* [(2006.01)]

(86) International application number:
**PCT/JP2011/054368**

(87) International publication number:
**WO 2011/105573 (01.09.2011 Gazette 2011/35)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **26.02.2010 JP 2010041547**

(71) Applicant: **Forerunner Pharma Research Co., Ltd.
Meguro-ku
Tokyo 153-0041 (JP)**

(72) Inventor: **KIMURA Naoki
Tokyo 153-0041 (JP)**

(74) Representative: **Woods, Geoffrey Corlett
J A Kemp
14 South Square
Gray's Inn
London WC1R 5JJ (GB)**

(54) **ANTI-ICAM3 ANTIBODY AND USE THEREOF**

(57)    Based on an anti-ICAM3 antibody obtained by immunizing a mouse with BaF3 cells having artificially over-expressed ICAM3, a chimeric antibody has been successfully prepared which exerts cytotoxic actions, including both a proliferation-suppressing activity and an ADCC activity, on a blood cancer cell line, and which exerts a tumor regression activity in vivo.

EP 2 540 827 A1

**Description**

[Technical Field]

The present invention relates to an anti-ICAM3 antibody and use thereof. More specifically, the present invention relates to an anti-ICAM3 antibody having a cytotoxic activity and a pharmaceutical use and other uses of the anti-ICAM3 antibody.

[Background Art]

ICAMs (interacellular adhesion molecules) are glycoproteins belonging to the immunoglobulin superfamily having sevaral extracellular immunoglobulin-like C-type domains. So far, ICAM1 to ICAM5 have been identified. ICAM3 (CD50) is a member of the ICAM family and has five immunoglobulin-like C-type domains (NPLs 1 to 3). ICAM3 has a structure very similar to those of other ICAM family molecules ICAM1 and ICAM2. While the other ICAM family molecules show a relatively ubiquitous expression distribution such as in endothelium, ICAM3 shows a unique expression pattern confined to blood cells such as lymphocytes, leukocytes, and thymocytes (NPL 1). Similarly to ICAM1 and ICAM2, ICAM3 binds to LFA-1 (lymphocyte function antigen 1; CD11a/CD18). Besides, ICAM3 binds to CD11d/CD18, and is thought to function as an accessory molecule in the immune response (NPLs 1 to 4) .

ICAM3 functions as a signaling molecule, which transmits various kinds of information from the outside into the inside of the cell. Particularly, it is reported that the cross-linking with an antibody suppresses the proliferation and induces apoptosis of some blood cells. This suggests that controlling apoptosis of blood cells in vivo is also a function of ICAM3.

For example, it is reported that an anti-ICAM3 antibody suppresses the effect of promoting T cell proliferation by PMA stimulation or the like (NPL 5). Moreover, it is reported that cross-linking ICAM3 in a human thymocyte with an anti-ICAM3 antibody induces the apoptosis while increasing intracellular calcium concentration (NPL 6). Further, it is reported that apoptosis is induced also in blood cells prepared from normal human bone marrow and leukemic cell line (U937, Jurkat) (NPL 7). Furthermore, another group has reported that anti-ICAM3 antibody induces apoptosis of granulocytes, particularly basophils and neutrophils, also (NPL 8).

However, the antibodies reported so far cannot be said to have high apoptosis-inducing activity and cell-proliferation-suppressing activity. These antibodies are therefore thought to be insufficient to exert an anti-tumor effect in vivo.

[Citation List]

[Non Patent Literature]

[NPL 1] Vazeux R et al. Nature. 1992, 360, 485-488
[NPL 2] Fawcett J et al. Nature. 1992, 360, 481-484
[NPL 3] de Fougerolles & Spronger. J. Exp. Med. 1992, 175, 185-190
[NPL 4] Van der Vieren M et al. Immunity. 1995, 3, 683-690
[NPL 5] Green J. & Thompson C. Cell Immunology. 1996. 171. 126-131
[NPL 6] Martinez-Caceres E et al. 1996. 48. 626-635
[NPL 7] Stucki A et al. Br J Haematol. 2000. 108. 157-166
[NPL 8] Kessel J et al. J Allergy Clin Immunol. 2006. 118. 831-836

[Summary of Invention]

[Technical Problem]

The present invention has been made in view of the above-described problems in the conventional techniques. An object of the present invention is to provide an antibody capable of exerting a high anti-tumor effect in vivo.

[Solution to Problem]

In order to achieve the above object, the present inventors have attempted to produce an anti-ICAM3 antibody having a high proliferation-suppressing action. First, BaF3 cells having artificially overexpressed ICAM3 were established and used for immunization of a mouse to produce an anti-ICAM3 antibody. Next, the hybridoma cells were screened for based only on cell-proliferation-suppressing activity. A monoclonal antibody IB23 having a high proliferation-suppressing activity was selected there from. The selected mouse monoclonal antibody IB23 bound to the surfaces of various cancer cell lines, but not to the family molecules having a high homology with ICAM3, thus showing ICAM3-specific binding. Further, the present inventors determined the sequences of the heavy chain variable region and light chain variable region of the mouse monoclonal antibody IB23, followed by modification to a human chimeric antibody. Then, the in vitro cytotoxic activity of this antibody was analyzed. As a result, it was revealed that the IB23 human chimeric antibody alone exhibited a high proliferation-suppressing activity on various blood cancer cell lines. Moreover, the ADCC activity was also analyzed. As a result, it was found out that the human chimeric antibody had a high ADCC activity. In other words, the IB23 antibody was revealed as an antibody which exerts cytotoxic actions, including both a proliferation-suppressing activity and an ADCC activity, on blood cancer cell lines.

Next, whether or not the IB23 antibody exhbits an in vivo tumor regression activity was analyzed. As a result, it was found out that the IB23 human chimeric antibody showed a significant tumor regression effect on a human leukemia mouse model. A tumor regression effect was also observed from a deglycosylated IB23 antibody which had lost the ADCC activity by deglycosylation. Nevertheless, the drug action of the IB23 human chimeric antibody having both a proliferation-suppressing activity and an ADCC activity was superior to that of the deglycosylated antibody. This proved that the drug action in vivo was successfully enhanced by adding an ADCC activity to a proliferation-suppressing activity of the antibody.

Specifically, the present invention relates to an anti-ICAM3 antibody having a cytotoxic activity and a pharmaceutical use and other uses of the anti-ICAM3 antibody. More specifically, the present invention provides the following inventions.

(1) An anti-ICAM3 antibody having a cytotoxic activity.
(2) The anti-ICAM3 antibody according to (1), wherein the cytotoxic activity is an ADCC activity.
(3) The anti-ICAM3 antibody according to any one of (1) and (2), which further has a cell-proliferation-suppressing activity.
(4) The anti-ICAM3 antibody according to any one of (1) to (3), which has an anti-tumor activity in vivo.
(5) The anti-ICAM3 antibody according to any one of (1) to (4), which does not substantially bind to ICAM1 and/or ICAM5.
(6) An antibody of any one of (a) to (c) below:

(a) an antibody comprising heavy chain CDR1 having an amino acid sequence of SEQ ID NO: 15, heavy chain CDR2 having an amino acid sequence of SEQ ID NO: 16, heavy chain CDR3 having an amino acid sequence of SEQ ID NO: 17, light chain CDR1 having an amino acid sequence of SEQ ID NO: 22, light chain CDR2 having an amino acid sequence of SEQ ID NO: 23, and light chain CDR3 having an amino acid sequence of SEQ ID NO: 24;
(b) an antibody of (a) in which one or more amino acids are substituted, deleted, added and/or inserted, the antibody of (b) having an activity equivalent to that of the antibody of (a); and
(c) an antibody which recognizes a same epitope as an epitope recognized by the antibody of any one of (a) and (b).

(7) The anti-ICAM3 antibody according to any one of (1) to (6), comprising a human-derived constant region.
(8) A DNA encoding the antibody according to any one of (1) to (7).
(9) A pharmaceutical composition comprising the antibody according to any one of (1) to (7) as an active ingredient.
(10) The pharmaceutical composition according to (9), which is an anti-cancer agent.
(11) The pharmaceutical composition according to (10), wherein the cancer is leukemia, myeloma, or malignant lymphoma.
(12) A diagnosis method for a cancer, characterized by comprising the step of detecting any one of an ICAM3 protein and a gene encoding the ICAM3 protein.

[Advantageous Effects of Invention]

An antibody of the present invention binds to a cell having ICAM3 expressed on the surface thereof, and shows a high cell-proliferation action and a high antibody-dependent cell-mediated cytotoxic action. Particularly, when acting on tumor cells, the antibody of the present invention demonstrates an excellent anti-tumor effect in vitro and in vivo. Thus, the antibody of the present invention makes it possible to effectively treat ICAM3-involved diseases (particularly, cancers).

[Brief Description of Drawings]

[Fig. 1] Fig. 1 is a graph showing the result of detecting the cell-proliferation-suppressing activities of mouse anti-ICAM3 antibodies on KMS-12-BM cells.

[Fig. 2] Fig. 2 is a graph showing the result of detecting the binding activity of an IB23 antibody on various blood cancer lines.

[Fig. 3] Fig.3 is graphs showing the result of detecting the binding activity of the IB23 antibody on an ICAM1-expressing BaF3 transfectant (ICAM1/BaF3) and on ICAM5-expressing BaF3 transfectant (ICAM5/BaF3).

[Fig. 4] Fig. 4 is graphs showing the result of detecting the proliferation-suppressing activity of an IB23 human chimeric antibody on various blood cancer lines.

[Fig. 5] Fig. 5 is a graph showing the result of detecting the cell-proliferation-suppressing activities of the IB23 human chimeric antibody (IB23 standard antibody) and a deglycosylated IB23 human chimeric antibody on SKM-1 cells.

[Fig. 6] Fig. 6 is graphs showing the result of detecting the ADCC activity of the IB23 human chimeric antibody (IB23 standard antibody) on an ICAM3-expressing BaF3 transfectant (ICAM3/BaF3) and various blood cancer lines.

[Fig. 7] Fig. 7 is a graph showing the result of detecting the ADCC activities of the IB23 human chimeric antibody (IB23 standard antibody) and the deglycosylated IB23 human chimeric antibody on an ICAM3-expressing BaF3 transfectant (ICAM3/BaF3).

[Fig. 8A] Fig. 8A is a graph showing the result of detecting the in vivo anti-tumor activity of the IB23 human chimeric antibody (IB23 standard antibody).

[Fig. 8B] Fig. 8B is a graph showing the result of detecting the in vivo anti-tumor activity of the deglycosylated IB23 human chimeric antibody.

[Description of Embodiments]

[ICAM3]

"ICAM3 (interacellular adhesion molecule 3)" targeted by an antibody of the present invention is a member of the ICAM family and has five immunoglobulin-like C-type domains (NPLs 1 to 3). The amino acid sequence of human ICAM3, for example, is described in GenBank Accession No. NM_002162. The amino acid sequence of human ICAM3 is shown in SEQ ID NO: 2, and the base sequence of a DNA encoding the amino acid sequence is shown in SEQ ID NO: 1. ICAM3 maybe derived not only from human but also from other mammals (for example, rat, mouse, dog, cat, cattle, horse, sheep, pig, goat, rabbit, monkey). When the antibody of the present invention is used for treatment or diagnosis of human, human ICAM3 is targeted. In addition, ICAM3 may exist in a form having a naturally mutated amino acid, besides one having a typical amino acid sequence. Thus, ICAM3 targeted by the antibody of the present invention also includes natural mutants of amino acids. ICAM3 derived from mammals other than human and mutants of ICAM3 may differ from the human ICAM3 in amino acid sequence by one or more amino acids. Normally, such ICAM3 or the mutant is a polypeptide having a homology of 70% or more with the amino acid sequence of the above human ICAM3, preferably a polypeptide having a homology of 80% or more, further preferably a polypeptide having a homology of 90% or more, and more preferably a polypeptide having a homology of 95% or more.

In the present invention, when used as an antigen for producing the antibody of the present invention, "ICAM3" may be variants having one or more amino acids altered, besides ones having a natural amino acid sequence. An example of the aforementioned variant having one or more amino acids altered in the sequence is a polypeptide having a homology of 70% or more with the above amino acid sequence, preferably a polypeptide having a homology of 80% or more, further preferably a polypeptide having a homology of 90% or more, and more preferably a polypeptide having a homology of 95% or more. Furthermore, the variant may be a partial peptide of these ICAM3. In a case where an antibody drug or a diagnostic agent targeting human is to be developed, a human ICAM3 protein is preferable.

[Anti-ICAM3 antibody]

The anti-ICAM3 antibody of the present invention may be a polyclonal antibody or a monoclonal antibody. The "polyclonal antibody" is an antibody preparation including different antibodies against different epitopes. Meanwhile, the "monoclonal antibody" means an antibody (including an antibody fragment) obtained from a substantially homogeneous antibody population. In contrast to the polyclonal antibody, the monoclonal antibody recognizes a single determinant on an antigen. The anti-ICAM3 of the present invention is preferably a monoclonal antibody. The origin, type, shape, and the like of the anti-ICAM3 antibody of the present invention are not particularly limited, as long as the anti-ICAM3 antibody binds to an ICAM3 protein. Specifically, it is possible to use antibodies such as an antibody derived from a non-human animal (for example, mouse antibody, rat antibody, camel antibody), a human antibody derived from human, a chimeric antibody, and a humanized antibody.

The anti-ICAM3 antibody of the present invention is preferably an anti-human ICAM3 antibody. The anti-human ICAM3 antibody may be an antibody specifically binding to human ICAM3, or may be an antibody binding to ICAM3 derived from other animals (for example, mouse ICAM3) besides human ICAM3.

The anti-ICAM3 antibody of the present invention may be obtained as a polyclonal antibody or a monoclonal antibody using known means. The anti-ICAM3 antibody of the present invention is particularly preferably a monoclonal antibody derived from a mammal. Examples of the monoclonal antibody derived from a mammal include ones produced by a hybridoma, ones produced by a host transformed with an expression vector containing an antibody gene by a genetic engineering technique, and the like.

The anti-ICAM3 antibody of the present invention may be modified with various molecules such as polyethylene glycol (PEG). In addition, as will be described later, the anti-ICAM3 antibody may be modified with a chemotherapy agent having a cytotoxic activity, a radioactive chemical substance, or the like.

The anti-ICAM3 antibody of the present invention is preferably an antibody having one or more activities described in (1) to (6) below.

(1) Cytotoxic Activity

In the treatment of cell proliferative diseases such as cancers, an antibody desirably retains its effector activity. Specifically, a preferable antibody in the present invention has both a binding affinity for ICAM3 and an effector function. The effector function of an antibody includes an antibody-dependent cell-mediated cytotoxicity (ADCC) activity and a complement-dependent cytotoxicity (CDC) activity. In the present invention, an antibody for treatment particularly preferably has an ADCC activity as the effector function. In a case where the antibody of the present invention is used for treatment, the antibody is preferably an antibody having a cytotoxic activity. Examples of the cytotoxic activity include an ADCC activity, a CDC activity, and the like. In the present invention, the "ADCC activity" means an activity to damage a target cell when an Fc$\gamma$ receptor-bearing cell (an immune cell or the like) binds to an Fc portion of a specific antibody through the Fc$\gamma$ receptor, the specific antibody having attached to a cell-surface antigen of the target cell. Meanwhile, the "CDC activity" means a cytotoxic activity by the complement system.

The present example demonstrated that the anti-ICAM3 antibody of the present invention even at a low concentration had an ADCC activity on cells expressing ICAM3 (Example 6). The anti-ICAM3 antibody of the present invention is preferably an antibody having an ADCC activity at 0.1 $\mu$g/ml on cells expressing ICAM3. Examples of the cells expressing ICAM3 include BaF3 cells expressing ICAM3 and various blood cancer cells (such as SKM-1 cell, U937 cell, KMS12BM cell). The ADCC activity of the anti-ICAM3 antibody used at 0.1 Hg/ml is preferably, when evaluated by percentage of calcein released as described in the present example, 5% or more (10% or more, 15% or more, 20% or more, 25% or more, 30% or more, 35% or more, 40% or more, 45% or more, 50% or more, 55% or more) by percentage of calcein released.

Whether the anti-ICAM3 antibody has an ADCC activity or a CDC activity can be determined by known methods (for example, Current protocols in Immunology, Chapter 7. Immunologic studies in humans, Editor, John E, Coligan et al., John Wiley & Sons, Inc., (1993), and the like). Specifically, first, effector cells, complement solution, and target cells are prepared.

i) Preparation of Effector Cells

Effector cells can be prepared as follows. The spleen is extracted from a CBA/N mouse or the like, and the spleen cells are separated in an RPMI1640 medium (manufactured by Invitrogen Corporation). After washing with the same medium containing 10% fetal bovine serum (FBS, manufactured by HyClone Laboratories, Inc.), the cell concentration is adjusted to $5 \times 10^6$/ml.

ii) Preparation of Complement Solution

A complement solution can be prepared from Baby Rabbit Complement (manufactured by Cedarlane Laboratories Limited) which is diluted 10-fold with a 10% FBS-containing medium (manufactured by Invitrogen Corporation).

iii) Preparation of Target Cells

Target cells can be prepared as follows. Cells expressing an ICAM3 protein are cultured together with 0.2 mCi of $^{51}$Cr-sodium chromate (manufactured by GE Healthcare Bio-Sciences Ltd.) in a 10% FBS-containing DMEM medium at 37°C for one hour so that the target cells can be radioactively labeled. As the cells expressing an ICAM3 protein, it is possible to use cells transformed using a gene encoding the ICAM3 protein, various blood cancer cells (such as SKM-1 cell, U937 cell, KMS12BM cells), and the like. After the radioactive labeling, the cells are washed with a 10% FBS-containing RPMI1640 medium three times, and the cell concentration is adjusted to $2\times10^5$/ml.

The ADCC activity or the CDC activity can be measured by methods described below. In a case where the ADCC activity is measured, the target cells and the anti-ICAM3 antibody are added each by 50 $\mu$l to a 96-well U-bottomed plate (manufactured by Becton Dickinson and Company), and are reacted on ice for 15 minutes. Then, 100 $\mu$l of the effector cells were added thereto, and cultured in a carbon dioxide gas incubator for 4 hours. The final concentration of the antibody is adjusted to 0 or 10 $\mu$g/ml. After the culturing, 100 $\mu$l of the supernatant is collected, and the radioactivity is measured with a gamma counter (COBRAII AUTO-GAMMA, MODEL D5005, manufactured by Packard Instrument Company). Using the obtained values, the cytotoxic activity (%) can be calculated according to the equation: (A-C)/(B-C)$\times$100. A represents the radioactivity (cpm) of each sample, B represents the radioactivity (cpm) of a sample to which 1%NP-40 (manufactured by nacalai tesque, Inc.) has been added, and C represents the radioactivity (cpm) of a sample containing only the target cells.

Alternatively, instead of the method using radioactive labeling ($^{51}$Cr-sodium chromate), the ADCC activity can be evaluated by measuring the fluorescent intensity (percentage of specific calcein released) using a fluorescent dye such as calcein AM (Wako Pure Chemical Industries, Ltd., 349-07201) as described in the present example. When this approach is employed, the cytotoxic activity (%) can be calculated, using the obtained values, according to the equation: (A-C)/(B-C)$\times$100. A is a fluorescent value in each sample, B is an average fluorescent value of the cells lysed and released into a medium with Nonidet P-40 having a final concentration of 1%, and C is an average fluorescent value when only the medium was added.

On the other hand, in a case where the CDC activity is measured, the target cells and the anti-ICAM3 antibody are added each by 50 $\mu$l to a 96-well bottomed-plate (manufactured by Becton Dickinson and Company), and are reacted on ice for 15 minutes. Then, 100 $\mu$l of the complement solution was added thereto, and cultured in a carbon dioxide gas incubator for 4 hours. The final concentration of the antibody is adjusted to 0 or 3 $\mu$g/ml. After the culturing, 100 $\mu$l of the supernatant is collected, and the radioactivity is measured with a gamma counter. The cytotoxic activity can be calculated in the same manner as the measurement of the ADCC activity. The cytotoxic activity can be measured using a fluorescent dye similarly to the above.

Meanwhile, when the cytotoxic activity of an antibody conjugate is measured, the target cells and an anti-ICAM3 antibody conjugate are added each by 50 $\mu$l to a 96-well bottomed-plate (manufactured by Becton Dickinson and Company), and are reacted on ice for 15 minutes. The resultant is cultured in a carbon dioxide gas incubator for 1 to 4 hours. The final concentration of the antibody is adjusted to 0 or 3 $\mu$g/ml. After the culturing, 100 $\mu$l of the supernatant is collected, and the radioactivity is measured with a gamma counter. The cytotoxic activity can be calculated in the same manner as the measurement of the ADCC activity. The cytotoxic activity can be measured using a fluorescent dye similarly to the above.

(2) Cell-Proliferation-Suppressing Activity

A preferable embodiment of the anti-ICAM3 antibody of the present invention is an antibody having a cell-proliferation-suppressing activity. For example, in order to evaluate or measure the in vitro cell-proliferation-suppressing activity, ICAM3-expressing cells are seeded into a 96-well plate at an appropriate number per well. The anti-ICAM3 antibody is added thereto, and cultured approximately for several days to one week. Then, the number of viable cells can be counted using Cell Count Reagent SF (nacalai) in accordance with the attached instruction. As the negative control, PBS or the like may be added, or a control antibody having the same isotype may be added. The ICAM3-expressing cells are not particularly limited, and ICAM3-expressing BaF3 cells or various cancer cells (for example, blood cancer cell) can be used.

The present example demonstrated that the anti-ICAM3 antibody of the present invention even at a low concentration had a proliferation-suppressing activity on cells expressing ICAM3 (Example 7). The anti-ICAM3 antibody of the present invention is preferably an antibody having a proliferation-suppressing activity at 0.06 $\mu$g/ml (60 ng/ml) on cells expressing ICAM3. Particularly preferably, the antibody demonstrates a maximum proliferation-suppressing effect at 0.06 $\mu$g/ml (60 ng/ml) on cells expressing ICAM3. Examples of the cells expressing ICAM3 include various blood cancer cells (HL60 cell, SKM-1 cell, KMS12BM cell, IM9 cell, ARH77 cell, Kijk cell, and MC/CAR cell).

(3) In vivo Anti-Tumor Activity

Another preferable embodiment of the anti-ICAM3 antibody of the present invention is an antibody having an anti-tumor activity in vivo. For example, in order to evaluate or measure the in vivo anti-tumor activity, ICAM3-expressing cancer cells are transplanted intravenously or subcutaneously into a non-human test animal. Then, from the day of the transplantation or the next day, a test antibody is intravenously or intraperitoneally administered every day or once every several days. By measuring the size of a tumor chronologically, the anti-tumor activity can be measured. As the negative control, PBS or the like may be administered, or a control antibody having the same isotype may be administered. If the sizes of tumors in the anti-ICAM3 antibody-administered group are smaller than the sizes of tumors in the negative control-administered group (although not particularly limited, for example, a ratio of tumor proliferation suppressed by 5-mg/kg administration is 20% or more, preferably 30% or more, and a ratio of tumor proliferation suppressed by 25-mg/kg administration is 30% or more, preferably 40% or more than that of the negative control-administered group), it can be determined that the antibody has an anti-tumor activity. The cancer cell is not particularly limited, but is preferably blood cancer cell (for example, leukemia cells such as SKM-1 cell). Moreover, the mouse to which the cancer cells are administered is not particularly limited, but is preferably a SCID mouse. More specifically, for example, it is possible to measure the anti-tumor activity of the antibody in vivo by the method described in Example in this application.

Further, as a preferable embodiment of the anti-ICAM3 antibody of the present invention, the anti-ICAM3 antibody alone preferably has an anti-tumor effect without requiring cross-linking with another antibody, or the like.

(4) Cross-Reaction

Another preferable embodiment of the anti-ICAM3 antibody of the present invention is an antibody which does not substantially bind to ICAM1 and/or ICAM5. Particularly preferably, the antibody binds to human ICAM3, but does not bind to human ICAM1 and/or human ICAM5. Whether or not an antibody binds to human ICAM1 and/or human ICAM5 can be checked by methods known to those skilled in the art. For example, it is possible to check by whether or not a test antibody binds to human ICAM1- or human ICAM5-expressing BaF3 cells. The sequences of human ICAM1 and human ICAM5 are known. For example, GenBank Accession No. X06990 and GenBank Accession No. U72671 can be referred to. An amino acid sequence of human ICAM1 is shown in SEQ ID NO: 4, and a DNA encoding the amino acid sequence is shown in SEQ ID NO: 3. Moreover, an amino acid sequence of human ICAM5 is shown in SEQ ID NO: 6, and a DNA encoding the amino acid sequence is shown in SEQ ID NO: 5.

(5) Internalization Activity

Moreover, the anti-ICAM3 antibody of the present invention may have an internalization activity. In the present invention, the "antibody having an internalization activity" means an antibody which is transported into the inside of a cell (into, for example, the cytoplasm, vesicle, and other organelles) when binding to ICAM3.

Whether or not an antibody has an internalization activity can be checked by methods known to those skilled in the art. For example, it is possible to check by: a method for checking whether or not a labeled substance bound to an anti-ICAM3 antibody is incorporated into a cell expressing ICAM3 by bringing the antibody into contact with the cell; a method for checking whether or not cell death is induced in an ICAM3-expressing cell by bringing an anti-ICAM3 antibody having a cytotoxic substance bound thereto into contact with the cell expressing ICAM3; or other methods.

The antibody having an internalization activity can be used as a pharmaceutical composition such as an anti-cancer agent to be described later, for example, by binding the aforementioned cytotoxic substance thereto.

(6) Apoptosis-Inducing Activity

Additionally, the anti-ICAM3 antibody of the present invention may have an apoptosis-inducing activity. Whether or not an antibody has an apoptosis-inducing activity can be checked by methods known to those skilled in the art (for example, Japanese Unexamined Patent Application Publication No. Hei 9-295999 and the like). For example, it is possible to check by methods such as detecting apoptosis by the MTS assay or flow cytometry after culturing, in the presence of a test antibody, ICAM3-expressing cells into which an ICAM3 gene has been introduced or human leukocyte cells.

The nature, shape, and the like of the anti-ICAM3 antibody of the present invention are not particularly limited, as long as the antibody binds to an ICAM3 protein. The anti-ICAM3 antibody of the present invention may be such antibodies as (a) to (f) below, for example.

(a) Conjugated Antibody

A cytotoxic substance such as a chemotherapy agent, a toxic peptide or a radioactive chemical substance may be bound to the anti-ICAM3 antibody of the present invention. Such a modified antibody (hereinafter referred to as an antibody conjugate) can be obtained by chemically modifying an obtained antibody. Note that the method for modifying an antibody has already been established in this field.

Examples of the chemotherapy agent bound to the anti-ICAM3 antibody to function the cytotoxic activity include chemotherapy agents as follow: azaribine, anastrozole, azacytidine, bleomycin, bortezomib, bryostatin-1, busulfan, camptothecin, 10-hydroxycamptothecin, carmustine, celebrex, chlorambucil, cisplatin, irinotecan, carboplatin, cladribine, cyclophosphamide, cytarabine, dacarbazine, docetaxel, dactinomycin, daunomycin glucuronide, daunorubicin, dexamethasone, diethylstilbestrol, doxorubicin, doxorubicin glucuronide, epirubicin, ethinyl estradiol, estramustine, etoposide, etoposide glucuronide, floxuridine, fludarabine, flutamide, fluorouracil, fluoxymesterone, gemcitabine, hydroxyprogesterone caproate, hydroxyurea, idarubicin, ifosamide, leucovorin, lomustine, mechlorethamine, medroxyprogesteroneacetate, megestrol acetate, melphalan, mercaptopurine, methotrexate, mitoxantrone, mithramycin, mitomycin, mitotane, phenylbutyrate, prednisone, procarbazine, paclitaxel, pentostatin, semustine, streptozocin, tamoxifen, taxanes, taxol, testosterone propionate, thalidomide, thioguanine, thiotepa, teniposide, topotecan, uracil mustard, vinblastine, vinorelbine, and vincristine.

A preferable chemotherapy agent is a low-molecular-weight chemotherapy agent. The low-molecular-weight chemotherapy agent is less likely to interfere with the function of the antibody even after bound to the antibody. In the present invention, the low-molecular-weight chemotherapy agent has a molecular weight of normally 100 to 2000, preferably 200 to 1000. All of the chemotherapy agents exemplified herein are low-molecular-weight chemotherapy agents. These chemotherapy agents in the present invention include a prodrug which is converted into an active chemotherapy agent in vivo. The activation of such a prodrug may be through enzymatic conversion or nonenzymatic conversion.

In addition, the antibody may be modified with a toxic peptide. Examples of the toxic peptide include the followings: diphtheria toxin A chain (Langone J. J., et al., Methods in Enzymology, 93, 307-308, 1983), Pseudomonas exotoxin (Nature Medicine, 2, 350-353, 1996), ricin A chain (Fulton R. J., et al., J. Biol. Chem. , 261, 5314-5319, 1986; SivamG., et al. , Cancer Res., 47, 3169-3173, 1987; Cumber A. J. et al., J. Immunol. Methods, 135, 15-24, 1990; Wawrzynczak E. J., etal., Cancer Res., 50, 7519-7562, 1990; Gheeite V., et al., J. Immunol. Methods, 142, 223-230, 1991); deglycosylated ricin A chain (Thorpe P. E., et al., Cancer Res., 47, 5924-5931, 1987); abrin A chain (Wawrzynczak E. J., et al., Br. J. Cancer, 66, 361-366, 1992; Wawrzynczak E. J., et al., Cancer Res., 50, 7519-7562, 1990; Sivam G. , et al. , Cancer Res. , 47, 3169-3173, 1987; Thorpe P. E., et al., Cancer Res., 47, 5924-5931, 1987); gelonin (SivamG., etal., Cancer Res., 47, 3169-3173, 1987; Cumber A. J. et al., J. Immunol. Methods, 135, 15-24, 1990; WawrzynczakE. J., et al., Cancer Res. , 50, 7519-7562, 1990; Bolognesi A., et al., Clin. exp. Immunol., 89, 341-346, 1992); pokeweed anti-viral protein from seeds (PAP-s) (Bolognesi A., et al., Clin. exp. Immunol., 89, 341-346, 1992); briodin (Bolognesi A., et al., Clin. exp. Immunol., 89, 341-346, 1992); saporin (Bolognesi A., et al., Clin. exp. Immunol., 89, 341-346, 1992); momordin (Cumber A. J., et al., J. Immunol. Methods, 135, 15-24, 1990; Wawrzynczak E. J., et al. , Cancer Res., 50, 7519-7562, 1990; Bolognesi A., et al., Clin. exp. Immunol., 89, 341-346, 1992); momorcochin (Bolognesi A., et al., Clin. exp. Immunol., 89, 341-346, 1992); dianthin 32 (Bolognesi A., et al., Clin. exp. Immunol., 89, 341-346, 1992); dianthin 30 (Stirpe F., Barbieri L., FEBS letter 195, 1-8, 1986) ; Modeccin (Stirpe F., Barbieri L., FEBS letter 195, 1-8, 1986); viscumin (Stirpe F., Barbieri L., FEBS letter 195, 1-8, 1986); volkesin (Stirpe F., Barbieri L., FEBS letter 195, 1-8, 1986); dodecandrin (Stirpe F., Barbieri L., FEBS letter 195, 1-8, 1986) ; tritin (Stirpe F., Barbieri L. , FEBS letter 195, 1-8, 1986) ; luffin (Stirpe F., Barbieri L., FEBS letter 195, 1-8, 1986) ; and trichokirin (Casellas P., et al., Eur. J. Biochem. 176, 581-588, 1988; Bolognesi A. , et al., Clin. exp. Immunol., 89, 341-346, 1992).

In the present invention, a radioactive chemical substance refers to a chemical substance including a radioisotope. The radioisotope is not particularly limited, and any radioisotope may be used. For example, $^{32}$P, $^{14}$C, $^{125}$I, $^{3}$H, $^{131}$I, $^{186}$Re, $^{188}$Re, or the like can be used.

Moreover, in another embodiment, one of or two or more of the low-molecular-weight chemotherapy agent and the toxic peptide can be used in combination for modification of the antibody. In the binding of the anti-ICAM3 antibody and the low-molecular-weight chemotherapy agent, covalent bonding or non-covalent bonding can be utilized. Methods for producing antibodies to which these chemotherapy agents are bound are known.

The proteinaceous drug or toxin can be bound to the antibody according to a genetic engineering procedure. Specifically, for example, a recombinant vector can be constructed from an expression vector in which a DNA encoding the toxic peptide and a DNA encoding the anti-ICAM3 antibody are fused in-frame and incorporated. The vector is then introduced into an appropriate host cell to obtain transformed cells which are then cultured. The incorporated DNAs are expressed, and thus an anti-ICAM3 antibody to which the toxic peptide is bound can be obtained as a fusion protein. When a fusion protein with the antibody is to be obtained, generally, the proteinaceous drug or toxin is located at the C-terminal side of the antibody. A peptide linker may also be added between the antibody and the proteinaceous drug or toxin.

(b) Bispecific Antibody

The anti-ICAM3 antibody of the present invention may be a bispecific antibody. The bispecific antibody refers to an antibody having variable regions for recognizing different epitopes in the same antibody molecule. In the presentinvention,thebispecificantibody may haveantigen binding sites for recognizing different epitopes on an ICAM3 molecule. Such a bispecific antibody enables two antibody molecules to bind to one ICAM3 molecule. As a result, a higher cytotoxic action can be expected.

Alternatively, the bispecific antibody may have one of the antigen binding sites recognizing ICAM3, and the other antigen binding site recognizing a cytotoxic substance. Specifically, the cytotoxic substance includes a chemotherapy agent, a toxic peptide, a radioactive chemical substance, or the like. Such a bispecific antibody binds to a cell expressing ICAM3, while trapping the cytotoxic substance. As a result, it is possible to make the cytotoxic substance directly act on ICAM3-expressing cells. In other words, the bispecific antibody recognizing a cytotoxic substance can specifically damage tumor cells and suppress proliferation of the tumor cells.

Further, in the present invention, it is also possible to use a bispecific antibody combined with an antigen binding site for recognizing an antigen other than ICAM3. For example, it is possible to produce a bispecific antibody combined with such an antigen binding site as to recognize an antigen which is different from ICAM3, and which is specifically expressed on the cell surface of a cancer cell targeted similarly to ICAM3.

The method for producing the bispecific antibody is known. For example, a bispecific antibody can be produced by combining two types of antibody which recognize different antigens. Each of the antibodies to be combined may be a 1/2 molecule having a heavy chain and a light chain, or may be a 1/4 molecule consisting only of a heavy chain. Alternatively, a bispecific antibody-producing fusion cell can be produced by fusing hybridomas that produce different monoclonal antibodies from each other. Further, a bispecific antibody can be produced by a genetic engineering technique.

Known means can be used to measure the antigen binding activity of the antibody (Antibodies A Laboratory Manual. Ed Harlow, David Lane, Cold Spring Harbor Laboratory, 1988). For example, ELISA (enzyme-linked immunosorbent assay), EIA (enzyme immunoassay), RIA (radioimmunoassay), fluorescence immunoassay, or the like can be used.

(c) Sugar Chain-Modified Antibody

The anti-ICAM3 antibody of the present invention may be an antibody having a modified sugar chain. It is known that modifying a sugar chain of an antibody can enhance the cytotoxic activity of the antibody. Known examples as the antibody having a modified sugar chain include an antibody having a modified sugar chain (International Publication No. WO99/5434 and the like), an antibody deficient in fucose that is to be added to a sugar chain (International Publication Nos. WO00/61739, WO02/31140, and the like), an antibody having a sugar chain with bisecting GlcNAc (International Publication No. WO02/79255, and the like), and the like.

(d) Gene Recombinant Anti-ICAM3 Antibody

In a case where the antibody is to be administered to human, the antibody may be a gene recombinant antibody that is artificially modified so as to reduce the immunogenicity to human, or other purposes. Examples of the gene recombinant antibody include a chimeric antibody, a humanized antibody, and the like. These modified antibodies can be produced by known methods.

i) Chimeric Antibody

A chimeric antibody refers to an antibody in which variable regions and constant regions derived from different origins are linked to each other. For example, an antibody composed of variable regions of a heavy chain and a light chain of a mouse antibody and constant regions of a heavy chain and a light chain of a human antibody is a mouse-human-heterochimeric antibody. A recombinant vector which expresses a chimeric antibody can be produced from an expression vector in which DNAs encoding variable regions of a mouse antibody are ligated to DNAs encoding constant regions of a human antibody and incorporated. It is possible to obtain the chimeric antibody which is produced during culturing of recombinant cells transformed by the vector to thereby express the incorporated DNAs.

As the constant regions of the chimeric antibody, normally, those of a human antibody are used. For example, Cγ1, Cγ2, Cγ3, Cγ4, Cμ, Cδ, Cα1, Cα2, and Cε can be used as the constant region of the heavy chain. Moreover, Cκ and Cλ can be used as the constant region of the light chain. The amino acid sequence of these constant regions and the base sequences encoding these amino acid sequences are known. To improve the stability of the antibody itself or the stability of producing the antibody, one or more amino acids in the constant regions of the human antibody may be substituted, deleted, added and/or inserted.

ii) Humanized Antibody

Generally, a chimeric antibody includes variable regions of an antibody derived from an animal other than human and constant regions derived from a human antibody, while a humanized antibody includes complementarity determining regions (CDRs) of an antibody derived from an animal other than human, framework regions (FRs) derived from a human antibody, and constant regions derived from a human antibody. The humanized antibody is also called a reshaped human antibody. Specifically, there is known, for example, a humanized antibody obtained by grafting the CDRs of an antibody from an animal other than human, for example, mouse, into a human antibody. Since having a low immunogenicity in a human body, a humanized antibody is useful as an active ingredient of a therapeutic agent of the present invention.

A variable region of an antibody is normally composed of three CDRs flanked by four FRs. CDRs are regions substantially determining the binding specificity of an antibody. The amino acid sequence of a CDR is rich in diversity. Meanwhile, the amino acid sequence of an FR often shows a high homology even among antibodies having different binding specificities. For this reason, generally it is said that grafting CDRs enables transfer of the binding specificity of a certain antibody to another antibody.

General gene recombination techniques to obtain a humanized antibody are also known. Specifically, as a method for grafting a CDR of a mouse antibody to a human FR, for example, overlap extension PCR is known. In overlap extension PCR, a primer to which the base sequence encoding a CDR of a mouse antibody to be grafted is added is used as a primer for synthesizing an FR of a human antibody. The primer is prepared for each of four FRs. Generally, in grafting a mouse CDR to a human FR, it is said that selecting a human FR having a high homology with a mouse FR is advantageous to retain the function of the CDR. In other words, generally, it is preferable to use a human FR whose amino acid sequence has a high homology with the amino acid sequence of an FR adjacent to a mouse CDR to be grafted.

Moreover, base sequences to be ligated are designed to be connected to each other in-frame. Human FRs are individually synthesized by primers, respectively. As a result, a product is obtained in which DNAs encoding mouse CDRs are added to the FRs. In the product, the base sequences encoding mouse CDRs are designed to overlap each other. Subsequently, overlapped CDR portions anneal to each other for the complementary strand synthesis reaction. By this reaction, the human FRs are ligated to each other with the sequences of the mouse CDRs in between.

A full-length gene of the variable region in which three CDRs and the four FRs are ligated finally is amplified with primers which anneal to the 5' end and the 3' end, and to which an appropriate restriction enzyme recognition sequence is added. The DNA obtained as described above and a DNA encoding a human antibody constant region are inserted into an expression vector in such a manner as to be fused in-frame. Thereby, an expression vector for humanized antibody can be created. This vector is introduced into a host to establish recombinant cells. Then, the recombinant cells are cultured and the DNAs encoding the humanized antibody are expressed. Thus, the humanized antibody is produced in a culture of the cultured cells (see European Patent Application Publication No. 239400 and International Publication No. WO96/02576).

By qualitatively or quantitatively measuring and evaluating the binding activity of the humanized antibody produced as described above to an antigen, it is possible to suitably select such human antibody FRs that enable CDRs to form a favorable antigen binding site when the FRs are ligated with the CDRs in between. As necessary, amino acid residues of FRs may be substituted so that CDRs of the humanized antibody can form an appropriate antigen binding site. For example, it is possible to introduce a mutation into the amino acid sequence of an FR by applying the PCR used in grafting the mouse CDRs to the human FRs. Specifically, a partial base sequence mutation can be introduced into a primer annealing to an FR. The base sequence mutation is introduced in an FR synthesized by such a primer. By measuring and evaluating the binding activity of the mutant antibody with the substituted amino acid to an antigen by the above-described method, it is possible to select a mutated FR sequence having a desired characteristic (Sato, K.et al., Cancer Res, 1993, 53, 851-856).

(e) Polyvalent Antibody

As long as binding to an ICAM3 protein, the antibody of the present invention includes not only a bivalent antibody represented by an IgG (such as IgG1, IgG2, IgG4), but also a monovalent antibody or a polyvalent antibody represented by IgM. The polyvalent antibody of the present invention includes a polyvalent antibody having antigen binding sites that are all the same, or a polyvalent antibody having antigen binding sites that are partially or all different from each other.

(f) Low-Molecular-Weight Antibody

The antibody of the present invention is not limited to a full-length molecule of the antibody, and may be a low-molecular-weight antibody or a modified product thereof, as long as binding to an ICAM3 protein.

The low-molecular-weight antibody includes an antibody fragment missing a part of a whole antibody (for example, whole IgG, or the like). As long as having a binding ability to an ICAM3 antigen, an antibody molecule may be partially deleted. The antibody fragment in the present invention preferably includes any or both of a heavy chain variable region (VH) and a light chain variable region (VL). Moreover, the antibody fragment in the present invention preferably includes a CDR. The number of CDRs the antibody fragment of the present invention includes is not particularly limited, but the antibody fragment of the present invention preferably includes at least six: heavy chain CDR1, CDR2, CDR3, and light chain CDR1, CDR2, CDR3.

The amino acid sequence of VH or VL may include substitution, deletion, addition and/or insertion. Further, as long as the binding ability to an ICAM3 antigen is retained, any of VH and VL or part of both may be deleted. Moreover, the variable region may be chimeric or humanized. Specific examples of the antibody fragment include Fab, Fab', F(ab') 2, Fv, and the like. Moreover, specific examples of the low-molecular-weight antibody include Fab, Fab', F(ab')2, Fv, scFv (single chain Fv), diabody, sc (Fv) 2 (single chain (Fv)2), scFv-Fc, and the like. In the present invention, a preferable low-molecular-weight antibody is a diabody or sc(Fv)2. The low-molecular-weight antibody of the present invention also includes multimers (for example, dimer, trimer, tetramer, polymer) of these antibodies.

A fragment of the antibody can be obtained by treating the antibody with an enzyme to produce antibody fragments. The digestive enzyme cleaves the antibody fragment at a specific position, providing the antibody fragment with a specific structure. As the enzyme for producing an antibody fragment, for example, papain, pepsin, plasmin, or the like is known. By papain digestion, F(ab)2 or Fab is produced. By pepsin digestion, F(ab')2 or Fab' is produced. Alternatively, these antibody fragments can be expressed in an appropriate host cell after a gene encoding the antibody fragments is constructed and introduced into an expression vector (see, for example, Co, M. S. et al., J. Immunol. (1994) 152, 2968-2976, Better, M. & Horwitz, A. H. Methods in Enzymology (1989) 178, 476-496, Plueckthun, A. & Skerra, A. Methods in Enzymology (1989) 178, 497-515, Lamoyi, E., Methods in Enzymology (1986) 121, 652-663, Rousseaux, J. et al., Methods in Enzymology (1986) 121, 663-669, Bird, R. E. et al., TIBTECH (1991) 9, 132-137).

Any part of the antibody can be deleted from the enzymatically-obtained antibody fragment by using a genetic engineering technique. The low-molecular-weight antibody in the present invention may be an antibody fragment having any region deleted, as long as having the binding affinity to ICAM3.

i) Diabody

A diabody refers to a bivalent antibody fragment constructed by gene fusion (Holliger P et al., Proc. Natl. Acad. Sci. USA 90: 6444-6448 (1993), European Patent Application Publication No. 404,097, International Publication No. WO93/11161, and the like). A diabody is a dimer formed from two polypeptide chains. Normally, in each of the polypeptide chains forming a dimer, a heavy chain variable region and a light chain variable region are connected to each other on the same chain by a linker. The linker in a diabody is generally so short that the heavy chain variable region and light chain variable region cannot be connected to each other. Specifically, the amino acid residue forming the linker is, for example, approximately 5 residues long. Hence, the heavy chain variable region and the light chain variable region encoded on the same polypeptide chain cannot form a single-chain variable region fragment, but form a dimer together with a different single-chain variable region fragment. As a result, a diabody has two antigen binding sites.

ii) ScFv

An scFv obtained by linking together a heavy chain variable region and a light chain variable region of an antibody. In an scFv, the heavy chain variable region and the light chain variable region are linked by a linker, preferably a peptide linker (Huston, J. S. et al., Proc. Natl. Acad. Sci. U.S.A, 1988, 85, 5879-5883). The heavy chain variable region and the light chain variable region in an scFv may be derived from any antibody described in this description. The peptide linker linking the variable regions is not particularly limited. For example, any single-chain peptide made of approximately 3 to 25 residues can be used as the linker. Specifically, for example, peptide linkers to be described below or the like can be used.

The variable regions of both chains can be linked, for example, by PCR. To link the variable regions by PCR, among a DNA sequence encoding a heavy chain or a heavy chain variable region of an antibody, and a DNA sequence encoding a light chain or a light chain variable region of an antibody, a DNA encoding all or a desired part of an amino acid sequence is first used as a template.

The DNAs encoding the heavy chain and light chain variable regions are amplified by PCR using a pair of primers having sequences corresponding to the sequences of both ends of the DNA to be amplified. Next, a DNA encoding a peptide linker part is prepared. The DNA encoding the peptide linker can be synthesized using PCR, also. To the 5' side of the primer used in this event, a base sequence that can be ligated to an amplified product of each variable region synthesized separately is added. Next, a PCR reaction is carried out using DNAs of [heavy chain variable region DNA]-[peptide linker DNA]-[light chain variable region DNA] and primers for assembly PCR.

The primers for assembly PCR are a combination of a primer annealing to the 5' side of the [heavy chain variable region DNA] and a primer annealing to the 3' side of the [light chain variable region DNA]. In other words, the primers for assembly PCR are a primer set that can amplify the DNAs encoding the full-length sequence of the scFv to be synthesized. Meanwhile, base sequences that can be ligated to the DNAs of the variable regions are added to the [peptide linker DNA]. As a result, these DNAs are ligated. Furthermore, the full-length scFv is finally produced as amplified products by the primers for assembly PCR. Once the DNAs encoding the scFv are produced, an expression vector containing these and recombinant cells transformed with the expression vector can be obtained in accordance with conventional processes. In addition, the scFv can be obtained by culturing the resulting recombinant cells and expressing the DNAs encoding the scFv.

iii) ScFv-Fc

An scFv-Fc is a low-molecular-weight antibody obtained by fusing an Fc region to an scFv (Cellular & Molecular Immunology 2006; 3: 439-443). The origin of the scFv used in an scFv-Fc is not particularly limited. For example, an scFv derived from IgM can be used. Moreover, the origin of the Fc is not particularly limited. For example, a human IgG (such as human IgG1) can be used. Thus, an example of a preferable embodiment of the scFv-Fc is an scFv-Fc in which a scFv fragment of an IgM antibody is linked to CH2 (for example, C$\gamma$2) and CH3 (for example, C$\gamma$3) of human IgG1 by a hinge region (H$\gamma$) of human IgG1.

iv) Sc(Fv)2

An sc (Fv) 2 is a single-stranded low-molecular-weight antibody obtained by linking two heavy chain variable regions (VH) and two light chain variable regions (VL) by a linker or the like (Hudson et al., J Immunol. Methods 1999; 231: 177-189). An sc(Fv)2 can be produced by, for example, linking scFvs by a linker. To link four antibody variable regions, normally three linkers are needed.
In addition, a preferable antibody is characterized in that two VH and two VL are aligned in the order of VH, VL, VH, VL ([VH]-linker-[VL]-linker-[VH]-linker-[VL]) with the N-terminal side of a single-stranded polypeptide as the starting point.
The order of two VH and two VL is not particularly limited to the above configuration, and the regions may be aligned in any order. Examples of the order include the followings.

[VL]-linker-[VH]-linker-[VH]-linker-[VL]
[VH]-linker-[VL]-linker-[VL]-linker-[VH]
[VH]-linker-[VH]-linker-[VL]-linker-[VL]
[VL]-linker-[VL]-linker-[VH]-linker-[VH]
[VL]-linker-[VH]-linker-[VL]-linker-[VH]

As the linker linking variable regions of the antibody, it is possible to use any peptide linker that can be introduced by genetic engineering, linkers disclosed as a synthetic compound linker (see, for example, Protein Engineering, 9 (3), 299-305, 1996), and the like. The multiple linkers may be the same, or different linkers may be used. In the present invention, a peptide linker is preferable. The length of the peptide linker is not particularly limited, and can be selected as appropriate by those skilled in the art, depending on the purpose. Normally, the number of amino acid residues making up the peptide linker is 1 to 100 amino acids, preferably 3 to 50 amino acids, further preferably 5 to 30 amino acids, and particularly preferably 12 to 18 amino acids (for example, 15 amino acids).
The amino acid sequence of the peptide linker may be any sequence, as long as the binding action of the scFv is not inhibited. The amino acid sequence of the peptide linker can be selected as appropriate by those skilled in the art, depending on the purpose.
Hence, in the present invention, as a particularly preferable embodiment of the sc(Fv)2, for example, the following sc(Fv)2 can be cited:

```
[VH]-peptide linker (15 amino acids)-[VL]-peptide linker
(15  amino  acids)-[VH]-peptide  linker  (15  amino
acids)-[VL].
```

Alternatively, the variable regions can be linked using a synthetic chemical linker (chemical crosslinking agent). Crosslinking agents normally used for crosslinking peptide compounds or the like can be used in the present invention. For example, chemical crosslinking agents as follow are known. These crosslinking agents are commercially available:

N-hydroxysuccinimide (NHS), disuccinimidyl suberate (DSS), bis(sulfosuccinimidyl)suberate (BS3), dithiobis(succinimidyl propionate) (DSP), dithiobis(sulfosuccinimidyl propionate) (DTSSP), ethylene glycol bis(succinimidyl succinate) (EGS), ethylene glycol bis(sulfosuccinimidyl succinate) (sulfo-EGS), disuccinimidyl tartrate (DST), disulfosuccinimidyl tartrate (sulfo-DST), bis[2-(succinimidooxycarbonyloxy)ethyl]sulfone (BSOCOES), bis[2-(sulfosuccinimidooxycarbonyloxy)ethyl]sulfone (sulfo-BSOCOES), and the like.

Examples of the antibody which recognize and binds to ICAM3, and which is used in the present invention, include antibodies below:

(a) an antibody comprising heavy chain CDR1 having an amino acid sequence of SEQ ID NO: 15, heavy chain CDR2 having an amino acid sequence of SEQ ID NO: 16, heavy chain CDR3 having an amino acid sequence of SEQ ID NO: 17, light chain CDR1 having an amino acid sequence of SEQ ID NO: 22, light chain CDR2 having an amino acid sequence of SEQ ID NO: 23, and light chain CDR3 having an amino acid sequence of SEQ ID NO: 24;
(b) an antibody of (a) in which one or more amino acids are substituted, deleted, added and/or inserted, the antibody of (b) having an activity equivalent to that of the antibody of (a); and
(c) an antibody which recognizes a same epitope as an epitope recognized by the antibody of any one of (a) and (b).

In a case where the above-described antibodies comprise a constant region, the constant region to be used is not particularly limited, and any constant region may be used. An example of a preferable constant region used in the present invention is a human-derived constant region. For example, as a heavy chain constant region, it is possible to use a constant region derived from human IgG1, a constant region derived from human IgG2, a constant region derived from human IgG3, a constant region derived from human IgG4, or the like. Moreover, for example, as a light chain constant region, it is possible to use a constant region derived from human κ chain, a constant region derived from human λ chain, or the like. The amino acid sequence of the heavy chain constant region derived from human IgG1 is shown in SEQ ID NO: 26, and the base sequence of a DNA encoding the amino acid sequence is shown in SEQ ID NO: 25. Moreover, the amino acid sequence of the light chain constant region derived from human IgG1 is shown in SEQ ID NO: 28, and the base sequence of a DNA encoding the amino acid sequence is shown in SEQ 10 NO: 27. The constant region used in the present invention may be a constant region having a natural sequence, or a variant of a constant region having a natural sequence in which one or more amino acids are modified.

In a case where the above-described antibody comprise an FR, the FR to be used is not particularly limited, and any FR may be used, as long as the binding activity to human ICAM3 is retained. Examples of the FR include FR1 to FR4 of a heavy chain variable region represented by amino acid sequences of SEQ ID NOs: 11 to 14, and FR1 to FR4 of a light chain variable region represented by amino acid sequences of SEQ ID NOs: 18 to 21. A preferable example of the FR used in the present invention is an FR derived from a human antibody. There is known a technique of substituting FRs while retaining the binding activity of an antibody against an antigen; accordingly, those skilled in the art can select an FR as appropriate. The FR used in the present invention may be an FR having a natural sequence, or an FR having a natural sequence in which one or more amino acids are modified.

An example of the antibody comprising heavy chain CDR1 having an amino acid sequence of SEQ ID NO: 15, heavy chain CDR2 having an amino acid sequence of SEQ ID NO: 16, heavy chain CDR3 having an amino acid sequence of SEQ ID NO: 17, light chain CDR1 having an amino acid sequence of SEQ ID NO: 22, light chain CDR2 having an amino acid sequence of SEQ ID NO: 23, and light chain CDR3 having an amino acid sequence of SEQ ID NO: 24 is an antibody comprising, as variable regions, a heavy chain variable region having an amino acid sequence of SEQ ID NO: 8 (IB23 heavy chain variable region) and a light chain variable region having an amino acid sequence of SEQ ID NO: 10 (IB23 light chain variable region). In a case where the antibody is a chimeric antibody, the antibody may comprise, as constant regions, the human-IgG heavy chain constant region described in SEQ ID NO: 26 and the human-IgG light chain constant region described in SEQ ID NO: 28.

In the present invention, having an activity equivalent to that of the antibody of the present invention refers to being equivalent in binding activity to ICAM3, cell-proliferation-suppressing activity, cell death-inducing activity, and cytotoxic activity (such as ADCC activity) on cells expressing ICAM3, and/or anti-tumor activity. In the present invention, to be equivalent in an activity, the activity does not necessary have to be identical. For example, such activities only need to be 50% or more, preferably 70% or more, and further preferably 90% or more, of the activities of the above-described antibodies. The upper limit of the activities is not particularly limited, and examples thereof include 1000% or less, 500% or less, 300% or less, 150% or less, 100% or less, and the like.

The antibody of the present invention in which one or more amino acids are substituted, deleted, added and/or inserted is also within the scope of the present invention. Such an antibody may be artificially produced, or naturally occur. An example of the method for introducing a mutation into a polypeptide is site-directed mutagenesis (Hashimoto-Gotoh, T. et al. (1995) Gene 152, 271-275, Zoller, MJ, and Smith, M. (1983) Methods Enzymol. 100, 468-500, Kramer, W. et al. (1984) Nucleic Acids Res. 12, 9441-9456, Kramer W, and Fritz HJ (1987) Methods. Enzymol. 154, 350-367, Kunkel, TA (1985) Proc Natl Acad Sci USA. 82, 488-492, Kunkel (1988) Methods Enzymol. 85, 2763-2766) or the like. This is one of methods well known to those skilled in the art to prepare a polypeptide functionally equivalent to a certain polypeptide. Those skilled in the art could prepare an antibody functionally equivalent to the antibody of the present invention by introducing a mutation into the antibody as appropriate using such a method. Meanwhile, such a mutation in an amino acid may naturally occur. In this manner, the antibody of the present invention also includes an antibody comprising an amino acid sequence of the antibody of the present invention in which one or more amino acids are mutated, the antibody being functionally equivalent to the antibody.

The number of amino acids mutated in such a mutant is normally 50 amino acids or less, preferably 30 amino acids or less, and further preferably 10 amino acids or less (for example, 5 amino acids or less).

The properties of an amino acid side chain of an amino acid residue to be mutated are desirably conserved in another amino acid thus mutated. For example, based on the properties of the amino acid side chain, categories as follow have been established:

hydrophobic amino acids (A, I, L, M, F, P, W, Y, V); hydrophilic amino acids (R, D, N, C, E, Q, G, H, K, S, T); amino acids having an aliphatic side chain (G, A, V, L, I, P) ; amino acids having a hydroxyl group-containing side chain (S, T, Y) ; amino acids having a sulfur atom-containing side chain (C, M); amino acids having a carboxylic acid- and amide-containing side chain (D, N, E, Q); amino acids having a base-containing side chain (R, K, H); and amino acids having an aromatic group-containing side chain (H, F, Y, W) (all of the amino acids are represented by single-letter codes in parentheses).

It has already been known that a polypeptide having an amino acid sequence which is modified from a certain amino acid sequence by deletion and/or addition of one or more amino acid residues and/or substitution with another amino acid retains the biological activity of the original polypeptide (Mark, D. F. et al., Proc. Natl. Acad. Sci. USA (1984) 81, 5662-5666, Zoller, M. J. and Smith, M., Nucleic Acids Research (1982) 10, 6487-6500, Wang, A. et al., Science 224, 1431-1433, Dalbadie-McFarland, G. etal., Proc. Natl. Acad. Sci. USA (1982) 79, 6409-6413). Specifically, generally, it is said that when an amino acid in the amino acid sequence of a certain polypeptide is substituted with an amino acid within the same category group, the activity of the polypeptide is highly likely to be retained. In the present invention, a substitution between amino acids within the same amino acid group described above refers to a conservative substitution.

The present invention also provides a peptide comprising a heavy chain or a light chain of an antibody comprising a CDR identified in the present invention, or a variable region of these chains. A preferable peptide is a peptide comprising the heavy chain of the antibody of the present invention having the amino acid sequences of SEQ ID NOs: 15 to 17, or a variable region thereof, and particularly preferably is a peptide comprising the amino acid sequence of SEQ ID NO: 8. Another preferable peptide is a peptide comprising the light chain of the antibody of the present invention having the amino acid sequences of SEQ ID NOs: 22 to 24, or a variable region thereof, and particularly preferably, a peptide comprising the amino acid sequence of SEQ ID NO: 10. By linking these peptides with, for example, a linker or the like, a functional antibody can be produced.

Moreover, the present invention also provides an antibody which binds to a same epitope as an epitope the above-described antibody binds to.

Whether or not a test antibody shares an epitope with a certain antibody can be checked by competition for the same epitope between the two. The competition between antibodies is detected by a cross-blocking assay or the like. For example, competitive ELISA assay is preferably a cross-blocking assay.

Specifically, in the cross-blocking assay, after preincubation of microtiter plate wells coated with an ICAM3 protein in the presence or absence of a candidate competing antibody, the anti-ICAM3 antibody of the present invention is added thereto. The amount of the anti-ICAM3 antibody of the present invention bound to the ICAM3 protein in the wells correlates indirectly with the binding ability of the candidate competing antibody (test antibody) that competes in binding to the same epitope. In other words, if the affinity of the test antibody to the same epitope increases, the amount of the anti-ICAM3 antibody of the present invention bound to the wells coated with the ICAM3 protein is decreased; meanwhile, the amount of the test antibody bound to the wells coated with the ICAM3 protein is increased.

The amount of an antibody bound to wells can be easily measured by labeling the antibody in advance. For example, a biotin-labeled antibody can be measured by using an avidin-peroxidase conjugate and an appropriate substrate. A cross-blocking assay using an enzyme label such as peroxidase particularly refers to a competitive ELISA assay. An antibody can be labeled with other labeled substances that can be detected or measured. Specifically, radiolabels, fluorescent labels, or the like are known.

Further, when a test antibody has a constant region derived from a species different from the anti-ICAM3 antibody of the present invention, an antibody bound to wells can be measured with a certain labeled antibody that recognizes the constant region. Alternatively, even when an antibody is derived from the same species but a different class, an antibody bound to wells can be measured with an antibody that can distinguish classes.

If a candidate antibody can block binding of the anti-ICAM3 antibody at least 20%, preferably at least 30%, further preferably at least 50%, and more preferably at least 80% in comparison with the binding activity obtained in a control test conducted in the absence of the competing antibody, the candidate antibody is an antibody which binds to substantially the same epitope as that of the anti-ICAM3 antibody of the present invention, or which competes in binding to the same epitope.

The antibody of the present invention is capable of exerting high cell-proliferation-suppressing activity, cell death-inducing activity, cytotoxic activity (for example, ADCC activity), and/or anti-tumor activity, and is therefore useful as a drug, particularly an anti-cancer agent.

[Production of Anti-ICAM3 Antibody]

1. Production of Anti-ICAM3 Antibody by Monoclonal Antibody-Producing Hybridoma

A hybridoma producing a monoclonal antibody can be produced according to known techniques as follows. First, an ICAM3 protein or a partial peptide thereof to be described later is used as a sensitizing antigen to immunize an animal according to a normal immunization method. The obtained immune cells are fused with known parent cells by a normal cell fusion method, and hybridomas are obtained. Further, the hybridomas are screened for cells producing a target antibody by a normal screening method, and thereby a hybridoma producing an anti-ICAM3 antibody is selected. A desired anti-ICAM3 monoclonal antibody is obtained from the hybridoma thus selected. Specifically, the following procedure is performed.

(1) Preparation of ICAM3 Protein

First, the ICAM3 gene is expressed, and thereby an ICAM3 protein used as the sensitizing antigen to obtain the antibody can be obtained. Specifically, the gene sequence encoding ICAM3 is inserted into a known expression vector and an appropriate host cell is transformed therewith. Then, from the host cell or culture supernatant, a target human ICAM3 protein is purified by a known method. A purified natural ICAM3 protein, or a fusion protein obtained by fusing a desired partial polypeptide of an ICAM3 protein with a different polypeptide, can also be used as the immunogen. To produce the fusion protein serving as the immunogen, for example, an antibody Fc fragment, a peptide tag, or the like can be used. A vector expressing the fusion protein can be produced by fusing genes encoding desired two or more kinds of polypeptide fragments in-frame, and inserting the fusion gene into an expression vector. The method for producing the fusion protein is described in Molecular Cloning, 2nd ed. (Sambrook, J et al., Molecular Cloning, 2nd ed. , 9.47-9.58, Cold Spring Harbor Lab. press, 1989).

The ICAM3 protein purified in this manner can be used as the sensitizing antigen for use in the immunization of a mammal. A partial peptide of ICAM3 can also be used as the sensitizing antigen. For example, peptides as follow can be used as the sensitizing antigen.

The region and the size of ICAM3 used as the partial peptide are not limited. The number of amino acids making up the peptide serving as the sensitizing antigen is at least 3 or more, for example, 5 or more, or preferably 6 or more. More specifically, a peptide made of 8 to 50, preferably 10 to 30, residues can be used as the sensitizing antigen.

(2) Immunization with ICAM3 Protein

A mammal is immunized with the ICAM3 protein or the partial peptide as the sensitizing antigen. The mammal to be immunized is not particularly limited. However, in order to obtain a monoclonal antibody by the cell fusion method, it is preferable to select an immunized animal by taking the compatibility with the parent cells used for the cell fusion into consideration. Generally, rodent animals are preferable as the immunized animal. Specifically, mouse, rat, hamster, or rabbit can be used as the immunized animal. Besides, monkey and the like can also be used as the immunized animal.

The above animals can be immunized with the sensitizing antigen according to known methods. For example, as a general method, a mammal can be immunized by intraperitoneal or subcutaneous injection of the sensitizing antigen. Specifically, the sensitizing antigen is administered to the mammal several times every 4 to 21 days. The sensitizing antigen is diluted to an appropriate dilution with PBS (Phosphate-Buffered Saline), physiological saline, or the like for use in the immunization. Further, the sensitizing antigen may be coadministered with an adjuvant. For example, the sensitizing antigen may be prepared by mixing with Freund' s complete adjuvant, followed by emulsification. Moreover, an appropriate carrier can be used in the immunization with the sensitizing antigen. Particularly, in a case where a partial peptide of a low molecular weight is used as the sensitizing antigen, the sensitizing antigen peptide is desirably attached to a carrier protein such as albumin or keyhole limpet hemocyanin for the immunization.

(3) DNA Immunization

The monoclonal antibody can also be obtained by DNA immunization. DNA immunization is a method for providing immune stimulation, including: administering to an animal to be immunized a vector DNA constructed in such a manner that a gene encoding an antigen protein can be expressed in the immunized animal; and expressing the immunizing antigen in the body of the immunized animal. In comparison with general immunization methods of administering a protein antigen, advantages as follow can be expected from the DNA immunization.

· It is possible to provide immune stimulation while retaining the structure of a membrane protein such as ICAM3.
· No purification of immunizing antigen is necessary.

To obtain a monoclonal antibody of the present invention by the DNA immunization, first, a DNA expressing the ICAM3 protein is administered to an animal to be immunized. The DNA encoding ICAM3 can be synthesized by known methods such as PCR. The obtained DNA is inserted into an appropriate expression vector, and administered to the animal to be immunized. As the expression vector, for example, commercially-available expression vectors such as pcDNA3.1 can be used. As the method for administering the vector into the body, generally-used methods can be employed. For example, the DNA immunization can be carried out by injecting gold particles having an expression vector adhered thereto into cells with a gene gun.

(4) Production of Hybridoma

When a desired increase in an antibody amount in a serum is observed after a mammal is immunized as described above, immune cells are collected from the mammal and subjected to cell fusion. As preferable immune cells, particularly spleen cells can be used.
As the cells fused with the immune cells, mammalian myeloma cells are used. The myeloma cells preferably include an appropriate selection marker for screening. The selection marker refers to such a trait that cells can (or cannot) survive under a specific culture condition. As the selection marker, hypoxanthine-guanine-phosphoribosyl transferase deficiency (hereinafter abbreviated as "HGPRT deficiency"), thymidine kinase deficiency (hereinafter abbreviated as "TK deficiency"), or the like is known. HGPRT- and TK-deficient cells are sensitive to hypoxanthine-aminopterin-thymidine (hereinafter abbreviated as "HAT sensitive"). HAT sensitive cells cannot synthesize DNA in a HAT selection medium and die. Meanwhile, when fused with normal cells, the HAT sensitive cells can utilize the salvage path of the normal cells and continue DNA synthesis, accordingly proliferating even in a HAT selection medium.
HGPRT-deficient or TK-deficient cells can be selected with a medium containing 6-thioguanine, 8-azaguanine (hereinafter abbreviated as "8AG"), or 5'-bromodeoxyuridine. Normal cells incorporate these pyrimidine analogues into the DNA and die. Meanwhile, cells deficient in these enzymes cannot incorporate these pyrimidine analogues and can survive in the selection medium. Besides, a selection marker called G418 resistance provides resistance to 2-deoxystreptamine antibiotics (gentamicin analogues) via a neomycin resistance gene. Various myeloma cells suitable for cell fusion are known. For example, myeloma cells as follow can be used for production of the monoclonal antibody in the present invention.
P3 (P3x63Ag8.653) (J. Immunol. (1979) 123, 1548-1550), P3x63Ag8U.1 (Current Topics in Microbiology and Immunology (1978) 81, 1-7), NS-1 (Kohler. G. and Milstein, C. Eur. J. Immunol. (1976) 6, 511-519), MPC-11 (Margulies. D. H. et al., Cell (1976) 8, 405-415), SP2/0 (Shulman, M. et al., Nature (1978) 276, 269-270), FO (de St. Groth, S. F. et al., J. Immunol. Methods (1980) 35, 1-21), S194 (Trowbridge, I. S. J. Exp. Med. (1978) 148, 313-323), R210 (Galfre, G. et al., Nature (1979) 277, 131-133), and the like.
Basically, cell fusion between the immune cells and the myeloma cells are carried out in accordance with a known method such as, for example, the method of Kohler and Milstein (Kohler. G. and Milstein, C., Methods Enzymol. (1981) 73, 3-46).
More specifically, the cell fusion can be carried out in a normal nutrient culture solution in the presence of a cell fusion promoter, for example. As the fusion promoter, for example, polyethylene glycol (PEG), Sendai virus (HVJ), and the like can be used. Further, an auxiliary substance such as dimethyl sulfoxide can be added, if desired, to increase the fusion efficiency.
Any ratio of immune cells to myeloma cells may be set for use. For example, immune cells are preferably 1 to 10 times as large as myeloma cells. As the culture solution used for the cell fusion, it is possible to use, for example, culture solutions normally used for this type of cell culturing such as an RPMI1640 culture solution, a MEM culture solution, and others that are suitable for proliferation of myeloma cell line. Further, a serum supplement such as fetal calf serum (FCS) can also be added to the culture solution.
In the cell fusion, predetermined amounts of immune cells and myeloma cells are mixed well in the culture solution, and mixed with a PEG solution preheated to approximately 37°C. Thus, target fusion cells (hybridomas) are formed. In the cell fusion method, for example, PEG having an average molecular weight of approximately 1000 to 6000 can be added normally at a concentration of 30 to 60% (w/v). Subsequently, a cell fusion agent and the like unfavorable for growth of the hybridomas are removed by repeating an operation of sequentially adding an appropriate culture solution noted above and removing the supernatant by centrifugation.
The hybridomas obtained in this manner can be selected by utilizing a selection culture solution corresponding to the selection marker of the myeloma used in the cell fusion. For example, cells having HGPRT- or TK-deficiency can be selected by culturing with a HAT culture solution (culture solution containing hypoxanthine, aminopterin and thymidine). Specifically, when HAT-sensitive myeloma cells are used in the cell fusion, cells successfully fused with normal cells can be selectively proliferated in a HAT culture solution. Culturing using the HAT culture solution is continued for a period enough to kill cells (non-fusion cells) but not the target hybridomas. Specifically, generally, the target hybridomas can be selected by culturing for several days to several weeks. Next, by conducting a normal limiting dilution procedure, the hybridoma producing the target antibody can be screened and monocloned.

The screening for the target antibody and monocloning can be suitably carried out by a screening method based on a known antigen-antibody reaction. For example, an antigen is bound to a carrier such as beads made of polystyrene or the like or a commercially-available 96-well microtiter plate, and is reacted with the culture supernatant of the hybridomas. Next, after the carrier is rinsed, an enzyme-labeled secondary antibody or the like is reacted therewith. If the target antibody reacting with the sensitizing antigen is contained in the culture supernatant, the secondary antibody binds to the carrier via the antibody. Finally, by detecting the secondary antibody binding to the carrier, whether or not the target antibody is present in the culture supernatant can be determined. The hybridoma producing a desired antibody having a binding ability to the antigen can be cloned by the limiting dilution procedure or the like. In this process, as the antigen, one used in the immunization or one that is substantially the same as the ICAM3 protein can be suitably used. For example, cell lines expressing ICAM3, soluble ICAM3, or the like can be used as the antigen.

To produce an antibody against human ICAM3, the method described in International Publication No. WO03/104453 can also be employed.

Moreover, instead of the method for obtaining the hybridoma by immunizing an animal other than human with an antigen, the target antibody can be obtained by sensitizing a human lymphocyte with an antigen. Specifically, first, human lymphocytes are sensitized with an ICAM3 protein in vitro. Next, the immunosensitized lymphocytes are fused with an appropriate fusion partner. As the fusion partner, for example, it is possible to use myeloma cells which are derived from human and which are capable of dividing indefinitely (see Japanese Examined Patent Application Publication No. Hei 1-59878).

Further, the anti-ICAM3 human antibody can be obtained by administering an ICAM3 protein, as an antigen, to a transgenic animal having all the repertoires of the human antibody gene, or by immunizing such an animal with a DNA constructed in such a manner that ICAM3 is expressed in the animal. Antibody-producing cells of the immunized animal can be immortalized by a treatment such as cell fusion with an appropriate fusion partner or infection with Epstein-Barr virus. A human antibody against the ICAM3 protein can be isolated from the immortalized cells thus obtained (see International Publication Nos. WO94/25585 WO93/12227, WO92/03918, WO94/02602). Further, by cloning the immortalized cells, it is possible to clone cells producing an antibody having target reaction specificity. When a transgenic animal is used as the immunized animal, the immune system of the animal recognizes human ICAM3 as a foreign substance. In this manner, a human antibody against human ICAM3 can be easily obtained.

(5) Acquisition of Monoclonal Antibody from Hybridoma

The monoclonal antibody-producing hybridoma produced as described above can be subcultured in a normal culture solution. Moreover, the hybridoma can be preserved in liquid nitrogen for an extended period.

The target monoclonal antibody can be obtained from culture supernatant of the hybridoma cultured by a normal method. Alternatively, the monoclonal antibody can be obtained in the form of ascitic fluid by administering the hybridoma to a mammal compatible therewith and allowing the hybridoma to proliferate. The former method is suitable for obtaining a high purity antibody.

2. Production of Anti-ICAM3 Antibody by Genetic Engineering Technique

(1) Cloning of Antibody Gene

Using an antibody gene cloned from an antibody-producing cell, the antibody can be produced by employing a genetic engineering technique. The cloned antibody gene can be expressed as an antibody by incorporating the gene into an appropriate vector which is introduced into a host. The method for isolating the antibody gene, introducing it into the vector, and transforming the host cell has already been established (see, for example, Vandamme, A. M. et al., Eur. J. Biochem. (1990) 192, 767-775).

For example, a cDNA encoding a variable region of the anti-ICAM3 antibody can be obtained from cells of a hybridoma that produces the anti-ICAM3 antibody. For this, normally, first, total RNA is extracted from the hybridoma. As the method for extracting mRNA from the cell, for example, the following methods can be employed.

· Guanidine ultracentrifugation method (Chirgwin, J. M. et al., Biochemistry (1979) 18, 5294-5299)
· AGPC method (Chomczynski, P. et al., Anal. Biochem. (1987) 162, 156-159)

The extracted mRNA can be purified using mRNA Purification Kit (manufactured by GE Healthcare Bio-Sciences Ltd.) or the like. Alternatively, kits for extracting total mRNA directly from a cell, such as QuickPrep mRNA Purification Kit (manufactured by GE Healthcare Bio-Sciences Ltd.), are also commercially available. Using such a kit, total mRNA can be obtained from the hybridoma. The cDNA encoding the variable region of the antibody can be synthesized from the obtained mRNA using a reverse transcriptase. In this event, any sequence of 15-30 bases selected from a sequence common to antibody genes can be used as a primer. The cDNA can be synthesized with AMV ReverseTranscriptase First-strand cDNA Synthesis Kit (manufactured by Seikagaku Corporation) or the like. Moreover, to synthesize and amplify the cDNA, it is possible to use 5'-Ampli FINDER RACE Kit (manufactured by Clontech Laboratories, Inc.) and the 5'-RACE method utilizing PCR (Frohman, M. A. et al., Proc. Natl. Acad. Sci. USA (1988) 85, 8998-9002, Belyavsky, A. et al., Nucleic Acids Res. (1989) 17, 2919-2932). Further, in such a process of synthesizing the cDNA, an appropriate restriction enzyme site described below can be introduced to both ends of the cDNA.

A target cDNA fragment from the obtained PCR product is purified, and then ligated to a vector DNA. Recombinant vectors produced in this manner are introduced into Escherichia coli or the like. After colonies are selected, a desired recombinant vector can be prepared from colony-forming Escherichia coli. Then, the base sequence of the cDNA can be verified by a known method, for example, dideoxynucleotide chain termination method, or the like.

Furthermore, to obtain the gene encoding the variable region of the antibody, a cDNA library can also be used. First, a cDNA is synthesized using the mRNA extracted from the antibody-producing cells as a template to obtain a cDNA library. To synthesize the cDNA library, it is convenient to use a commercially-available kit. In practice, the amount of mRNA obtained only from a few cells is quite small, which results in a low yield if the mRNA is purified directly therefrom. Hence, normally, the mRNA is purified after a carrier RNA clearly not containing the antibody gene is added. Alternatively, if a certain amount of RNA can be extracted from the antibody-producing cells, efficient extraction is possible without addition of the carrier RNA. For example, the addition of the carrier RNA may not be necessary when the RNA is extracted from 10 or more, or 30 or more, preferably 50 or more antibody-producing cells.

The antibody gene is amplified by PCR using the obtained cDNA library as a template. Primers for amplifying an antibody gene by PCR are known. For example, it is possible to design primers for amplification of a human antibody gene based on the disclosures of the article (J. Mol. Biol. (1991) 222, 581-597) and the like. The base sequences of these primers are different for each subclass of immunoglobulin. Hence, when a cDNA library of an unknown subclass is used as a template, PCR is carried out selecting a primer with all possibilities taken into consideration.

Specifically, for example, when a gene encoding human IgG is to be obtained, primers capable of amplifying genes encoding $\gamma 1$ to $\gamma 5$ as the heavy chain and $\kappa$ and $\lambda$ chains as the light chain can be used. To amplify an IgG variable region gene, generally a primer annealing to a part corresponding to a hinge region is used as the primer at the 3' side. Meanwhile, primers corresponding to the respective subclasses can be used as the primer at the 5' side.

The PCR products produced by gene-amplification primers for each subclass of heavy chain and light chain can be used as libraries independently to each other. Using the libraries thus synthesized, an immunoglobulin composed of a combination of heavy and light chains can be reshaped. The target antibody can be screened for based on the binding activity of the reshaped immunoglobulin to ICAM3.

(2) Introduction of Antibody Gene into Host Cell

To produce the anti-ICAM3 antibody, the cloned antibody gene is incorporated into an expression vector so that the gene can be expressed under the control of an expression regulatory region. The expression regulatory region for expressing the antibody includes, for example, an enhancer and a promoter. Next, a recombinant cell expressing the DNA encoding the anti-ICAM3 antibody can be obtained by transforming an appropriate host cell with this expression vector.

For the expression of the antibody gene, it is possible to incorporate DNAs encoding the heavy chain and the light chain of the antibody into different expression vectors. By co-transforming (co-transfecting) a single host cell with the vectors incorporating the heavy chain and the light chain, an antibody molecule comprising the heavy chain and the light chain can be expressed. Alternatively, the host cell may be transformed by incorporating the DNAs encoding the heavy chain and the light chain into a single expression vector (see International Publication No. WO94/11523).

Many combinations of host and expression vector are known which produce an antibody by introducing isolated antibody genes into an appropriate host. Any of these expression systems are applicable to the present invention. When a eukaryotic cell is used as the host, it is possible to use an animal cell, a plant cell, or a fungal cell. Specifically, examples of the animal cell that can be used in the present invention include mammalian cells such as CHO, COS, myeloma, BHK (baby hamster kidney), Hela, Vero, HEK293, Ba/F3, HL-60, Jurkat, and SK-HEP1; amphibian cells such as Xenopus laevis oocytes; and insect cells such as sf9, sf21, and Tn5.

As for the plant cell, an antibody gene expression system using cells derived from the genus Nicotiana such as Nicotiana tabacum is known. For the transformation of the plant cell, callus cultured cells can be used.

Further, as the fungal cell, the following cells can be used. For example, as for a yeast, examples thereof include yeasts belonging to the genus Saccharomyces such as Saccharomyces cerevisiae, and yeasts belonging to the genus Pichia such as methylotrophic yeast (Pichiapastoris). Moreover, as for a filamentous fungus, examples thereof include filamentous fungi belonging to the genus Aspergillus such as Aspergillus niger.

Alternatively, an antibody gene expression system using prokaryotic cells is also known. For example, when a bacterial cell is used, bacterial cells such as Escherichia coli and Bacillus subtilis can be used in the present invention.

When a mammalian cell is used, a construct containing a commonly-used, useful promoter, an antibody gene to be expressed, and a poly-A signal functionally ligated downstream at the 3' side of the gene can be used for the expression. The construct may further contain an enhancer. An example of a promoter /enhancer is human cytomegalovirus immediate early promoter/enhancer.

Furthermore, a virus promoter/enhancer, a promoter/enhancer derived from mammalian cells such as human elongation factor $1\alpha$ (HEF1$\alpha$), or the like can be used for the antibody expression. Specific example of a virus capable of utilizing such a promoter/enhancer include retroviruses, polyomaviruses, adenoviruses, simian virus 40 (SV40), and the like.

When an SV40 promoter/enhancer is used, the method of Mulligan et al. (Nature (1979) 277, 108) can be employed. Moreover, an HEF1$\alpha$ promoter/enhancer can be used easily for the expression of the target gene by the method of Mizushima et al. (Nucleic Acids Res. (1990) 18, 5322).

When an animal cell is used to produce the antibody, a signal sequence of the heavy chain gene or the light chain gene of the antibody is desirably used as a signal sequence necessary for the secretion to the outside of the cell. Further, the signal sequence of a secretory protein such as IL-3 or IL-6 can also be used.

In a case of Escherichia coli, a construct in which a commonly-used, useful promoter, a signal sequence for the antibody secretion, and an antibody gene to be expressed are functionally ligated can be used for the expression of the gene. Examples of the promoter include a lacZ promoter and an araB promoter. When the lacZ promoter is used, the method of Ward et al. (Nature (1989) 341, 544-546; FASEB J. (1992) 6, 2422-2427) can be employed. Alternatively, the araB promoter can be used for the expression of the target gene by the method of Better et al. (Science (1988) 240, 1041-1043).

For production in the periplasm of Escherichia coli, the pelB signal sequence (Lei, S. P. et al., J. Bacteriol. (1987) 169, 4379) may be used as the signal sequence for the antibody secretion. After the antibody produced in the periplasm is separated, the antibody structure can be refolded using a protein denaturing agent such as urea or guanidine hydrochloride so that the antibody has a desired binding activity.

As a replication origin to be inserted into the expression vector, it is possible to use ones derived from SV40, polyomaviruses, adenoviruses, bovine papilloma virus (BPV), and the like. Further, to amplify gene copies in the host cell system, a selection marker can be inserted into the expression vector. Specifically, it is possible to use selection markers such as aminoglycoside phosphotransferase (APH) gene, thymidine kinase (TK) gene, Escherichia coli xanthine guanine phosphoribosyl transferase (Ecogpt) gene, and dihydrofolate reductase (dhfr) gene.

(3) Acquisition of Antibody from Host Cell

These expression vectors are introduced into host cells, and then the transformed host cells are cultured in vitro or in vivo to produce the target antibody. The host cells can be cultured by known methods. For example, as the culture solution, DMEM, MEM, RPMI1640, or IMDM can be used. A serum supplement such as fetal calf serum (FCS) can be used in combination.

The antibody expressed and produced as described above can be purified by employing known methods normally used in protein purification alone or in combination as appropriate. For example, the antibody can be separated and purified by appropriately selecting and combining an affinity column such as a protein A column, a chromatography column, and filter, as well as procedures such as ultrafiltration, salting out, and dialysis (Antibodies A Laboratory Manual. Ed Harlow, David Lane, Cold Spring Harbor Laboratory, 1988).

Thus, the present invention provides a gene encoding the antibody of the present invention. Moreover, the present invention provides a vector comprising the gene. Further, the present invention provides a host cell comprising the vector. Furthermore, the present invention provides a method for producing an antibody encoded by the gene, the method comprising the step of culturing the host cell.

3. Production of Antibody by Transgenic Animal

Besides the above-described host cell, a transgenic animal can also be used to produce a recombinant antibody. In other words, the target antibody can be obtained from an animal into which the gene encoding the target antibody is introduced. For example, the antibody gene can be constructed as a fusion gene by inserting the antibody gene in-frame within a gene encoding a protein that is produced specifically in milk. As the protein secreted into milk, for example, goat $\beta$-casein and the like can be used. A DNA fragment containing the fusion gene having the antibody gene inserted is injected into a goat embryo, and the injected embryo is introduced into a female goat. A transgenic goat (or progeny thereof) born from the embryo-receiving goat produces milk from which the desired antibody can be obtained as a fusion protein with the milk protein. Additionally, a hormone can be used as appropriate in a transgenic goat so as to increase the amount of milk containing the desired antibody that is produced from the transgenic goat (Ebert, K. M. et al., Bio/Technology (1994) 12, 699-702).

[Pharmaceutical Composition]

The anti-ICAM3 antibody is useful for treatment against a cancer expressing ICAM3, and so forth. Specifically, the present invention provides a pharmaceutical composition comprising the anti-ICAM3 antibody as an active ingredient. In a certain embodiment, the pharmaceutical composition of the present invention is a cell-proliferation depressant, particularly an anti-cancer agent. The cell-proliferation depressant and the anti-cancer agent of the present invention are preferably administered to a subject who has a cancer or a subject who may have a cancer.

The anti-ICAM3 antibody used in the pharmaceutical composition (for example, anti-cancer agent) of the present invention is not particularly limited. For example, any of the anti-ICAM3 antibodies described above can be used.

In the present invention, the phrase "comprising the anti-ICAM3 antibody as an active ingredient" means comprising the anti-ICAM3 antibody as a primary active component, and does not limit the content ratio of the anti-ICAM3 antibody.

In a case where the disease targeted by the pharmaceutical composition of the present invention is a cancer, the targeted cancer is not particularly limited, but is preferably leukemia (for example, acute myeloid leukemia and the like), myeloma, and malignant lymphoma. The cancer may be any of primary cancer and metastatic cancer.

The pharmaceutical composition of the present invention can be administered to a patient by any of oral administration and parenteral administration. Parenteral administration is preferable. Specific examples of the administration method include injection administration, transnasal administration, transpulmonary administration, transdermal administration, and the like. Examples of the injection administration include intravenous injection, intramuscular injection, intraperitoneal injection, subcutaneous injection, and the like. By carrying out these administration methods, the pharmaceutical composition of the present invention can be systemically or locally administered. Moreover, the administration method can be selected as appropriate in accordance with the age and condition of the patient. As the dose, for example, the dose in a single administration can be selected from the range of 0.0001 mg to 1000 mg per kg body weight. Alternatively, for example, the dose can be selected from the range of 0.001 to 100000 mg per patient. However, the pharmaceutical composition of the present invention is not limited to these doses.

The pharmaceutical composition of the present invention can be formulated in accordance with a conventional process (for example, Remington's Pharmaceutical Science, latest edition, Mark Publishing Company, Easton, U.S.A), and may also comprise a pharmaceutically acceptable carrier or an additive in addition to the active ingredient. Examples of the carrier and additive include surfactants, excipients, coloring agents, flavoring agents, preservatives, stabilizers, buffers, suspensions, isotonic agents, binders, disintegrators, lubricants, fluidity improvers, taste masking agents, and the like. Without limitation thereto, other commonly-used carriers and additives can be further used as appropriate. Specific examples of the carrier include light anhydrous silicic acid, lactose, crystalline cellulose, mannitol, starch, carmellose calcium, carmellose sodium, hydroxypropyl cellulose, hydroxypropyl methylcellulose, polyvinyl acetal diethylaminoacetate, polyvinyl pyrrolidone, gelatin, medium chain fatty acid triglycerides, polyoxyethylene hydrogenated caster oil 60, white sugar, carboxymethyl cellulose, corn starch, inorganic salts, and the like.

When brough tinto contact with ICAM3-expressing cells, the anti-ICAM3 antibody of the present invention can induce cell death in the cells, damage, and/or suppress the cell proliferation. In the present invention, "contact" is carried out, for example, by adding the antibody to a culture solution of ICAM3-expressing cells cultured in vitro. Further, in the present invention, "contact" is carried out also by administering the antibody to a non-human animal into which ICAM3-expressing cells have been transplanted or an animal having a cancer cell endogenously expressing ICAM3. Such methods for using the anti-ICAM3 antibody of the present invention are also within the scope of the present invention. The anti-ICAM3 antibody to be used is not particularly limited, For example, any of the anti-ICAM3 antibodies described above can be used. The cell to which the anti-ICAM3 antibody binds is not particularly limited, as long as the cell expresses ICAM3. A preferable ICAM3-expressing cell in the present invention is a cancer cell. The anti-ICAM3 antibody of the present invention is more preferably used against leukemia, myeloma, or malignant lymphoma.

[Diagnostic Agent (Diagnosis Method)]

Furthermore, the present invention provides a diagnosis method for a cancer, characterized by comprising the step of:

detecting any one of an ICAM3 protein and a gene encoding the ICAM3 protein. The expression level of ICAM3 has been found significantly high in cancer cell lines, particularly leukemia (for example, acute myeloid leukemia and the like) and myeloma. Hence, ICAM3 is useful as a marker for specifically detecting a cancer.

One of specific examples of the diagnosis method of the present invention is a diagnosis method for a cancer, comprising the steps of:

preparing a sample isolated from a subject; and
detecting a level of any one of an ICAM3 protein and an ICAM3 gene expressed in the sample. The method of the present invention may further comprise a step of:
evaluating a possibility that the subj ect has a cancer on a basis of the level of any one of an ICAM3 protein and an ICAM3 gene expressed.

(1) Detection of ICAM3 Protein

One embodiment of the method of the present invention is to diagnose a cancer by detecting an ICAM3 protein in a sample. Preferably, the ICAM3 protein is detected by using an antibody for recognizing the ICAM3 protein.
In the present invention, detection is meant to include quantitative or qualitative detection. Examples of the qualitative detection include measurement only for whether or not the ICAM3 protein is present, measurement for whether or not the ICAM3 protein is present in a certain amount or larger, measurement to compare the amount of the ICAM3 protein with that of another sample (for example, a control sample or the like), and other similar measurements. Meanwhile, examples of the quantitative detection include measurement for the concentration of the ICAM3 protein, measurement for the amount of the ICAM3 protein, and other similar measurements.
In the present invention, a test sample is not particularly limited, as long as there is a possibility that the sample contains an ICAM3 protein. Specifically, a sample collected from a body of an organism such as a mammal is preferable. A further preferable sample is a sample collected from human. Specific examples of the test sample include blood, interstitial fluid, plasma, extravascular fluid, cerebrospinal fluid, synovial fluid, pleural fluid, serum, lymph, saliva, urine, tissue, and the like. A preferable sample is a sample obtained from a test sample such as a specimen of fixed tissue or cells collected from a body of an organism, or a culture solution of the cells.
The cancer to be diagnosed by the present invention is not particularly limited and may be any cancer. Specific examples thereof include leukemia (for example, acute myeloid leukemia and the like) and myeloma. In the present invention, any of primary lesions and metastatic lesions can be diagnosed.
In the present invention, if the protein is detected in a test sample, the diagnosis of the cancer is given using the level of the protein as an indicator. Specifically, if the amount of the ICAM3 protein detected in a test sample is larger than that of a negative control or healthy subj ects, this indicates that the subject has a cancer or is likely to develop a cancer in the future. In other words, the present invention relates to a diagnosis method for a cancer, comprising the steps of:

detecting an ICAM3 expression level in a biological sample collected from a subject (step (1)); and
determining that the subject has a cancer if the ICAM3 expression level detected in the step (1) is higher than that of a control (step (2)).

In the present invention, the control refers to a sample serving as a reference for comparison, and includes negative controls and biological samples from healthy subjects. A negative control can be obtained by collecting biological samples from healthy subjects, and mixing together as necessary. The ICAM3 expression level of the control can be detected concurrently with the ICAM3 expression level in a biological sample from a subject. Alternatively, the ICAM3 expression level in biological samples from a large number of healthy subjects is detected in advance, and a standard expression level of the healthy subjects is statistically determined. The standard value thus determined can be used as a control value. Specifically, for example, mean value $\pm 2 \times$ standard deviation (S.D.), or mean value $\pm 3 \times$ standard deviation (S.D.), can be used as a standard value. Statistically, the mean value $\pm 2 \times$ standard deviation (S.D.) includes the values of 80% of the healthy subjects, and the mean value $\pm 3 \times$ standard deviation (S.D.) includes the values of 90%.
Alternatively, the ICAM3 expression level in the control can be set using an ROC curve. The ROC curve (receiver operating characteristic curve) is a graph showing the detection sensitivity on the vertical axis and the false-positive rate (i.e., "1-specificity") on the horizontal axis. In the present invention, an ROC curve can be obtained by plotting changes in sensitivity and false-positive rate when the reference value for determining the ICAM3 expression level in a biological sample is varied continuously.
Note that the "reference value" for obtaining the ROC curve is a numerical value used temporarily for statistical analysis. Generally, the "reference value" for obtaining the ROC curve is continuously varied within a range that can cover all the reference values possibly selected. For example, the reference value can be varied between the minimum value and the maximum value of the measured values of ICAM3 in the population to be analyzed.

Based on the obtained ROC curve, a desired detection sensitivity and a standard value expected to be accurate can be selected. A standard value statistically set with an ROC curve or the like is also called a cut-off value. In the detection method for a cancer based on a cut-off value, the ICAM3 expression level detected in the step (1) is compared with the cut-off value in the step (2). Then, if the ICAM3 expression level detected in the step (1) is higher than the cut-off value, a cancer is detected in the subject.

In the present invention, the ICAM3 expression level can be determined by any method. Specifically, the ICAM3 expression level can be estimated by evaluating the amounts of the ICAM3 mRNA and the ICAM3 protein or the biological activity of the ICAM3 protein. The amounts of the ICAM3 mRNA and protein can be determined by the methods described in this description.

In the present invention, a particularly suitable subject is human. Note that when an animal other than human is a subject, the ICAM3 protein in the animal species is detected. The method for detecting the ICAM3 protein included in a test sample is not particularly limited, but detection using the anti-ICAM3 antibody by immunological methods as exemplified below is preferable.

Enzyme-linked immunosorbent assay (ELISA), radioimmunoassay (RIA), enzyme immunoassay (EIA), fluorescence immunoassay (FIA), luminescence immunoassay (LIA), immunoprecipitation (IP), turbidimetric immunoassay (TIA), western blotting (WB), immunohistochemistry (IHC), single radial immunodiffusion (SRID), dot blotting, and slot blotting.

Among these procedures, immunohistochemistry (IHC) includes the step of detecting an ICAM3 protein on a section of fixed tissue or cells obtained from a patient having a cancer, and is one of immunological assays preferable as the diagnosis method for a cancer. The above-described immunological methods such as immunohistochemistry (IHC) are methods known to those skilled in the art.

Since ICAM3 is a membrane protein with specifically enhanced expression in cancer cells, cancer cells (including cancer cells in cancer tissues) can be detected by the anti-ICAM3 antibody. Thus, cancer cells included in cells and tissue collected from a body are detected by the above immunohistological analysis.

In another preferable embodiment, cancer cells in a body can be detected with the anti-ICAM3 antibody. To trace the antibody administered to the body, it is possible to use an anti-ICAM3 antibody which is detectably labeled. To be more specific, this method includes the steps of: administering an anti-ICAM3 antibody labeled with a labeled substance, such as a radioisotope, to a subject; and detecting accumulation of the labeled substance. For example, using a fluorescent substance, a luminescent substance, or a radioisotope as the labeled substance, the behavior of an antibody labeled with these in the body is traced, and thereby cancer cells in the body can be detected. The antibody labeled with a fluorescent substance or a luminescent substance can be observed by using an endoscope or laparoscope. When a radioisotope is used as the labeled substance, the localization of the antibody can be imaged by tracing the radioactivity. In the present invention, the localization of the anti-ICAM3 antibody in the body indicates the presence of cancer cells.

As the radioisotope for labeling the antibody to detect a cancer in a body, positron emitting nuclides can be used. For example, the antibody can be labeled with positron emitting nuclides such as 18F, 55Co, 64Cu, 66Ga, 68Ga, 76Br, 89Zr, and 124I. For labeling of the anti-ICAM3 antibody with these positron emitting nuclides, a known method (Acta Oncol. 32, 825-830, 1993) can be employed.

After the anti-ICAM3 antibody labeled with positron emitting nuclides is administered to human or an animal, the radiation emitted from the radioactive nuclide is measured from the outside of the body with a PET (positron emission tomography device), and converted to an image by a computer tomography procedure. The PET is a device for obtaining data on the behavior and the like of a drug in the body non-invasively. With the PET, the emission intensity can be imaged quantitatively as a signal strength. The use of the PET as described above enables detection of an antigen molecule that is highly expressed in a specific cancer without collecting a sample from a patient. The anti-ICAM3 antibody may also be radiolabeled with a short-lived nuclide using a positron emitting nuclide such as 11C, 13N, 150, 18F, and 45Ti besides the above-described nuclides.

Research and development have been progressed concerning: production of short-lived nuclides using the above nuclides with a medical cyclotron; manufacturing techniques of short-lived radiolabeled compounds; and the like. By these techniques, an anti-ICAM3 antibody can be labeled with various radioisotopes. The anti-ICAM3 antibody administered to a patient accumulates in primary and metastatic lesions by the specificity of the anti-ICAM3 antibody against the pathological tissue at each site. When the anti-ICAM3 antibody is labeled with a positron emitting nuclide, the presence of primary and metastatic lesions can be detected according to the localization of the radioactivity. For the diagnostic application, a gamma-particle or positron emission energy of 25-4000 keV as an activity value can be suitably used. In addition, by selecting an appropriate nuclide and further by administering the nuclide in a large amount, a therapeutic effect can also be expected. To obtain an anti-cancer action by radiation, a nuclide providing a gamma-particle or positron emission energy of 70-700 keV can be used.

(2) Detection of Polynucleotide Encoding ICAM3 Protein

In another embodiment of the method of the present invention, expression of a polynucleotide of ICAM3 is detected. In the present invention, the polynucleotide to be detected is not particularly limited, but an mRNA is preferable. In the present invention, detection is meant to include quantitative or qualitative detection. Examples of the qualitative detection include measurement only for whether or not the ICAM3 mRNA is present, measurement for whether or not the ICAM3 mRNA is present in a certain amount or larger, measurement to compare the amount of the ICAM3 mRNA with that of another sample (for example, a control sample or the like), and other similar measurements. Meanwhile, examples of quantitative detection include measurement for the concentration of the ICAM3 mRNA, measurement for the amount of the ICAM3 mRNA, and other similar measurements.

In the present invention, as the test sample, any sample that possibly contains the ICAM3 mRNA can be used. The sample is preferably a sample collected from a body of an organism such as a mammal, further preferably a sample collected from human. Specific examples of the test sample include blood, interstitial fluid, plasma, extravascular fluid, cerebrospinal fluid, synovial fluid, pleural fluid, serum, lymph, saliva, urine, tissue, and the like. The test sample of the present invention also includes a sample obtained from a test sample such as a specimen of fixed tissue or cells collected from a body of an organism, or a culture solution of the cells.

An in situ hybridization method is suitably employed when a sample used is obtained from a test sample such as a specimen of fixed tissue or cells collected from a body of an organism, or a culture solution of the cells. The in situ hybridization method has been developed as means for verifying the presence or distribution of a specific DNA or RNA in cells and tissues, and the degree of the expression. As the principle, the method utilizes the nature of a probe nucleic acid having a base sequence complementary to the sequence of a specific nucleic acid in a cell, the probe nucleic acid specifically forming a complex. If the probe is labeled in advance with radioisotope (RI), an antigenic substance (hapten), or the like, detecting the label makes a hybridized site distinguishable. For this reason, the in situ hybridization method is employed for detection of DNA, RNA, and the like in cells. As the label for the probe, a radioisotope can be suitably used. Other suitable examples of the label include fluorescent labels utilizing a hapten such as a non-radioactive substance biotin and digoxigenin, and the like. An example of a particularly suitable detection method is a detection method by fluorescence in situ hibridization, so-called FISH.

The cancer diagnosed by the present invention is not particularly limited. Specific examples thereof include leukemia and myeloma. In the present invention, any of primary lesions and metastatic lesions can be diagnosed.

In the present invention, any animal species expressing the ICAM3 gene can be a subject. A particularly suitable subject is human. Note that when an animal species other than human is a subject, the ICAM3 gene of the animal species is detected.

A specific embodiment of the detection method will be described below. First, a sample is prepared from a subject. Next, the ICAM3 mRNA included in the sample is detected. In the present invention, a cDNA synthesized from the mRNA can also be detected. In the present invention, when the ICAM3 mRNA or the cDNA encoding ICAM3 is detected in the test sample, it is concluded that there is a possibility of a cancer. For example, when the amount of the ICAM3 mRNA or cDNA encoding ICAM3 detected in the test sample is larger than that of a negative control or healthy subjects, this indicates that the subject has a cancer or is likely to develop a cancer in the future.

The method for detecting an mRNA is known. Specifically, for example, using a solidified sample selected from a gene chip, a cDNA array, and a membrane filter, nucleic acid hybridization, RT-PCR, real-time PCR, subtractiontechnique, differential display, differential hybridization, cross hybridization, and the like can be employed in the present invention.

The detection method of the present invention may be automated using various automatic detection devices. The automation enables inspection of a large number of samples in a short period of time.

[Kit for Diagnosing Cancer]

The present invention also provides a diagnostic agent or a kit for diagnosing a cancer, the diagnostic agent or the kit comprising a reagent for detecting an ICAM3 protein in a test sample. The diagnostic agent of the present invention comprises at least the anti-ICAM3 antibody.

A kit for diagnosing a cancer can be prepared by combining the reagent for diagnosing a cancer of the present invention with another component used for detection of ICAM3. In other words, the present invention relates to a kit for diagnosing a cancer which comprises an antibody binding to ICAM3 and a reagent for detecting binding between the antibody and the ICAM3, and which may further comprise a control sample of a biological sample including ICAM3. The kit of the present invention may further comprise an instruction for explaining measurement procedure.

[Examples]

Hereinafter, the present invention will be more specifically described based on Examples and comparative example. However, the present invention is not to be limited to Examples below.

(Example 1) Acquisition of Mouse Anti-ICAM3 Antibody

(1) Establishment of ICAM3-Expressing BaF3

An ICAM3 (GenBank No. NM_002162) cDNA fragment (SEQ ID NO: 1) was introduced into an expression vector for animal cell. The expression vector thus prepared was introduced into Ba/F3 cells by electroporation, and an ICAM3-expressing BaF3 transfectant (ICAM3/BaF3) was established. Note that the base sequence was checked using BigDye Terminator Cycle Sequencing Kit (Applied Biosystems) and DNA Sequencer ABI PRISM 3700 DNA Sequencer (Applied Biosystems) in accordance with the attached instructions.

(2) Production of Mouse Anti-ICAM3 Antibody-Producing Hybridoma

i) Production of Hybridoma Using ICAM3/BaF3 Cell-Immunized Mice

For immunization, 4-week-old male MRL/lpr mice (Charles River Laboratories Japan, Inc.) were used. The ICAM3/BaF3 cells were suspended in 100 $\mu$l of PBS at a cell concentration of $1\times10^7$ to $3\times10^7$ cells. This was intravenously administered to the mice. After the weekly immunization four times, the mouse spleen cells and mouse myeloma cells P3X63Ag8U.1 (hereinafter P3U1, ATCC CRL-1597) were subjected to cell fusion using PEG1500 (Roche Diagnostics) in accordance with a conventional process. The fusion cells (i.e., hybridomas) were cultured in a HAT medium (RPMI1640+PS, 10% FCS, HAT (Sigma, H0262) with 5% BM condimed H1 (Roche: #1088947)).

ii) 1.2.2. Selection of Hybridoma Cells

Approximately one week after the cell fusion, primary screening was carried out based on cell agglutination-inducing activity. The cell agglutination-inducing activity was checked as follows. The ICAM3/BaF3 cells were seeded into a 96-well plate at $4\times10^3$ cells/well in 25 $\mu$l, and 80 $\mu$l of the culture supernatant of each hybridoma was added thereto, followed by culturing at 37°C overnight. Each well was observed with a microscope, and wells in which the cells agglutinated were visually determined. Hybridoma cells in the wells with cell agglutination were selected as positive clones.

Subsequently, secondary screening was carried out as follows. KMS-12-BM cells were reacted with the hybridoma culture supernatant, and the binding activity to the KMS-12-BM cells was analyzed by FACS. From the hybridomas, clones producing an antibody strongly binding to the KMS-12-BM cells were selected as positive clones.

The hybridoma cells in the selected wells were seeded again into a 96-well plate at 1 cell/well, followed by culturing for approximately 10 days. Then, the binding activities to the BaF3 cells and ICAM3/BaF3 were examined by FACS, and monoclones specifically reacting with ICAM3/BaF3 were selected. Thereby, hybridomas producing the anti-ICAM3 monoclonal antibody were established.

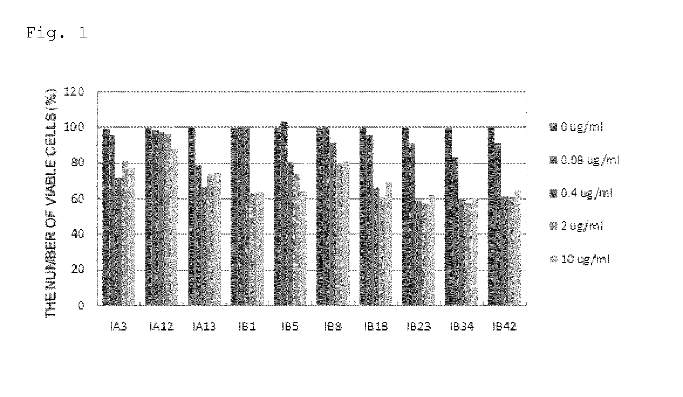

Among monoclonal antibodies obtained in this manner, a clone having the highest proliferation-suppressing activity was selected by the following procedure. First, the antibodies were purified from the hybridoma culture supernatant using Hi Trap Protein G HP 1 ml column (Amersham Biosciences #17-0404-01) in accordance with the attached instruction. Using the purified antibodies, the cell-proliferation-suppressing activity on KMS-12-BM cells was analyzed as follows. KMS-12-BM cells were seeded into a 96-well plate at $1\times10^4$ cells/well. The purified antibodies were added to each well at 0 to 10 $\mu$g/ml, followed by culturing for 6 days. Then, the number of viable cells was counted using Cell Count Reagent SF (nacalai) in accordance with the attached instruction. Based on this measurement result, a clone IB23 having the highest cell-proliferation-suppressing activity was selected (Fig. 1).

(Example 2) Comparison of Binding Activities of IB23 Antibody on Various Blood Cancer Lines

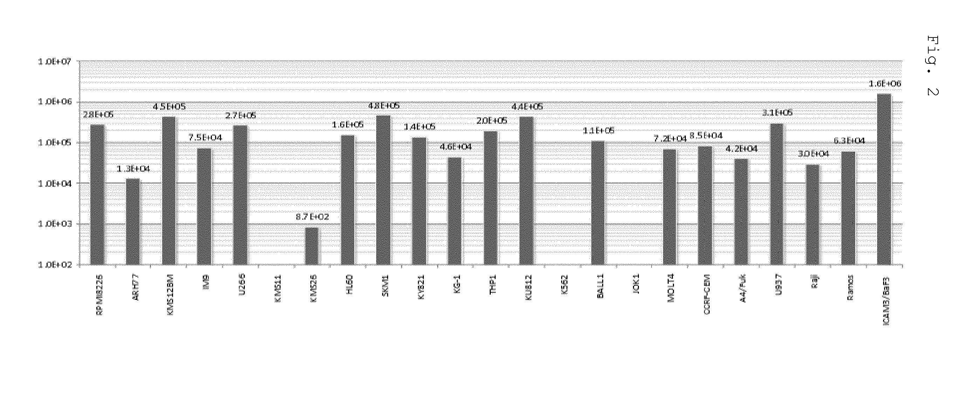

The number of cell surface ICAM3 antigens on various blood cancer lines was calculated based on a binding activity unit of the IB23 antibody. Table 1 shows a list of the blood cancer cell lines used in the measurement. The number of ICAM3 antigens (i.e., binding units of the IB23 antibody) on these cells was analyzed using QIFI KIT (manufactured by Dako, K0078). The various blood cancer cell lines were stained with a mouse anti-IB23 antibody (30 $\mu$g/ml), and the binding activity was measured by FACS analysis. Based on this binding activity (fluorescent intensity), the number of ICAM3 antigens was obtained by calculation in accordance with the attached instruction. Fig. 2 shows the result.

[Table 1]

| Call line | Cancer type | Origin |
|---|---|---|
| RPMI8226 | myeloma | Japan Health Sciences Foundation |
| ARH77 | myeloma | ATCC |
| KMS12BM | myeloma | Japan Health Sciences Foundation |
| IM9 | myeloma | ATCC |
| U266 | myeloma | ATCC |
| KMS11 | myeloma | Japan Health Sciences Foundation |
| KMS26 | myeloma | Japan Health Sciences Foundation |
| HL60 | AML | Japan Health Sciences Foundation |
| SKM1 | AML | Japan Health Sciences Foundation |
| KY821 | AML | Japan Health Sciences Foundation |
| KG-1 | AML | Japan Health Sciences Foundation |
| THP1 | AML | Japan Health Sciences Foundation |
| KU812 | CML | Japan Health Sciences Foundation |
| K562 | CML | Japan Health Sciences Foundation |
| BALL1 | B-ALL | Japan Health Sciences Foundation |
| JOK1 | B-CLL | Fujisaki Cell Center, Hayashibara Biochemical Labs., Inc. |
| MOLT4 | T-ALL | Japan Health Sciences Foundation |
| CCRF-CEM | T-ALL | Dainippon Pharmaceutical Co. Ltd. |
| A4/Fuk | lymphoma | Japan Health Sciences Foundation |
| U937 | lymphoma | Japan Health Sciences Foundation |
| Raji | lymphoma | Japan Health Sciences Foundation |
| Ramos | lymphoma. | Japan Health Sciences Foundation |
| ICAM3/BaF3 | Transfectant | Construted by yourselves |

(Example 3) Analysis on Cross-Reactivity with ICAM Family

ICAM3 has a high homology with ICAM1 and ICAM5 among the ICAM family molecules. Hence, the cross-reactivity with these family molecules was analysed.

A human ICAM1 (GenBank No. X06990) cDNA fragment (SEQ ID NO: 3) was amplified by PCR using human lung marathon-ready cDNA (takara, S0629) as a template, and a human ICAM5 (GenBank No. U72671) cDNA fragment (SEQ ID NO: 5) was amplified by PCR using human brain marathon-ready cDNA (takara, S0598) as a template. The products were introduced into expression vectors for animal cell. The base sequences were checked using BigDye Terminator Cycle Sequencing Kit (Applied Biosystems) and DNA sequencer ABI PRISM 3700 DNA Sequencer (Applied Biosystems) in accordance with the attached instructions. Each of the produced expression vectors was introduced into Ba/F3 cells. Thus, an ICAM1-expressing BaF3 transfectant (ICAM1/BaF3) and ICAM5-expressing BaF3 transfectant (ICAM5/BaF3) were established.

Next, using these cell lines thus established, the cross-reactivity of IB23 with the ICAM family was analyzed. Each 10 μg/ml of the IB23 antibody, an anti-human ICAM1 antibody (R&D Systems, Inc., #BBA3), and an anti-human ICAM5 antibody (R&D Systems, Inc., #MAB1950) were reacted with ICAM1/BaF3 and ICAM5/BaF3. Then, after staining with an anti-mouse IgG-FITC antibody (Beckman Coulter # IM0819), the binding of each antibody was analyzed by FACS (Becton, Dickinson and Company).

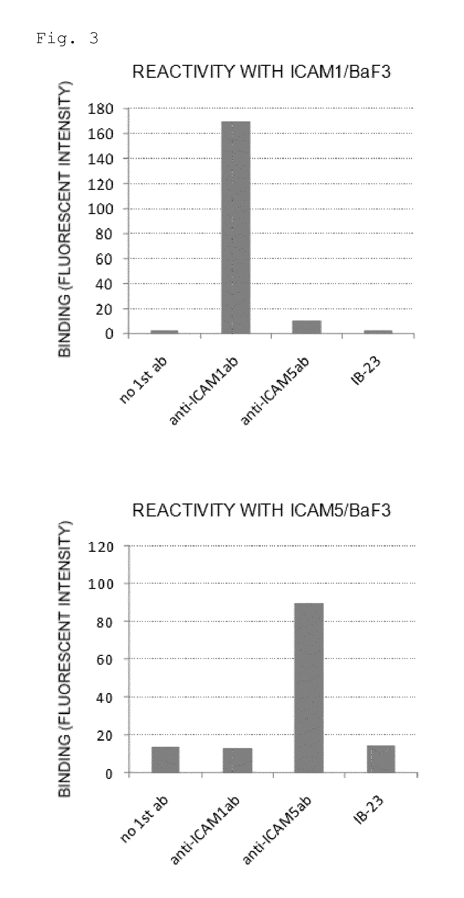

As a result, it was revealed that IB23 did not bind to ICAM1/BaF3 nor ICAM5/BaF3, but specifically reacted with ICAM3 (Fig. 3).

(Example 4) Determination of Variable Regions of Mouse Anti-ICAM3 Antibody: IB23

Total RNA was extracted from the hybridoma cells using RNeasy Mini Kits (QIAGEN), and a cDNA was synthesized with SMART RACE cDNA Amplification Kit (BD Biosciences). Using the produced cDNA as a template, DNAs corresponding to variable regions of the antibody were amplified by PCR. The obtained genes of the variable regions of the antibody were inserted into a cloning vector. The base sequence of each DNA fragment was determined using BigDye Terminator Cycle Sequencing Kit (Applied Biosystems) andDNAsequencer ABI PRISM 3700 DNA Sequencer (Applied Biosystems) in accordance with the attached instructions. The determined base sequence of the heavy chain variable region of the mouse IB23 antibody is shown in SEQ ID NO: 7, and that of the light chain variable region is shown in SEQ ID NO: 9. CDRs and FRs were determined according to Kabat numbering.

(Example 5) Production of IB23 Human Chimeric Antibody

A gene fragment obtained by linking the heavy chain variable region (SEQ ID NO: 7) of the mouse IB23 antibody to a heavy chain constant region (SEQ ID NO: 25) of human IgG1 was introduced into an expression vector for animal cell. Moreover, a gene fragment obtained by linking the light chain variable region (SEQ ID NO: 9) of the mouse IB23 antibody to a light chain constant region (SEQ ID NO: 27) of human IgG1 was introduced into an expression vector for animal cell. The antibody was expressed and purified by the method described in Reference Example 1 below.

(Example 6) Production of deglycosylated IB23 human chimeric antibody

The followings are known from literatures. If asparagine at position 297 in the Fc region of human IgG1 is substituted with alanine and deglycosylated, this weakens binding of effector cells to an Fc receptor. Thereby, the ADCC activity by the effector cells is lost. For this reason, a deglycosylated human chimeric antibody was produced from the IB23 antibody.

According to the literature information (Cancer Res 2008, 68, 9832-9838), a heavy chain constant region (CH_N297A) (SEQ ID NO: 29) was produced by substituting alanine for asparagine at position 297 in the EU numbering in the human IgG1 Fc region. The heavy chain variable region (SEQ ID NO: 7) of the mouse IB23 antibody was linked to CH_N297A to thereby form a heavy chain, which was then introduced into an expression vector for animal cell.

Moreover, the light chain variable region (SEQ ID NO: 9) of the mouse IB23 antibody was linked to the light chain constant region (SEQ ID NO: 27) of human IgG1 to thereby form a light chain, which was then introduced into an expression vector for animal cell. The antibody was expressed and purified by the method described in Reference Example 1 below.

(Example 7) Proliferation-Suppressing Activity of IB23 Antibody on Various Blood Cancer Lines

Using human blood cancer cell lines expressing ICAM3, the cell-proliferation-suppressing activity of the IB23 human chimeric IgG1 antibody was measured as described below.

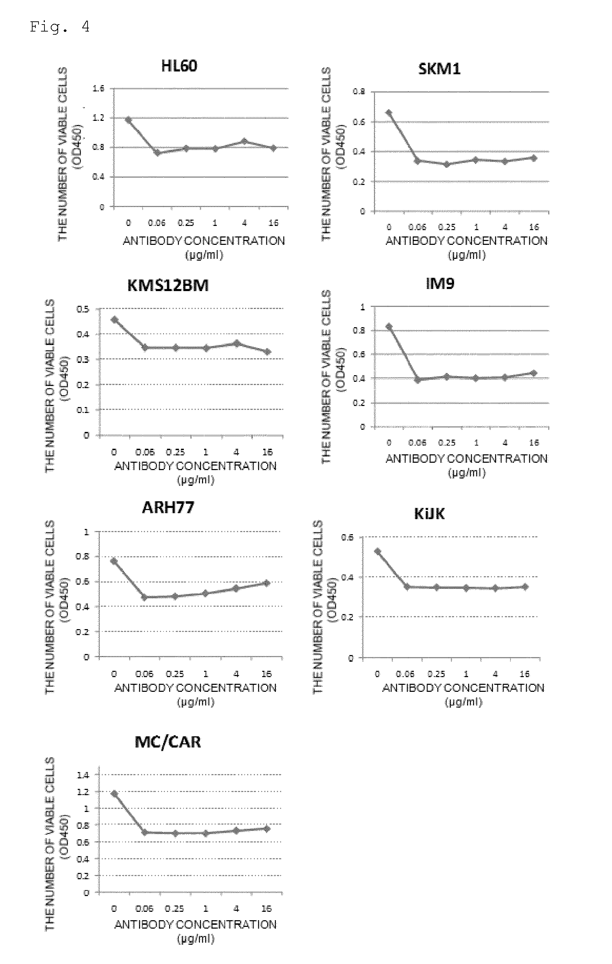

Various blood cancer cell lines were seeded into a 96-well plate at $3\times10^3$ to $6\times10^3$ cells/well to which the purified IB23 human chimeric antibody was added so that the final concentration was 0 to 16 $\mu$g/ml. After culturing for 4 days, the number of viable cells was counted using WST-8 (cell counting kit-8). The measurement was performed with n=3, and a mean value was calculated for each measurement result. As a result, as shown in Fig. 4, the IB23 human chimeric antibody exhibited a cell-proliferation-suppressing activity on the various blood cancer cell lines. It was revealed that the activity demonstrated a maximum proliferation-suppressing effect even at a concentration as low as 60 ng/ml. It can be seen from this result that the IB23 antibody alone is capable of suppressing cell proliferation quite considerably.

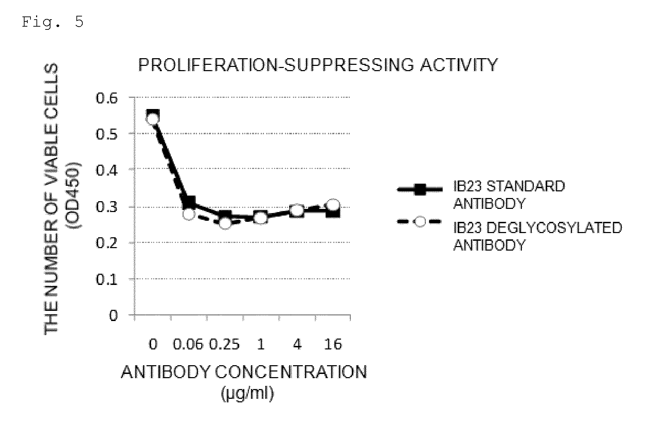

Next, it was examined whether or not a difference was observed between the IB23 human chimeric antibody and the deglycosylated IB23 human chimeric antibody in cell-proliferation-suppressing activity. SKM-1 cells were seeded into a 96-well plate at $3\times10^3$ cells/well. To such plates, each of the IB23 human chimeric antibody (standard antibody) and the deglycosylated IB23 human chimeric antibody was separately added in the same manner as the above. The number of viable cells was counted using WST8 (cell counting kit-8), and the proliferation-suppressing activity was compared between the two antibodies. As a result, it was verified that the proliferation-suppressing activity of even the deglycosylated type did not differ from that of the standard IB23 antibody (Fig. 5).

(Example 8) Analysis on ADCC Activity of IB23 Antibody

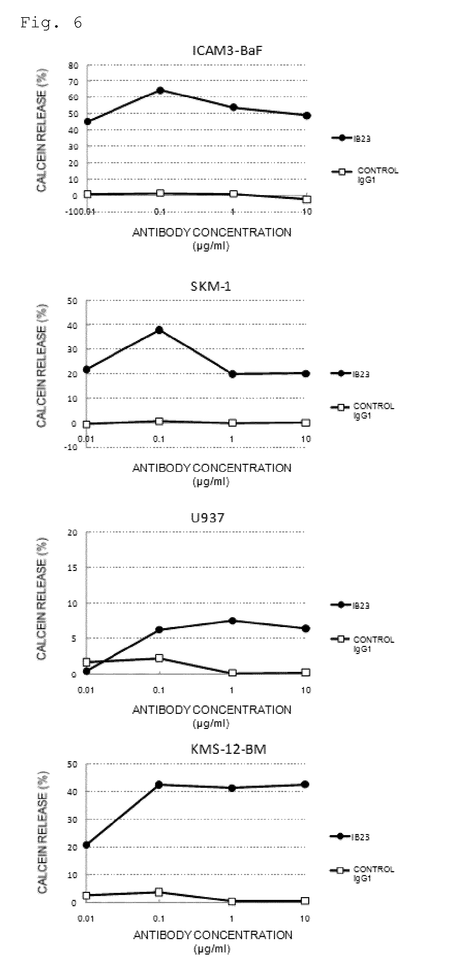

The ADCC activity of the IB23 standard antibody (human chimeric IgG1) on the blood cancer cell lines were analyzed. The ADCC activity was analyzed by an calcein release assay. Calcein AM (Wako Pure Chemical Industries, Ltd., 349-07201) was added to each of ICAM3/BaF3 cells, SKM-1 cells, U937 cells, and KMS-12-BM cells which had been respectively seeded into 96-well plates. By culturing for 90 minutes, calcein was incorporated into the cells. Then, the cells were washed with a culture solution and suspended in a fresh culture solution. Subsequently, the antibody was added thereto. Further, to each well, effector cells (recombinant cells of NK-92 [ATCC, CRL-2407] which were forced to express mouse Fc-gamma receptor 3 [NM_010188]) were added in an amount approximately 5 times as large as the target cell. Each of the plates was allowed to stand in a $5\%CO_2$ incubator at 37°C for 4 hours. After allowed to stand, the plate was centrifuged, and a certain amount of supernatant was collected from each well to measure the fluorescent intensity of calcein released into the medium by the cell injury ($\lambda$ex = 490 nm, $\lambda$em = 515 nm). As a result, as shown in Fig. 6, the IB23 standard antibody induced a quite high ADCC activity not only on the ICAM3/BaF3 but also on the blood cancer cell lines. This revealed that the IB23 antibody alone was capable of not only suppressing cell proliferation but also inducing the ADCC activity.

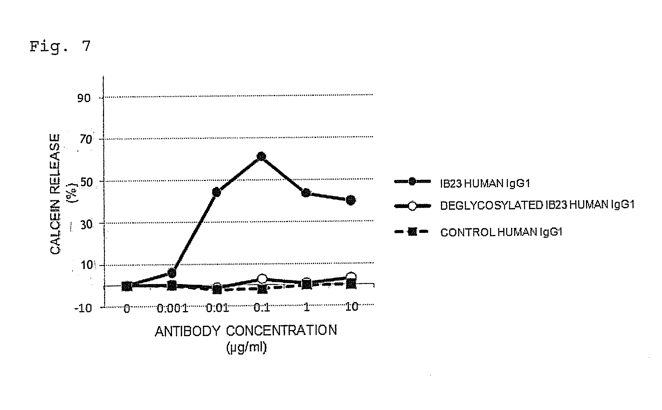

Next, the ADCC activity was compared between the IB23 standard antibody and the IB23 deglycosylated antibody and analyzed using ICAM3/BaF3 cells as a target cell. Fig. 7 shows the result. It was verified that a quite high ADCC activity was observed in the standard antibody, and that the deglycosylation of the antibody made the ADCC activity completely lost.

Note that the percentage of specific calcein released was calculated according to the equation: "percentage of specific calcein released (%)=(A-C)$\times$100/(B-C)." Here, A is the fluorescent value in each well, B is an average fluorescent value of the cells lysed and released into the medium with Nonidet P-40 having a final concentration of 1%, and C is an average fluorescent value when only the medium was added.

(Example 9) Analysis on In vivo Anti-Tumor Activity of IB23 Antibody

A human leukemia cell line, SKM-1 cells ($1\times10^7$ cells), was prepared in RPMI1640 (SIGMA Cat. No. R8758) and subcutaneously transplanted into the abdomen of SCID mice (Icr-scid Jcl, female, 6 weeks old, CLEA Japan, Inc.) to which 0.2 mg of an anti-asialo GM1 antibody (manufactured by Wako Pure Chemical Industries, Ltd.) had been intraperitoneally administered on the day before. The engraftment of tumor was checked, and the mice were grouped into 5 on day 23 after the transplantation according to the tumor volume and body weight (one control group, four drug-administered group, each n=6).

On the day of grouping (day 23) and on day 30, PBS was intravenously administered to the control group at 10 mL/kg, while the IB23 standard antibody and the IB23 deglycosylated antibody were intravenously administered to the drug administered groups at doses of 5 mg/kg and 25 mg/kg, respectively. Then, the tumor volume was measured over time, and plotted on a graph.

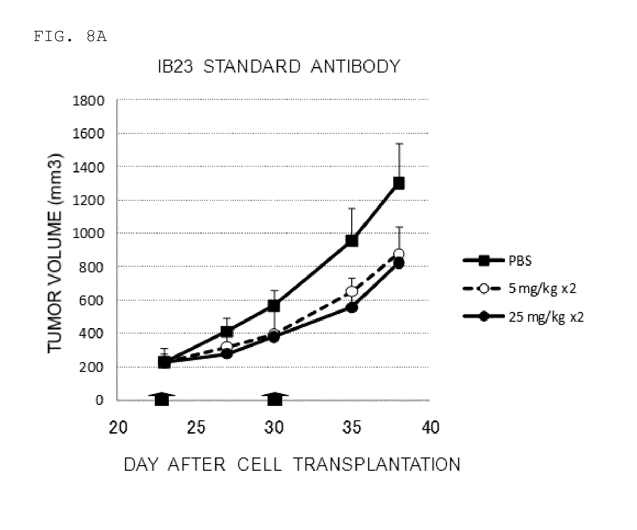

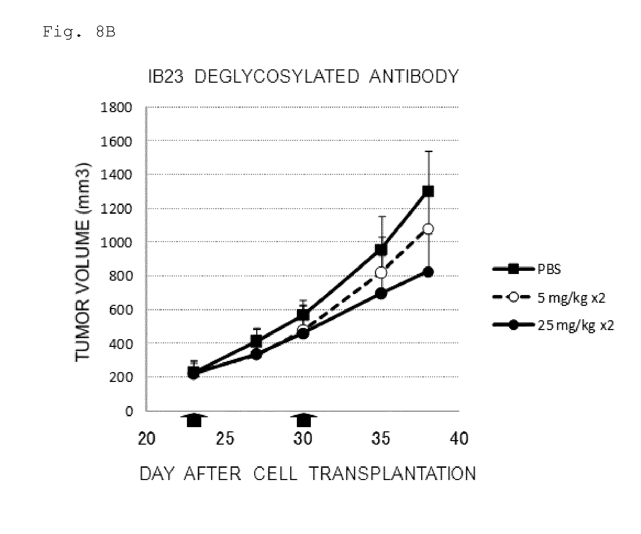

Fig. 8 shows the result. When the measurement was completed, the ratio of tumor proliferation suppressed by each dose of the IB23 standard antibody was 39% in the 5-mg/kg administered group and 44% in the 25-mg/kg administered group (Fig. 8A). Meanwhile, even by the administration of the IB23 deglycosylated antibody having no ADCC activity, the ratio of tumor proliferation suppressed was found to be 29% in the 5-mg/kg administered group and 43% in the 25-mg/kg administered group (Fig. 8B).

Since the tumor regression was observed by the IB23 deglycosylated antibody having no ADCC activity, it was verified that a sufficient anti-tumor action was satisfactorily exerted only by the proliferation-suppressing activity of the IB23 antibody. Moreover, since a superior drug action was observed in the standard antibody-administered groups, it was suggested that in addition to the proliferation-suppressing activity, the ADCC activity additively acted and enhanced the anti-tumor action of the IB23 antibody.

(Reference Example 1) Production of Expression Vector for Antibody, and Expression and Purification of the Antibody

Genes encoding the base sequences of heavy and light chains of the target antibody were obtained according to a method known to those skilled in the art using Assemble PCR and the like. Amino acid substitution was introduced using QuikChange Site-Directed Mutagenesis Kit (Stratagene), PCR, or the like according to a method known to those skilled in the art. Obtained plasmid fragments were inserted into expression vectors for animal cell to produce target heavy chain- and light chain-expression vectors. The base sequences of the obtained expression vectors were determined according to a method known to those skilled in the art. An antibody was expressed employing the following method. Each antibody expression vector, 15 $\mu$g, was cleaved with pvuI, and introduced into DG44 line (Invitrogen) by electroporation, which was seeded into a 96-well plate. After culturing in a CHO-S-SFM-II (Invitrogen) medium containing 500 $\mu$g/ml of G418 (Invitrogen), antibody production in the culture supernatant in a G418-resistance well was detected by ELISA. A clone observed to produce an antibody was further amplified, and the culture supernatant was collected and used for antibody purification. After cells were removed by centrifugation (approximately 2000 g, 5 minutes, room temperature), the culture supernatant was further passed through 0.22-$\mu$m filter MILLEX (R)-GV (Millipore). From the resultant culture supernatant, an antibody was purified using Hi Trap Protein G HP column (Amersham Biosciences #17-0404-01) according to a method known to those skilled in the art. The purified antibody was passed through a 0.22-$\mu$m filter (MILLIPORE #SLGV033RS) and used for analysis.

[Industrial Applicability]

An anti-ICAM3 antibody of the present invention is capable of exerting excellent proliferation-suppressing action and cytotoxic action on cells having ICAM3 expressed on surfaces thereof. Since exerting a strong action on various blood cancer cell lines, the anti-ICAM3 antibody of the present invention is particularly useful for treatment against leukemia, myeloma, malignant lymphoma, or the like. Moreover, because of the specificity, the anti-ICAM3 antibody of the present invention is applicable also to detection and screening of cells having ICAM3 on surfaces thereof.

[Sequence Listing Free Text]

SEQ ID NO: 11
<223> IB23_VH_FR1
SEQ ID NO: 12
<223> IB23_VH_FR2
SEQ ID NO: 13
<223> IB23_VH_FR3
SEQ ID NO: 14
<223> IB23_VH_FR4
SEQ ID NO: 15
<223> IB23_VH_CDR1
SEQ ID NO: 16
<223> IB23_VH_CDR2
SEQ ID NO: 17
<223> IB23_VH_CDR3
SEQ ID NO: 18
<223> IB23_VL_FR1
SEQ ID NO: 19
<223> IB23_VL_FR2
SEQ ID NO: 20
<223> IB23_VL_FR3
SEQ ID NO: 21
<223> IB23_VL_FR4
SEQ ID NO: 22
<223> IB23_VL_CDR1
SEQ ID NO: 23
<223> IB23_VL_CDR2
SEQ ID NO: 24
<223> IB23_VL_CDR3

## Claims

1. An anti-ICAM3 antibody having a cytotoxic activity.

2. The anti-ICAM3 antibody according to claim 1, wherein the cytotoxic activity is an ADCC activity.

3. The anti-ICAM3 antibody according to any one of claims 1 and 2, which further has a cell-proliferation-suppressing activity.

4. The anti-ICAM3 antibody according to any one of claims 1 to 3, which has an anti-tumor activity in vivo.

5. The anti-ICAM3 antibody according to any one of claims 1 to 4, which does not substantially bind to ICAM1 and/or ICAM5.

6. An antibody of any one of (a) to (c) below:

   (a) an antibody comprising heavy chain CDR1 having an amino acid sequence of SEQ ID NO: 15, heavy chain CDR2 having an amino acid sequence of SEQ ID NO: 16, heavy chain CDR3 having an amino acid sequence of SEQ ID NO: 17, light chain CDR1 having an amino acid sequence of SEQ ID NO: 22, CDR2 having an amino acid sequence of SEQ ID NO: 23, and light chain CDR3 having an amino acid sequence of SEQ ID NO: 24;
   (b) an antibody of (a) in which one or more amino acids are substituted, deleted, added and/or inserted, the antibody of (b) having an activity equivalent to that of the antibody of (a); and
   (c) an antibody which recognizes a same epitope as an epitope recognized by the antibody of any one of (a) and (b).

7. The anti-ICAM3 antibody according to any one of claims 1 to 6, comprising a human-derived constant region.

8. A DNA encoding the antibody according to any one of claims 1 to 7.

9. A pharmaceutical composition comprising the antibody according to any one of claims 1 to 7 as an active ingredient.

10. The pharmaceutical composition according to claim 9, which is an anti-cancer agent.

11. The pharmaceutical composition according to claim 10, wherein the cancer is leukemia, myeloma, or malignant lymphoma.

12. A diagnosis method for a cancer, **characterized by** detecting any one of an ICAM3 protein and a gene encoding the ICAM3 protein.

Fig. 1

Fig. 2

Fig. 3

REACTIVITY WITH ICAM1/BaF3

REACTIVITY WITH ICAM5/BaF3

Fig. 4

HL60

SKM1

KMS12BM

IM9

ARH77

KiJK

MC/CAR

Fig. 5

PROLIFERATION-SUPPRESSING ACTIVITY

THE NUMBER OF VIABLE CELLS (OD450)

ANTIBODY CONCENTRATION (μg/ml)

IB23 STANDARD ANTIBODY

IB23 DEGLYCOSYLATED ANTIBODY

Fig. 6

ICAM3-BaF

SKM-1

U937

KMS-12-BM

Fig. 7

FIG. 8A

IB23 STANDARD ANTIBODY

Fig. 8B

IB23 DEGLYCOSYLATED ANTIBODY

DAY AFTER CELL TRANSPLANTATION

## INTERNATIONAL SEARCH REPORT

| | International application No. |
|---|---|
| | PCT/JP2011/054368 |

### A. CLASSIFICATION OF SUBJECT MATTER

*C12N15/09*(2006.01)i, *A61K39/395*(2006.01)i, *A61P35/00*(2006.01)i, *A61P35/02*
(2006.01)i, *C07K16/28*(2006.01)i, *C12Q1/68*(2006.01)i, *G01N33/53*(2006.01)i

According to International Patent Classification (IPC) or to both national classification and IPC

### B. FIELDS SEARCHED

Minimum documentation searched (classification system followed by classification symbols)
C12N15/09, A61K39/395, A61P35/00, A61P35/02, C07K16/28, C12Q1/68,
G01N33/53

Documentation searched other than minimum documentation to the extent that such documents are included in the fields searched
| | | | |
|---|---|---|---|
| Jitsuyo Shinan Koho | 1922-1996 | Jitsuyo Shinan Toroku Koho | 1996-2011 |
| Kokai Jitsuyo Shinan Koho | 1971-2011 | Toroku Jitsuyo Shinan Koho | 1994-2011 |

Electronic data base consulted during the international search (name of data base and, where practicable, search terms used)
CA/REGISTRY(STN), BIOSIS/MEDLINE(STN), JSTPlus/JMEDPlus/JST7580(JDreamII),
PubMed, WPI

### C. DOCUMENTS CONSIDERED TO BE RELEVANT

| Category* | Citation of document, with indication, where appropriate, of the relevant passages | Relevant to claim No. |
|---|---|---|
| X | GAVILONDO, J. V. and LARRICK, J. W., Antibody Engineering at the Millennium., BioTechniques, 2000, Vol.29, No.1, P.128-149, page 147 | 1-11 |
| X | LIGOCKI, M. et al., ANTI-TUMOR ACTIVITY OF AN ICAM-3 ANTIBODY (ICM3) AGAINST HUMAN LEUKEMIC XENOGRAFT TUMORS IN NUDE MICE., Exp. Hematol., 2000, Vol.28, P.59-60, entire text | 1-11 |
| X | KIM, Y. G. et al., ICAM-3-induced cancer cell proliferation through the PI3K/Akt pathway., Cancer Lett., 2006, Vol.239, P.103-110, Summary, Materials and methods | 1-11 |

☒ Further documents are listed in the continuation of Box C.  ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 30 March, 2011 (30.03.11) | 12 April, 2011 (12.04.11) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.

PCT/JP2011/054368

| C (Continuation). DOCUMENTS CONSIDERED TO BE RELEVANT | | |
|---|---|---|
| Category* | Citation of document, with indication, where appropriate, of the relevant passages | Relevant to claim No. |
| X | ZECCHINON, L. et al., Bind another day: The LFA-1/ICAM-1 interaction as therapeutic target., Clin. Appl. Immunol. Rev., 2006, Vol.6, P.173-189, pages 175 to 176, '2.3. Intercellular adhesion molecule-3' | 1-11 |

Form PCT/ISA/210 (continuation of second sheet) (July 2009)

## INTERNATIONAL SEARCH REPORT

International application No.

PCT/JP2011/054368

| Box No. II | Observations where certain claims were found unsearchable (Continuation of item 2 of first sheet) |
|---|---|

This international search report has not been established in respect of certain claims under Article 17(2)(a) for the following reasons:

1. ☒ Claims Nos.: 12
   because they relate to subject matter not required to be searched by this Authority, namely:
   The claim pertains to a diagnostic method to be practiced on the human body and thus relates to a subject matter which this International Searching Authority is not required, under the provisions of PCT Article 17(2)(a)(i) and PCT Rule 39.1(iv), to search.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

| Box No. III | Observations where unity of invention is lacking (Continuation of item 3 of first sheet) |
|---|---|

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**
☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP HEI9295999 B
- WO 995434 A
- WO 0061739 A
- WO 0231140 A
- WO 0279255 A
- EP 239400 A
- WO 9602576 A
- EP 404097 A

- WO 9311161 A
- WO 03104453 A
- JP HEI159878 B
- WO 9425585 A
- WO 9312227 A
- WO 9203918 A
- WO 9402602 A
- WO 9411523 A


**Non-patent literature cited in the description**

- **VAZEUX R et al.** *Nature,* 1992, vol. 360, 485-488
- **FAWCETT J et al.** *Nature,* 1992, vol. 360, 481-484
- **DE FOUGEROLLES ; SPRONGER.** *J. Exp. Med.,* 1992, vol. 175, 185-190
- **VAN DER VIEREN M et al.** *Immunity,* 1995, vol. 3, 683-690
- **GREEN J. ; THOMPSON C.** *Cell Immunology,* 1996, vol. 171, 126-131
- **STUCKI A et al.** *Br J Haematol.,* 2000, vol. 108, 157-166
- **KESSEL J et al.** *J Allergy Clin Immunol.,* 2006, vol. 118, 831-836
- Current protocols in Immunology. Immunologic studies in humans. John Wiley & Sons, Inc, 1993
- **LANGONE J. J. et al.** *Methods in Enzymology,* 1983, vol. 93, 307-308
- *Nature Medicine,* 1996, vol. 2, 350-353
- **FULTON R. J. et al.** *J. Biol. Chem.,* 1986, vol. 261, 5314-5319
- **SIVAMG. et al.** *Cancer Res.,* 1987, vol. 47, 3169-3173
- **CUMBER A. J. et al.** *J. Immunol. Methods,* 1990, vol. 135, 15-24
- **WAWRZYNCZAK E. J. et al.** *Cancer Res.,* 1990, vol. 50, 7519-7562
- **GHEEITE V. et al.** *J. Immunol. Methods,* 1991, vol. 142, 223-230
- **THORPE P. E. et al.** *Cancer Res.,* 1987, vol. 47, 5924-5931
- **WAWRZYNCZAK E. J. et al.** *Br. J. Cancer,* 1992, vol. 66, 361-366
- **SIVAM G. et al.** *Cancer Res.,* 1987, vol. 47, 3169-3173
- **WAWRZYNCZAKE. J. et al.** *Cancer Res.,* 1990, vol. 50, 7519-7562

- **BOLOGNESI A. et al.** *Clin. exp. Immunol.,* 1992, vol. 89, 341-346
- **STIRPE F. ; BARBIERI L.** *FEBS letter,* 1986, vol. 195, 1-8
- **CASELLAS P. et al.** *Eur. J. Biochem.,* vol. 176, 581-588
- Antibodies A Laboratory Manual. Cold Spring Harbor Laboratory, 1988
- **SATO, K. et al.** *Cancer Res,* 1993, vol. 53, 851-856
- **CO, M. S. et al.** *J. Immunol.,* 1994, vol. 152, 2968-2976
- **BETTER, M. ; HORWITZ, A. H.** *Methods in Enzymology,* 1989, vol. 178, 476-496
- **PLUECKTHUN, A. ; SKERRA, A.** *Methods in Enzymology,* 1989, vol. 178, 497-515
- **LAMOYI, E.** *Methods in Enzymology,* 1986, vol. 121, 652-663
- **ROUSSEAUX, J. et al.** *Methods in Enzymology,* 1986, vol. 121, 663-669
- **BIRD, R. E. et al.** *TIBTECH,* 1991, vol. 9, 132-137
- **HOLLIGER P et al.** *Proc. Natl. Acad. Sci. USA,* 1993, vol. 90, 6444-6448
- **HUSTON, J. S. et al.** *Proc. Natl. Acad. Sci. U.S.A,* 1988, vol. 85, 5879-5883
- *Cellular & Molecular Immunology,* 2006, vol. 3, 439-443
- **HUDSON et al.** *J Immunol. Methods,* 1999, vol. 231, 177-189
- *Protein Engineering,* 1996, vol. 9 (3), 299-305
- **HASHIMOTO-GOTOH, T. et al.** *Gene,* 1995, vol. 152, 271-275
- **ZOLLER, MJ ; SMITH, M.** *Methods Enzymol.,* 1983, vol. 100, 468-500
- **KRAMER, W. et al.** *Nucleic Acids Res.,* 1984, vol. 12, 9441-9456

- **KRAMER W ; FRITZ HJ.** *Methods. Enzymol.,* 1987, vol. 154, 350-367
- **KUNKEL, TA.** *Proc Natl Acad Sci USA.,* 1985, vol. 82, 488-492
- **KUNKEL.** *Methods Enzymol.,* 1988, vol. 85, 2763-2766
- **MARK, D. F. et al.** *Proc. Natl. Acad. Sci. USA,* 1984, vol. 81, 5662-5666
- **ZOLLER, M. J. ; SMITH, M.** *Nucleic Acids Research,* 1982, vol. 10, 6487-6500
- **WANG, A. et al.** *Science,* vol. 224, 1431-1433
- **DALBADIE-MCFARLAND, G. et al.** *Proc. Natl. Acad. Sci. USA,* 1982, vol. 79, 6409-6413
- **SAMBROOK, J et al.** Molecular Cloning. Cold Spring Harbor Lab. press, 1989, 9.47-9.58
- *J. Immunol.,* 1979, vol. 123, 1548-1550
- *Current Topics in Microbiology and Immunology,* 1978, vol. 81, 1-7
- **KOHLER. G. ; MILSTEIN, C.** *Eur. J. Immunol.,* 1976, vol. 6, 511-519
- **MARGULIES. D. H. et al.** *Cell,* 1976, vol. 8, 405-415
- **SHULMAN, M. et al.** *Nature,* 1978, vol. 276, 269-270
- **DE ST. GROTH, S. F. et al.** *J. Immunol. Methods,* 1980, vol. 35, 1-21
- **TROWBRIDGE, I. S.** *J. Exp. Med.,* 1978, vol. 148, 313-323
- **GALFRE, G. et al.** *Nature,* 1979, vol. 277, 131-133
- **KOHLER. G. ; MILSTEIN, C.** *Methods Enzymol.,* 1981, vol. 73, 3-46
- **VANDAMME, A. M. et al.** *Eur. J. Biochem.,* 1990, vol. 192, 767-775
- **CHIRGWIN, J. M. et al.** *Biochemistry,* 1979, vol. 18, 5294-5299
- **CHOMCZYNSKI, P. et al.** *Anal. Biochem.,* 1987, vol. 162, 156-159
- **FROHMAN, M. A. et al.** *Proc. Natl. Acad. Sci. USA,* 1988, vol. 85, 8998-9002
- **BELYAVSKY, A. et al.** *Nucleic Acids Res.,* 1989, vol. 17, 2919-2932
- *J. Mol. Biol.,* 1991, vol. 222, 581-597
- **MULLIGAN et al.** *Nature,* 1979, vol. 277, 108
- **MIZUSHIMA et al.** *Nucleic Acids Res.,* 1990, vol. 18, 5322
- **WARD et al.** *Nature,* 1989, vol. 341, 544-546
- *FASEB J.,* 1992, vol. 6, 2422-2427
- **BETTER et al.** *Science,* 1988, vol. 240, 1041-1043
- **LEI, S. P. et al.** *J. Bacteriol.,* 1987, vol. 169, 4379
- **ED HARLOW ; DAVID LANE.** Antibodies A Laboratory Manual. Cold Spring Harbor Laboratory, 1988
- **EBERT, K. M. et al.** *Bio/Technology,* 1994, vol. 12, 699-702
- Remington's Pharmaceutical Science. Mark Publishing Company
- *Acta Oncol.,* 1993, vol. 32, 825-830
- *Cancer Res,* 2008, vol. 68, 9832-9838

SEQUENCE LISTING

```
<110>   FORERUNNER PHARMA RESEARCH CO., LTD.
<120>   ANTI ICAM3 ANTIBODY AND USE THEREOF
<130>   M022WO
<150>   JP 2010-041547
<151>   2010-02-26
<160>   30
<170>   PatentIn version 3.1
<210>   1
<211>   1644
<212>   DNA
<213>   Homo sapiens
<220>
<221>   CDS
<222>   (1)..(1641)
<400>   1
atg gcc acc atg gta cca tcc gtg ttg tgg ccc agg gcc tgc tgg act      48
Met Ala Thr Met Val Pro Ser Val Leu Trp Pro Arg Ala Cys Trp Thr
1               5                   10                  15
ctg ctg gtc tgc tgt ctg ctg acc cca ggt gtc cag ggg cag gag ttc      96
Leu Leu Val Cys Cys Leu Leu Thr Pro Gly Val Gln Gly Gln Glu Phe
                20                  25                  30
ctt ttg cgg gtg gag ccc cag aac cct gtg ctc tct gct gga ggg tcc     144
Leu Leu Arg Val Glu Pro Gln Asn Pro Val Leu Ser Ala Gly Gly Ser
            35                  40                  45
ctg ttt gtg aac tgc agt act gat tgt ccc agc tct gag aaa atc gcc     192
Leu Phe Val Asn Cys Ser Thr Asp Cys Pro Ser Ser Glu Lys Ile Ala
        50                  55                  60
ttg gag acg tcc cta tca aag gag ctg gtg gcc agt ggc atg ggc tgg     240
Leu Glu Thr Ser Leu Ser Lys Glu Leu Val Ala Ser Gly Met Gly Trp
65                  70                  75                  80
gca gcc ttc aat ctc agc aac gtg act ggc aac agt cgg atc ctc tgc     288
Ala Ala Phe Asn Leu Ser Asn Val Thr Gly Asn Ser Arg Ile Leu Cys
                85                  90                  95
tca gtg tac tgc aat ggc tct cag ata aca ggc tcc tct aac atc acc     336
Ser Val Tyr Cys Asn Gly Ser Gln Ile Thr Gly Ser Ser Asn Ile Thr
                100                 105                 110
gtg tac agg ctc ccg gag cgt gtg gag ctg gca ccc ctg cct cct tgg     384
Val Tyr Arg Leu Pro Glu Arg Val Glu Leu Ala Pro Leu Pro Pro Trp
            115                 120                 125
cag ccg gtg ggc cag aac ttc acc ctg cgc tgc caa gtg gag gat ggg     432
Gln Pro Val Gly Gln Asn Phe Thr Leu Arg Cys Gln Val Glu Asp Gly
            130                 135                 140
tcg ccc cgg acc agc ctc acg gtg gtg ctg ctt cgc tgg gag gag gag     480
Ser Pro Arg Thr Ser Leu Thr Val Val Leu Leu Arg Trp Glu Glu Glu
145                 150                 155                 160
ctg agc cgg cag ccc gca gtg gag gag cca gcg gag gtc act gcc act     528
Leu Ser Arg Gln Pro Ala Val Glu Glu Pro Ala Glu Val Thr Ala Thr
                165                 170                 175
gtg ctg gcc agc aga gac gac cac gga gcc cct ttc tca tgc cgc aca     576
Val Leu Ala Ser Arg Asp Asp His Gly Ala Pro Phe Ser Cys Arg Thr
                180                 185                 190
gaa ctg gac atg cag ccc cag ggg ctg gga ctg ttc gtg aac acc tca     624
Glu Leu Asp Met Gln Pro Gln Gly Leu Gly Leu Phe Val Asn Thr Ser
                195                 200                 205
gcc ccc cgc cag ctc cga acc ttt gtc ctg ccc gtg acc ccc ccg cgc     672
Ala Pro Arg Gln Leu Arg Thr Phe Val Leu Pro Val Thr Pro Pro Arg
            210                 215                 220
ctc gtg gcc ccc cgg ttc ttg gag gtg gaa acg tcg tgg ccg gtg gac     720
Leu Val Ala Pro Arg Phe Leu Glu Val Glu Thr Ser Trp Pro Val Asp
225                 230                 235                 240
tgc acc cta gac ggg ctt ttt cca gcc tca gag gcc cag gtc tac ctg     768
Cys Thr Leu Asp Gly Leu Phe Pro Ala Ser Glu Ala Gln Val Tyr Leu
```

```
                    245                     250                     255
gcg ctg ggg gac cag atg ctg aat gcg aca gtc atg aac cac ggg gac    816
Ala Leu Gly Asp Gln Met Leu Asn Ala Thr Val Met Asn His Gly Asp
            260                     265                     270
acg cta acg gcc aca gcc aca gcc acg gcg cgc gcg gat cag gag ggt    864
Thr Leu Thr Ala Thr Ala Thr Ala Thr Ala Arg Ala Asp Gln Glu Gly
            275                     280                     285
gcc cgg gag atc gtc tgc aac gtg acc cta ggg ggc gag aga cgg gag    912
Ala Arg Glu Ile Val Cys Asn Val Thr Leu Gly Gly Glu Arg Arg Glu
        290                     295                     300
gcc cgg gag aac ttg acg gtc ttt agc ttc cta gga ccc att gtg aac    960
Ala Arg Glu Asn Leu Thr Val Phe Ser Phe Leu Gly Pro Ile Val Asn
305                     310                     315                 320
ctc agc gag ccc acc gcc cat gag ggg tcc aca gtg acc gtg agt tgc    1008
Leu Ser Glu Pro Thr Ala His Glu Gly Ser Thr Val Thr Val Ser Cys
                    325                     330                     335
atg gct ggg gct cga gtc cag gtc acg ctg gac gga gtt ccg gcc gcg    1056
Met Ala Gly Ala Arg Val Gln Val Thr Leu Asp Gly Val Pro Ala Ala
            340                     345                     350
gcc ccg ggg cag cca gct caa ctt cag cta aat gct acc gag agt gac    1104
Ala Pro Gly Gln Pro Ala Gln Leu Gln Leu Asn Ala Thr Glu Ser Asp
            355                     360                     365
gac gga cgc agc ttc ttc tgc agt gcc act ctc gag gtg gac ggc gag    1152
Asp Gly Arg Ser Phe Phe Cys Ser Ala Thr Leu Glu Val Asp Gly Glu
            370                     375                     380
ttc ttg cac agg aac agt agc gtc cag ctg cga gtc ctg tat ggt ccc    1200
Phe Leu His Arg Asn Ser Ser Val Gln Leu Arg Val Leu Tyr Gly Pro
385                     390                     395                 400
aaa att gac cga gcc aca tgc ccc cag cac ttg aaa tgg aaa gat aaa    1248
Lys Ile Asp Arg Ala Thr Cys Pro Gln His Leu Lys Trp Lys Asp Lys
                    405                     410                     415
acg aga cac gtc ctg cag tgc caa gcc agg ggc aac ccg tac ccc gag    1296
Thr Arg His Val Leu Gln Cys Gln Ala Arg Gly Asn Pro Tyr Pro Glu
            420                     425                     430
ctg cgg tgt ttg aag gaa ggc tcc agc cgg gag gtg ccg gtg ggg atc    1344
Leu Arg Cys Leu Lys Glu Gly Ser Ser Arg Glu Val Pro Val Gly Ile
            435                     440                     445
ccg ttc ttc gtc aac gta aca cat aat ggt act tat cag tgc caa gcg    1392
Pro Phe Phe Val Asn Val Thr His Asn Gly Thr Tyr Gln Cys Gln Ala
            450                     455                     460
tcc agc tca cga ggc aaa tac acc ctg gtc gtg gtg atg gac att gag    1440
Ser Ser Ser Arg Gly Lys Tyr Thr Leu Val Val Val Met Asp Ile Glu
465                     470                     475                 480
gct ggg agc tcc cac ttt gtc ccc gtc ttc gtg gcg gtg tta ctg acc    1488
Ala Gly Ser Ser His Phe Val Pro Val Phe Val Ala Val Leu Leu Thr
            485                     490                     495
ctg ggc gtg gtg act atc gta ctg gcc tta atg tac gtc ttc agg gag    1536
Leu Gly Val Val Thr Ile Val Leu Ala Leu Met Tyr Val Phe Arg Glu
            500                     505                     510
cac caa cgg agc ggc agt tac cat gtt agg gag gag agc acc tat ctg    1584
His Gln Arg Ser Gly Ser Tyr His Val Arg Glu Glu Ser Thr Tyr Leu
            515                     520                     525
ccc ctc acg tct atg cag ccg aca gaa gca atg ggg gaa gaa ccg tcc    1632
Pro Leu Thr Ser Met Gln Pro Thr Glu Ala Met Gly Glu Glu Pro Ser
530                     535                     540
aga gct gag tga                                                     1644
Arg Ala Glu
545
```

```
<210>   2
<211>   547
<212>   PRT
<213>   Homo sapiens
<400>   2
```

```
Met Ala Thr Met Val Pro Ser Val Leu Trp Pro Arg Ala Cys Trp Thr
1               5                   10                  15
Leu Leu Val Cys Cys Leu Leu Thr Pro Gly Val Gln Gly Gln Glu Phe
            20              25                  30
Leu Leu Arg Val Glu Pro Gln Asn Pro Val Leu Ser Ala Gly Gly Ser
        35              40                  45
Leu Phe Val Asn Cys Ser Thr Asp Cys Pro Ser Ser Glu Lys Ile Ala
    50              55                  60
Leu Glu Thr Ser Leu Ser Lys Glu Leu Val Ala Ser Gly Met Gly Trp
65              70                  75                  80
Ala Ala Phe Asn Leu Ser Asn Val Thr Gly Asn Ser Arg Ile Leu Cys
            85                  90                  95
Ser Val Tyr Cys Asn Gly Ser Gln Ile Thr Gly Ser Ser Asn Ile Thr
        100             105                 110
Val Tyr Arg Leu Pro Glu Arg Val Glu Leu Ala Pro Leu Pro Pro Trp
    115             120                 125
Gln Pro Val Gly Gln Asn Phe Thr Leu Arg Cys Gln Val Glu Asp Gly
    130             135                 140
Ser Pro Arg Thr Ser Leu Thr Val Val Leu Leu Arg Trp Glu Glu Glu
145             150                 155                 160
Leu Ser Arg Gln Pro Ala Val Glu Glu Pro Ala Glu Val Thr Ala Thr
        165                 170                 175
Val Leu Ala Ser Arg Asp Asp His Gly Ala Pro Phe Ser Cys Arg Thr
        180                 185                 190
Glu Leu Asp Met Gln Pro Gln Gly Leu Gly Leu Phe Val Asn Thr Ser
        195                 200                 205
Ala Pro Arg Gln Leu Arg Thr Phe Val Leu Pro Val Thr Pro Pro Arg
    210                 215                 220
Leu Val Ala Pro Arg Phe Leu Glu Val Glu Thr Ser Trp Pro Val Asp
225                 230                 235                 240
Cys Thr Leu Asp Gly Leu Phe Pro Ala Ser Glu Ala Gln Val Tyr Leu
            245                 250                 255
Ala Leu Gly Asp Gln Met Leu Asn Ala Thr Val Met Asn His Gly Asp
            260                 265                 270
Thr Leu Thr Ala Thr Ala Thr Ala Thr Ala Arg Ala Asp Gln Glu Gly
        275                 280                 285
Ala Arg Glu Ile Val Cys Asn Val Thr Leu Gly Gly Glu Arg Arg Glu
    290                 295                 300
Ala Arg Glu Asn Leu Thr Val Phe Ser Phe Leu Gly Pro Ile Val Asn
305             310                 315                 320
Leu Ser Glu Pro Thr Ala His Glu Gly Ser Thr Val Thr Val Ser Cys
            325                 330                 335
Met Ala Gly Ala Arg Val Gln Val Thr Leu Asp Gly Val Pro Ala Ala
        340                 345                 350
Ala Pro Gly Gln Pro Ala Gln Leu Gln Leu Asn Ala Thr Glu Ser Asp
        355                 360                 365
Asp Gly Arg Ser Phe Phe Cys Ser Ala Thr Leu Glu Val Asp Gly Glu
370                 375                 380
Phe Leu His Arg Asn Ser Ser Val Gln Leu Arg Val Leu Tyr Gly Pro
385                 390                 395                 400
Lys Ile Asp Arg Ala Thr Cys Pro Gln His Leu Lys Trp Lys Asp Lys
            405                 410                 415
Thr Arg His Val Leu Gln Cys Gln Ala Arg Gly Asn Pro Tyr Pro Glu
        420                 425                 430
Leu Arg Cys Leu Lys Glu Gly Ser Ser Arg Glu Val Pro Val Gly Ile
        435                 440                 445
Pro Phe Phe Val Asn Val Thr His Asn Gly Thr Tyr Gln Cys Gln Ala
    450                 455                 460
Ser Ser Ser Arg Gly Lys Tyr Thr Leu Val Val Val Met Asp Ile Glu
465             470                 475                 480
Ala Gly Ser Ser His Phe Val Pro Val Phe Val Ala Val Leu Leu Thr
            485                 490                 495
Leu Gly Val Val Thr Ile Val Leu Ala Leu Met Tyr Val Phe Arg Glu
        500                 505                 510
His Gln Arg Ser Gly Ser Tyr His Val Arg Glu Glu Ser Thr Tyr Leu
```

```
                515                 520                 525
Pro Leu Thr Ser Met Gln Pro Thr Glu Ala Met Gly Glu Glu Pro Ser
        530                 535                 540
Arg Ala Glu
545


<210>  3
<211>  1599
<212>  DNA
<213>  Homo sapiens
<220>
<221>  CDS
<222>  (1)..(1596)
<400>  3
atg gct ccc agc agc ccc cgg ccc gcg ctg ccc gca ctc ctg gtc ctg       48
Met Ala Pro Ser Ser Pro Arg Pro Ala Leu Pro Ala Leu Leu Val Leu
1               5                   10                  15
ctc ggg gct ctg ttc cca gga cct ggc aat gcc cag aca tct gtg tcc       96
Leu Gly Ala Leu Phe Pro Gly Pro Gly Asn Ala Gln Thr Ser Val Ser
                20                  25                  30
ccc tca aaa gtc atc ctg ccc cgg gga ggc tcc gtg ctg gtg aca tgc      144
Pro Ser Lys Val Ile Leu Pro Arg Gly Gly Ser Val Leu Val Thr Cys
            35                  40                  45
agc acc tcc tgt gac cag ccc aag ttg ttg ggc ata gag acc ccg ttg      192
Ser Thr Ser Cys Asp Gln Pro Lys Leu Leu Gly Ile Glu Thr Pro Leu
        50                  55                  60
cct aaa aag gag ttg ctc ctg cct ggg aac aac cgg aag gtg tat gaa      240
Pro Lys Lys Glu Leu Leu Leu Pro Gly Asn Asn Arg Lys Val Tyr Glu
65                  70                  75                  80
ctg agc aat gtg caa gaa gat agc caa cca atg tgc tat tca aac tgc      288
Leu Ser Asn Val Gln Glu Asp Ser Gln Pro Met Cys Tyr Ser Asn Cys
                85                  90                  95
cct gat ggg cag tca aca gct aaa acc ttc ctc acc gtg tac tgg act      336
Pro Asp Gly Gln Ser Thr Ala Lys Thr Phe Leu Thr Val Tyr Trp Thr
            100                 105                 110
cca gaa cgg gtg gaa ctg gca ccc ctc ccc tct tgg cag cca gtg ggc      384
Pro Glu Arg Val Glu Leu Ala Pro Leu Pro Ser Trp Gln Pro Val Gly
            115                 120                 125
aag aac ctt acc cta cgc tgc cag gtg gag ggt ggg gca ccc cgg gcc      432
Lys Asn Leu Thr Leu Arg Cys Gln Val Glu Gly Gly Ala Pro Arg Ala
        130                 135                 140
aac ctc acc gtg gtg ctg ctc cgt ggg gag aag gag ctg aaa cgg gag      480
Asn Leu Thr Val Val Leu Leu Arg Gly Glu Lys Glu Leu Lys Arg Glu
145                 150                 155                 160
cca gct gtg ggg gag ccc gct gag gtc acg acc acg gtg ctg gtg agg      528
Pro Ala Val Gly Glu Pro Ala Glu Val Thr Thr Thr Val Leu Val Arg
                165                 170                 175
aga gat cac cat gga gcc aat ttc tcg tgc cgc act gaa ctg gac ctg      576
Arg Asp His His Gly Ala Asn Phe Ser Cys Arg Thr Glu Leu Asp Leu
            180                 185                 190
cgg ccc caa ggg ctg gag ctg ttt gag aac acc tcg gcc ccc tac cag      624
Arg Pro Gln Gly Leu Glu Leu Phe Glu Asn Thr Ser Ala Pro Tyr Gln
            195                 200                 205
ctc cag acc ttt gtc ctg cca gcg act ccc cca caa ctt gtc agc ccc      672
Leu Gln Thr Phe Val Leu Pro Ala Thr Pro Pro Gln Leu Val Ser Pro
        210                 215                 220
cgg gtc cta gag gtg gac acg cag ggg acc gtg gtc tgt tcc ctg gac      720
Arg Val Leu Glu Val Asp Thr Gln Gly Thr Val Val Cys Ser Leu Asp
225                 230                 235                 240
ggg ctg ttc cca gtc tcg gag gcc cag gtc cac ctg gca ctg ggg gac      768
Gly Leu Phe Pro Val Ser Glu Ala Gln Val His Leu Ala Leu Gly Asp
                245                 250                 255
cag agg ttg aac ccc aca gtc acc tat ggc aac gac tcc ttc tcg gcc      816
Gln Arg Leu Asn Pro Thr Val Thr Tyr Gly Asn Asp Ser Phe Ser Ala
```

```
                 260                      265                      270
aag gcc tca gtc agt gtg acc gca gag gac gag ggc acc cag cgg ctg       864
Lys Ala Ser Val Ser Val Thr Ala Glu Asp Glu Gly Thr Gln Arg Leu
        275                      280                      285
acg tgt gca gta ata ctg ggg aac cag agc cag gag aca ctg cag aca       912
Thr Cys Ala Val Ile Leu Gly Asn Gln Ser Gln Glu Thr Leu Gln Thr
        290                      295                      300
gtg acc atc tac agc ttt ccg gcg ccc aac gtg att ctg acg aag cca       960
Val Thr Ile Tyr Ser Phe Pro Ala Pro Asn Val Ile Leu Thr Lys Pro
305                      310                      315                  320
gag gtc tca gaa ggg acc gag gtg aca gtg aag tgt gag gcc cac cct      1008
Glu Val Ser Glu Gly Thr Glu Val Thr Val Lys Cys Glu Ala His Pro
                         325                      330                  335
aga gcc aag gtg acg ctg aat ggg gtt cca gcc cag cca ctg ggc ccg      1056
Arg Ala Lys Val Thr Leu Asn Gly Val Pro Ala Gln Pro Leu Gly Pro
                340                      345                      350
agg gcc cag ctc ctg ctg aag gcc acc cca gag gac aac ggg cgc agc      1104
Arg Ala Gln Leu Leu Leu Lys Ala Thr Pro Glu Asp Asn Gly Arg Ser
        355                      360                      365
ttc tcc tgc tct gca acc ctg gag gtg gcc ggc cag ctt ata cac aag      1152
Phe Ser Cys Ser Ala Thr Leu Glu Val Ala Gly Gln Leu Ile His Lys
        370                      375                      380
aac cag acc cgg gag ctt cgt gtc ctg tat ggc ccc cga ctg gac gag      1200
Asn Gln Thr Arg Glu Leu Arg Val Leu Tyr Gly Pro Arg Leu Asp Glu
385                      390                      395                  400
agg gat tgt ccg gga aac tgg acg tgg cca gaa aat tcc cag cag act      1248
Arg Asp Cys Pro Gly Asn Trp Thr Trp Pro Glu Asn Ser Gln Gln Thr
                405                      410                      415
cca atg tgc cag gct tgg ggg aac cca ttg ccc gag ctc aag tgt cta      1296
Pro Met Cys Gln Ala Trp Gly Asn Pro Leu Pro Glu Leu Lys Cys Leu
                420                      425                      430
aag gat ggc act ttc cca ctg ccc atc ggg gaa tca gtg act gtc act      1344
Lys Asp Gly Thr Phe Pro Leu Pro Ile Gly Glu Ser Val Thr Val Thr
                435                      440                      445
cga gat ctt gag ggc acc tac ctc tgt cgg gcc agg agc act caa ggg      1392
Arg Asp Leu Glu Gly Thr Tyr Leu Cys Arg Ala Arg Ser Thr Gln Gly
        450                      455                      460
gag gtc acc cgc gag gtg acc gtg aat gtg ctc tcc ccc cgg tat gag      1440
Glu Val Thr Arg Glu Val Thr Val Asn Val Leu Ser Pro Arg Tyr Glu
465                      470                      475                  480
att gtc atc atc act gtg gta gca gcc gca gtc ata atg ggc act gca      1488
Ile Val Ile Ile Thr Val Val Ala Ala Ala Val Ile Met Gly Thr Ala
                485                      490                      495
ggc ctc agc acg tac ctc tat aac cgc cag cgg aag atc aag aaa tac      1536
Gly Leu Ser Thr Tyr Leu Tyr Asn Arg Gln Arg Lys Ile Lys Lys Tyr
                500                      505                      510
aga cta caa cag gcc caa aaa ggg acc ccc atg aaa ccg aac aca caa      1584
Arg Leu Gln Gln Ala Gln Lys Gly Thr Pro Met Lys Pro Asn Thr Gln
        515                      520                      525
gcc acg cct ccc tga                                                   1599
Ala Thr Pro Pro
        530
```

```
<210>   4
<211>   532
<212>   PRT
<213>   Homo sapiens
<400>   4
Met Ala Pro Ser Ser Pro Arg Pro Ala Leu Pro Ala Leu Leu Val Leu
1               5                   10                  15
Leu Gly Ala Leu Phe Pro Gly Pro Gly Asn Ala Gln Thr Ser Val Ser
                20                  25                  30
Pro Ser Lys Val Ile Leu Pro Arg Gly Gly Ser Val Leu Val Thr Cys
        35                  40                  45
```

```
Ser Thr Ser Cys Asp Gln Pro Lys Leu Leu Gly Ile Glu Thr Pro Leu
    50                  55                  60
Pro Lys Lys Glu Leu Leu Leu Pro Gly Asn Asn Arg Lys Val Tyr Glu
65              70                  75                      80
Leu Ser Asn Val Gln Glu Asp Ser Gln Pro Met Cys Tyr Ser Asn Cys
                85                  90                  95
Pro Asp Gly Gln Ser Thr Ala Lys Thr Phe Leu Thr Val Tyr Trp Thr
            100             105                 110
Pro Glu Arg Val Glu Leu Ala Pro Leu Pro Ser Trp Gln Pro Val Gly
        115                 120                 125
Lys Asn Leu Thr Leu Arg Cys Gln Val Glu Gly Gly Ala Pro Arg Ala
    130             135                 140
Asn Leu Thr Val Val Leu Leu Arg Gly Glu Lys Glu Leu Lys Arg Glu
145             150                 155                     160
Pro Ala Val Gly Glu Pro Ala Glu Val Thr Thr Thr Val Leu Val Arg
                165                 170                 175
Arg Asp His His Gly Ala Asn Phe Ser Cys Arg Thr Glu Leu Asp Leu
            180                 185                 190
Arg Pro Gln Gly Leu Glu Leu Phe Glu Asn Thr Ser Ala Pro Tyr Gln
        195                 200                 205
Leu Gln Thr Phe Val Leu Pro Ala Thr Pro Pro Gln Leu Val Ser Pro
    210                 215                 220
Arg Val Leu Glu Val Asp Thr Gln Gly Thr Val Val Cys Ser Leu Asp
225             230                 235                     240
Gly Leu Phe Pro Val Ser Glu Ala Gln Val His Leu Ala Leu Gly Asp
                245                 250                 255
Gln Arg Leu Asn Pro Thr Val Thr Tyr Gly Asn Asp Ser Phe Ser Ala
            260                 265                 270
Lys Ala Ser Val Ser Val Thr Ala Glu Asp Glu Gly Thr Gln Arg Leu
        275                 280                 285
Thr Cys Ala Val Ile Leu Gly Asn Gln Ser Gln Glu Thr Leu Gln Thr
    290                 295                 300
Val Thr Ile Tyr Ser Phe Pro Ala Pro Asn Val Ile Leu Thr Lys Pro
305             310                 315                     320
Glu Val Ser Glu Gly Thr Glu Val Thr Val Lys Cys Glu Ala His Pro
                325                 330                 335
Arg Ala Lys Val Thr Leu Asn Gly Val Pro Ala Gln Pro Leu Gly Pro
            340                 345                 350
Arg Ala Gln Leu Leu Leu Lys Ala Thr Pro Glu Asp Asn Gly Arg Ser
        355                 360                 365
Phe Ser Cys Ser Ala Thr Leu Glu Val Ala Gly Gln Leu Ile His Lys
370             375                 380
Asn Gln Thr Arg Glu Leu Arg Val Leu Tyr Gly Pro Arg Leu Asp Glu
385             390                 395                     400
Arg Asp Cys Pro Gly Asn Trp Thr Trp Pro Glu Asn Ser Gln Gln Thr
            405                 410                 415
Pro Met Cys Gln Ala Trp Gly Asn Pro Leu Pro Glu Leu Lys Cys Leu
        420                 425                 430
Lys Asp Gly Thr Phe Pro Leu Pro Ile Gly Glu Ser Val Thr Val Thr
    435                 440                 445
Arg Asp Leu Glu Gly Thr Tyr Leu Cys Arg Ala Arg Ser Thr Gln Gly
    450                 455                 460
Glu Val Thr Arg Glu Val Thr Val Asn Val Leu Ser Pro Arg Tyr Glu
465             470                 475                     480
Ile Val Ile Ile Thr Val Val Ala Ala Ala Val Ile Met Gly Thr Ala
                485                 490                 495
Gly Leu Ser Thr Tyr Leu Tyr Asn Arg Gln Arg Lys Ile Lys Lys Tyr
            500                 505                 510
Arg Leu Gln Gln Ala Gln Lys Gly Thr Pro Met Lys Pro Asn Thr Gln
    515                 520                 525
Ala Thr Pro Pro
    530
```

<210> 5

<211> 2775
<212> DNA
<213> Homo sapiens
<220>
<221> CDS
<222> (1)..(2772)
<400> 5

```
atg cca ggg cct tcg cca ggg ctg cgc cgg gcg cta ctc ggc ctc tgg      48
Met Pro Gly Pro Ser Pro Gly Leu Arg Arg Ala Leu Leu Gly Leu Trp
1               5                   10                  15

gct gct ctg ggc ctg ggg ctc ttc ggc ctc tca gcg gtc tcg cag gag      96
Ala Ala Leu Gly Leu Gly Leu Phe Gly Leu Ser Ala Val Ser Gln Glu
            20                  25                  30

ccc ttc tgg gcg gac ctg cag cct cgc gtg gcg ttc gtg gag cgc ggg     144
Pro Phe Trp Ala Asp Leu Gln Pro Arg Val Ala Phe Val Glu Arg Gly
        35                  40                  45

ggc tcg ctg tgg ctg aat tgc agc acc aac tgc cct cgg ccg gag cgc     192
Gly Ser Leu Trp Leu Asn Cys Ser Thr Asn Cys Pro Arg Pro Glu Arg
    50                  55                  60

ggt ggc ctg gag acc tcg ctg cgc cga aac ggg acc cag agg ggt ttg     240
Gly Gly Leu Glu Thr Ser Leu Arg Arg Asn Gly Thr Gln Arg Gly Leu
65                  70                  75                  80

cgt tgg ttg gcg cgg cag ctg gtg gac att cgc gag ccg gag act cag     288
Arg Trp Leu Ala Arg Gln Leu Val Asp Ile Arg Glu Pro Glu Thr Gln
                85                  90                  95

ccc gtc tgc ttc ttc cgc tgc gcg cgg cgc aca cta cag gcg cgt ggg     336
Pro Val Cys Phe Phe Arg Cys Ala Arg Arg Thr Leu Gln Ala Arg Gly
            100                 105                 110

ctc att cgc act ttc cag cga cca gat cgc gta gag ctg atg ccg ctg     384
Leu Ile Arg Thr Phe Gln Arg Pro Asp Arg Val Glu Leu Met Pro Leu
        115                 120                 125

cct ccc tgg cag ccg gtg ggc gag aac ttc acc ctg agc tgt agg gtc     432
Pro Pro Trp Gln Pro Val Gly Glu Asn Phe Thr Leu Ser Cys Arg Val
        130                 135                 140

ccc ggc gcc ggg ccc cgt gcg agc ctc acg ctg acc ctg ctg cgg ggc     480
Pro Gly Ala Gly Pro Arg Ala Ser Leu Thr Leu Thr Leu Leu Arg Gly
145                 150                 155                 160

gcc cag gag ctg atc cgc cgc agc ttc gcc ggt gaa cca ccc cga gcg     528
Ala Gln Glu Leu Ile Arg Arg Ser Phe Ala Gly Glu Pro Pro Arg Ala
                165                 170                 175

cgg ggc gcg gtg ctc aca gcc acg gta ctg gct cgg agg gag gac cat     576
Arg Gly Ala Val Leu Thr Ala Thr Val Leu Ala Arg Arg Glu Asp His
            180                 185                 190

gga gcc aat ttc tcg tgt cgc gcc gag ctg gac ctg cgg ccg cac gga     624
Gly Ala Asn Phe Ser Cys Arg Ala Glu Leu Asp Leu Arg Pro His Gly
        195                 200                 205

ctg gga ctg ttt gaa aac agc tcg gcc ccc aga gag ctc cga acc ttc     672
Leu Gly Leu Phe Glu Asn Ser Ser Ala Pro Arg Glu Leu Arg Thr Phe
        210                 215                 220

tcc ctg tct ccg gat gcc ccg cgc ctc gct gct ccc cgg ctc ttg gaa     720
Ser Leu Ser Pro Asp Ala Pro Arg Leu Ala Ala Pro Arg Leu Leu Glu
225                 230                 235                 240

gtt ggc tcg gaa agg ccc gtg agc tgc act ctg gac gga ctg ttt cca     768
Val Gly Ser Glu Arg Pro Val Ser Cys Thr Leu Asp Gly Leu Phe Pro
                245                 250                 255

gcc tca gag gcc agg gtc tac ctc gca ctg ggg gac cag aat ctg agt     816
Ala Ser Glu Ala Arg Val Tyr Leu Ala Leu Gly Asp Gln Asn Leu Ser
            260                 265                 270

cct gat gtc acc ctc gaa ggg gac gca ttc gtg gcc act gcc aca gcc     864
Pro Asp Val Thr Leu Glu Gly Asp Ala Phe Val Ala Thr Ala Thr Ala
        275                 280                 285

aca gct agc gca gag cag gag ggt gcc agg cag ctg atc tgc aac gtc     912
Thr Ala Ser Ala Glu Gln Glu Gly Ala Arg Gln Leu Ile Cys Asn Val
        290                 295                 300

acc ctg ggg ggc gaa aac cgg gag acc cgg gag aac gtg acc atc tac     960
```

```
Thr Leu Gly Gly Glu Asn Arg Glu Thr Arg Glu Asn Val Thr Ile Tyr
305                 310             315             320
agc ttc ccg gca cca ctc ctg acc ctg agc gaa ccc agc gtc tcc gag      1008
Ser Phe Pro Ala Pro Leu Leu Thr Leu Ser Glu Pro Ser Val Ser Glu
                325             330             335
ggg cag atg gtg aca gta acc tgc gca gct ggg acc caa gct ctg gtc      1056
Gly Gln Met Val Thr Val Thr Cys Ala Ala Gly Thr Gln Ala Leu Val
                340             345             350
aca ctg gag gga gtt cca gcc gcg gtc ccg ggg cag ccc gcc cag ctt      1104
Thr Leu Glu Gly Val Pro Ala Ala Val Pro Gly Gln Pro Ala Gln Leu
            355             360             365
cag cta aat gcc acc gag aac gac gac aga cgc agc ttc ttc tgc gac      1152
Gln Leu Asn Ala Thr Glu Asn Asp Asp Arg Arg Ser Phe Phe Cys Asp
        370             375             380
gcc acc ctc gat gtg gac ggg gag acc ctg atc aag aac agg agc gca      1200
Ala Thr Leu Asp Val Asp Gly Glu Thr Leu Ile Lys Asn Arg Ser Ala
385             390             395             400
gag ctt cgt gtc cta tac gct ccc cgg cta gac gat tcg gac tgc ccc      1248
Glu Leu Arg Val Leu Tyr Ala Pro Arg Leu Asp Asp Ser Asp Cys Pro
            405             410             415
agg agt tgg acg tgg ccc gag ggc cca gag cag acg ctg cgc tgc gag      1296
Arg Ser Trp Thr Trp Pro Glu Gly Pro Glu Gln Thr Leu Arg Cys Glu
            420             425             430
gcc cgc ggg aac cca gaa ccc tca gtg cac tgt gcg cgc tcc gac ggc      1344
Ala Arg Gly Asn Pro Glu Pro Ser Val His Cys Ala Arg Ser Asp Gly
            435             440             445
ggg gcc gtg ctg gct ctg ggc ctg ctg ggt cca gtc act cgg gcg ctc      1392
Gly Ala Val Leu Ala Leu Gly Leu Leu Gly Pro Val Thr Arg Ala Leu
            450             455             460
tca ggc act tac cgc tgc aag gcg gcc aat gat caa ggc gag gcg gtc      1440
Ser Gly Thr Tyr Arg Cys Lys Ala Ala Asn Asp Gln Gly Glu Ala Val
465             470             475             480
aag gac gta acg cta acg gtg gag tac gca cca gcg ctg gac agc gtg      1488
Lys Asp Val Thr Leu Thr Val Glu Tyr Ala Pro Ala Leu Asp Ser Val
            485             490             495
ggc tgc cca gaa cgc att act tgg ctg gag gga aca gaa gcc tcg ctg      1536
Gly Cys Pro Glu Arg Ile Thr Trp Leu Glu Gly Thr Glu Ala Ser Leu
            500             505             510
agc tgt gtg gcg cac ggg gta ccg ccg cct gat gtg atc tgc gtg cgc      1584
Ser Cys Val Ala His Gly Val Pro Pro Pro Asp Val Ile Cys Val Arg
            515             520             525
tct gga gaa ctc ggg gcc gtc atc gag ggg ctg ttg cgt gtg gcc cgg      1632
Ser Gly Glu Leu Gly Ala Val Ile Glu Gly Leu Leu Arg Val Ala Arg
            530             535             540
gag cat gcg ggc act tac cgc tgc gaa gcc acc aac cct cgg ggc tct      1680
Glu His Ala Gly Thr Tyr Arg Cys Glu Ala Thr Asn Pro Arg Gly Ser
545             550             555             560
gcg gcc aaa aat gtg gcc gtc acg gtg gaa tat ggc ccc agg ttt gag      1728
Ala Ala Lys Asn Val Ala Val Thr Val Glu Tyr Gly Pro Arg Phe Glu
            565             570             575
gag ccg agc tgc ccc agc aat tgg aca tgg gtg gaa gga tct ggg cgc      1776
Glu Pro Ser Cys Pro Ser Asn Trp Thr Trp Val Glu Gly Ser Gly Arg
            580             585             590
ctg ttt tcc tgt gag gtc gat ggg aag cca cag cca agc gtg aag tgc      1824
Leu Phe Ser Cys Glu Val Asp Gly Lys Pro Gln Pro Ser Val Lys Cys
            595             600             605
gtg ggc tcc ggg ggc gcc act gag ggg gtg ctg ctg ccg ctg gca ccc      1872
Val Gly Ser Gly Gly Ala Thr Glu Gly Val Leu Leu Pro Leu Ala Pro
            610             615             620
cca gac cct agt ccc aga gct ccc aga atc cct aga gtc ctg gca ccc      1920
Pro Asp Pro Ser Pro Arg Ala Pro Arg Ile Pro Arg Val Leu Ala Pro
625             630             635             640
ggt atc tac gtc tgc aac gcc acc aac cgc cac ggc tcc gtg gcc aaa      1968
Gly Ile Tyr Val Cys Asn Ala Thr Asn Arg His Gly Ser Val Ala Lys
            645             650             655
```

```
aca gtc gtc gtg agc gcg gag tcg cca ccg gag atg gat gaa tct acc      2016
Thr Val Val Val Ser Ala Glu Ser Pro Pro Glu Met Asp Glu Ser Thr
            660             665                 670
tgc cca agt cac cag acg tgg ctg gaa ggg gct gag gct tcc gcg ctg      2064
Cys Pro Ser His Gln Thr Trp Leu Glu Gly Ala Glu Ala Ser Ala Leu
            675             680                 685
gcc tgc gcc gcc cgg ggt cgc cct tcc cca gga gtg cgc tgc tct cgg      2112
Ala Cys Ala Ala Arg Gly Arg Pro Ser Pro Gly Val Arg Cys Ser Arg
            690             695                 700
gaa ggc atc cca tgg cct gag cag cag cgc gtg tcc cga gag gac gcg      2160
Glu Gly Ile Pro Trp Pro Glu Gln Gln Arg Val Ser Arg Glu Asp Ala
705             710                 715                 720
ggc act tac cac tgt gtg gcc acc aat gcg cat ggc acg gac tcc cgg      2208
Gly Thr Tyr His Cys Val Ala Thr Asn Ala His Gly Thr Asp Ser Arg
            725             730                 735
acc gtc act gtg ggc gtg gaa tac cgg cca gtg gtg gcc gaa ctt gct      2256
Thr Val Thr Val Gly Val Glu Tyr Arg Pro Val Val Ala Glu Leu Ala
            740             745                 750
gcc tcg ccc cct gga ggc gtg cgc cca gga gga aac ttc acg ttg acc      2304
Ala Ser Pro Pro Gly Gly Val Arg Pro Gly Gly Asn Phe Thr Leu Thr
            755             760                 765
tgc cgc gcg gag gcc tgg cct cca gcc cag atc agc tgg cgc gcg ccc      2352
Cys Arg Ala Glu Ala Trp Pro Pro Ala Gln Ile Ser Trp Arg Ala Pro
            770             775                 780
ccg ggg gcc ctc aac atc ggc ctg tcg agc aac aac agc aca ctg agc      2400
Pro Gly Ala Leu Asn Ile Gly Leu Ser Ser Asn Asn Ser Thr Leu Ser
785             790                 795                 800
gtg gca ggc gcc atg gga agc cac ggc ggc gag tac gag tgc gca cgc      2448
Val Ala Gly Ala Met Gly Ser His Gly Gly Glu Tyr Glu Cys Ala Arg
            805             810                 815
acc aac gcg cac ggg cgc cac gcg cgg cgc atc acg gtg cgc gtg gcc      2496
Thr Asn Ala His Gly Arg His Ala Arg Arg Ile Thr Val Arg Val Ala
            820             825                 830
ggt ccg tgg cta tgg gtc gcc gtg ggc ggc gcg gcg ggg ggc gcg gcg      2544
Gly Pro Trp Leu Trp Val Ala Val Gly Gly Ala Ala Gly Gly Ala Ala
            835             840                 845
ctg ctg gcc gcg ggg gcc ggc ctg gcc ttc tac gtg cag tcc acc gcc      2592
Leu Leu Ala Ala Gly Ala Gly Leu Ala Phe Tyr Val Gln Ser Thr Ala
            850             855                 860
tgc aag aag ggc gag tac aac gtg cag gag gcc gag agc tca ggc gag      2640
Cys Lys Lys Gly Glu Tyr Asn Val Gln Glu Ala Glu Ser Ser Gly Glu
865             870                 875                 880
gcc gtg tgt ctc aac gga gcg ggc ggc ggc gct ggc ggg gcg gca ggc      2688
Ala Val Cys Leu Asn Gly Ala Gly Gly Gly Ala Gly Gly Ala Ala Gly
            885             890                 895
gcg gag ggc gga ccc gag gcg gcg ggg ggc gcg gcc gag tcg ccg gcg      2736
Ala Glu Gly Gly Pro Glu Ala Ala Gly Gly Ala Ala Glu Ser Pro Ala
            900             905                 910
gag ggc gag gtc ttc gcc ata cag ctg aca tcg gcg tga                  2775
Glu Gly Glu Val Phe Ala Ile Gln Leu Thr Ser Ala
            915             920
```

```
<210>  6
<211>  924
<212>  PRT
<213>  Homo sapiens
<400>  6
Met Pro Gly Pro Ser Pro Gly Leu Arg Arg Ala Leu Leu Gly Leu Trp
1               5                   10                  15
Ala Ala Leu Gly Leu Gly Leu Phe Gly Leu Ser Ala Val Ser Gln Glu
                20                  25                  30
Pro Phe Trp Ala Asp Leu Gln Pro Arg Val Ala Phe Val Glu Arg Gly
                35                  40                  45
Gly Ser Leu Trp Leu Asn Cys Ser Thr Asn Cys Pro Arg Pro Glu Arg
```

```
                 50                    55                    60
      Gly Gly Leu Glu Thr Ser Leu Arg Arg Asn Gly Thr Gln Arg Gly Leu
      65                    70                    75                    80
      Arg Trp Leu Ala Arg Gln Leu Val Asp Ile Arg Glu Pro Glu Thr Gln
                          85                    90                    95
      Pro Val Cys Phe Phe Arg Cys Ala Arg Arg Thr Leu Gln Ala Arg Gly
                    100                   105                   110
      Leu Ile Arg Thr Phe Gln Arg Pro Asp Arg Val Glu Leu Met Pro Leu
                115                   120                   125
      Pro Pro Trp Gln Pro Val Gly Glu Asn Phe Thr Leu Ser Cys Arg Val
            130                   135                   140
      Pro Gly Ala Gly Pro Arg Ala Ser Leu Thr Leu Thr Leu Leu Arg Gly
      145                   150                   155                   160
      Ala Gln Glu Leu Ile Arg Arg Ser Phe Ala Gly Glu Pro Pro Arg Ala
                          165                   170                   175
      Arg Gly Ala Val Leu Thr Ala Thr Val Leu Ala Arg Arg Glu Asp His
                    180                   185                   190
      Gly Ala Asn Phe Ser Cys Arg Ala Glu Leu Asp Leu Arg Pro His Gly
                    195                   200                   205
      Leu Gly Leu Phe Glu Asn Ser Ser Ala Pro Arg Glu Leu Arg Thr Phe
            210                   215                   220
      Ser Leu Ser Pro Asp Ala Pro Arg Leu Ala Ala Pro Arg Leu Leu Glu
      225                   230                   235                   240
      Val Gly Ser Glu Arg Pro Val Ser Cys Thr Leu Asp Gly Leu Phe Pro
                    245                   250                   255
      Ala Ser Glu Ala Arg Val Tyr Leu Ala Leu Gly Asp Gln Asn Leu Ser
                    260                   265                   270
      Pro Asp Val Thr Leu Glu Gly Asp Ala Phe Val Ala Thr Ala Thr Ala
                275                   280                   285
      Thr Ala Ser Ala Glu Gln Glu Gly Ala Arg Gln Leu Ile Cys Asn Val
      290                   295                   300
      Thr Leu Gly Gly Glu Asn Arg Glu Thr Arg Glu Asn Val Thr Ile Tyr
      305                   310                   315                   320
      Ser Phe Pro Ala Pro Leu Leu Thr Leu Ser Glu Pro Ser Val Ser Glu
                    325                   330                   335
      Gly Gln Met Val Thr Val Thr Cys Ala Ala Gly Thr Gln Ala Leu Val
                340                   345                   350
      Thr Leu Glu Gly Val Pro Ala Ala Val Pro Gly Gln Pro Ala Gln Leu
                355                   360                   365
      Gln Leu Asn Ala Thr Glu Asn Asp Asp Arg Arg Ser Phe Phe Cys Asp
            370                   375                   380
      Ala Thr Leu Asp Val Asp Gly Glu Thr Leu Ile Lys Asn Arg Ser Ala
      385                   390                   395                   400
      Glu Leu Arg Val Leu Tyr Ala Pro Arg Leu Asp Asp Ser Asp Cys Pro
                    405                   410                   415
      Arg Ser Trp Thr Trp Pro Glu Gly Pro Glu Gln Thr Leu Arg Cys Glu
                420                   425                   430
      Ala Arg Gly Asn Pro Glu Pro Ser Val His Cys Ala Arg Ser Asp Gly
                435                   440                   445
      Gly Ala Val Leu Ala Leu Gly Leu Leu Gly Pro Val Thr Arg Ala Leu
            450                   455                   460
      Ser Gly Thr Tyr Arg Cys Lys Ala Ala Asn Asp Gln Gly Glu Ala Val
      465                   470                   475                   480
      Lys Asp Val Thr Leu Thr Val Glu Tyr Ala Pro Ala Leu Asp Ser Val
                    485                   490                   495
      Gly Cys Pro Glu Arg Ile Thr Trp Leu Glu Gly Thr Glu Ala Ser Leu
                500                   505                   510
      Ser Cys Val Ala His Gly Val Pro Pro Pro Asp Val Ile Cys Val Arg
            515                   520                   525
      Ser Gly Glu Leu Gly Ala Val Ile Glu Gly Leu Leu Arg Val Ala Arg
            530                   535                   540
      Glu His Ala Gly Thr Tyr Arg Cys Glu Ala Thr Asn Pro Arg Gly Ser
      545                   550                   555                   560
      Ala Ala Lys Asn Val Ala Val Thr Val Glu Tyr Gly Pro Arg Phe Glu
                    565                   570                   575
```

```
Glu Pro Ser Cys Pro Ser Asn Trp Thr Trp Val Glu Gly Ser Gly Arg
            580                 585                 590
Leu Phe Ser Cys Glu Val Asp Gly Lys Pro Gln Pro Ser Val Lys Cys
            595                 600                 605
Val Gly Ser Gly Gly Ala Thr Glu Gly Val Leu Leu Pro Leu Ala Pro
            610                 615                 620
Pro Asp Pro Ser Pro Arg Ala Pro Arg Ile Pro Arg Val Leu Ala Pro
625                     630                 635                 640
Gly Ile Tyr Val Cys Asn Ala Thr Asn Arg His Gly Ser Val Ala Lys
            645                 650                 655
Thr Val Val Val Ser Ala Glu Ser Pro Pro Glu Met Asp Glu Ser Thr
            660                 665                 670
Cys Pro Ser His Gln Thr Trp Leu Glu Gly Ala Glu Ala Ser Ala Leu
            675                 680                 685
Ala Cys Ala Ala Arg Gly Arg Pro Ser Pro Gly Val Arg Cys Ser Arg
            690                 695                 700
Glu Gly Ile Pro Trp Pro Glu Gln Gln Arg Val Ser Arg Glu Asp Ala
705                     710                 715                 720
Gly Thr Tyr His Cys Val Ala Thr Asn Ala His Gly Thr Asp Ser Arg
            725                 730                 735
Thr Val Thr Val Gly Val Glu Tyr Arg Pro Val Val Ala Glu Leu Ala
            740                 745                 750
Ala Ser Pro Pro Gly Gly Val Arg Pro Gly Gly Asn Phe Thr Leu Thr
            755                 760                 765
Cys Arg Ala Glu Ala Trp Pro Pro Ala Gln Ile Ser Trp Arg Ala Pro
770                     775                 780
Pro Gly Ala Leu Asn Ile Gly Leu Ser Ser Asn Asn Ser Thr Leu Ser
785                     790                 795                 800
Val Ala Gly Ala Met Gly Ser His Gly Gly Glu Tyr Glu Cys Ala Arg
            805                 810                 815
Thr Asn Ala His Gly Arg His Ala Arg Ile Thr Val Arg Val Ala
            820                 825                 830
Gly Pro Trp Leu Trp Val Ala Val Gly Gly Ala Ala Gly Gly Ala Ala
            835                 840                 845
Leu Leu Ala Ala Gly Ala Gly Leu Ala Phe Tyr Val Gln Ser Thr Ala
850                     855                 860
Cys Lys Lys Gly Glu Tyr Asn Val Gln Glu Ala Glu Ser Ser Gly Glu
865                     870                 875                 880
Ala Val Cys Leu Asn Gly Ala Gly Gly Gly Ala Gly Gly Ala Ala Gly
            885                 890                 895
Ala Glu Gly Gly Pro Glu Ala Ala Gly Gly Ala Ala Glu Ser Pro Ala
            900                 905                 910
Glu Gly Glu Val Phe Ala Ile Gln Leu Thr Ser Ala
            915                 920
```

```
<210>  7
<211>  411
<212>  DNA
<213>  Mus musculus
<220>
<221>  V_region
<222>  (1)..(411)
<220>
<221>  CDS
<222>  (1)..(411)
<400>  7
atg gaa agg cac tgg atc ttt ctc ttc ctg ttt tca gta act gca ggt      48
Met Glu Arg His Trp Ile Phe Leu Phe Leu Phe Ser Val Thr Ala Gly
1               5                   10                  15
gtc cac tcc cag gtc cag ctt cag cag tct ggg gct gaa ctg gca aaa      96
Val His Ser Gln Val Gln Leu Gln Gln Ser Gly Ala Glu Leu Ala Lys
                20                  25                  30
cct ggg gcc tca gtg aag atg tcc tgc aag gct tct ggc tac acc ttt     144
Pro Gly Ala Ser Val Lys Met Ser Cys Lys Ala Ser Gly Tyr Thr Phe
```

```
              35                    40                    45
act acc tac tgg atg cac tgg gta aaa cag agg cct gga cag ggt ctg    192
Thr Thr Tyr Trp Met His Trp Val Lys Gln Arg Pro Gly Gln Gly Leu
        50                    55                    60
gaa tgg att gga tac att aat cct aac act gat tat act gaa tac aat    240
Glu Trp Ile Gly Tyr Ile Asn Pro Asn Thr Asp Tyr Thr Glu Tyr Asn
65                    70                    75                    80
cag aag ttc aag gac aag gcc aca ttg act gca gac aag tcc tcc agc    288
Gln Lys Phe Lys Asp Lys Ala Thr Leu Thr Ala Asp Lys Ser Ser Ser
                85                    90                    95
aca gcc ttc atg caa ctg agc agc ctg aca tct gag gac tct gca gtc    336
Thr Ala Phe Met Gln Leu Ser Ser Leu Thr Ser Glu Asp Ser Ala Val
                100                   105                   110
tat tac tgt gca agc tcg agg gat gct tac cac ggg act tac tgg ggc    384
Tyr Tyr Cys Ala Ser Ser Arg Asp Ala Tyr His Gly Thr Tyr Trp Gly
                115                   120                   125
caa gga act ctg gtc act gtc tct gca                                411
Gln Gly Thr Leu Val Thr Val Ser Ala
        130                   135
```

```
<210>  8
<211>  137
<212>  PRT
<213>  Mus musculus
<400>  8
Met Glu Arg His Trp Ile Phe Leu Phe Leu Phe Ser Val Thr Ala Gly
1               5                   10                  15
Val His Ser Gln Val Gln Leu Gln Gln Ser Gly Ala Glu Leu Ala Lys
            20                  25                  30
Pro Gly Ala Ser Val Lys Met Ser Cys Lys Ala Ser Gly Tyr Thr Phe
        35                  40                  45
Thr Thr Tyr Trp Met His Trp Val Lys Gln Arg Pro Gly Gln Gly Leu
        50                  55                  60
Glu Trp Ile Gly Tyr Ile Asn Pro Asn Thr Asp Tyr Thr Glu Tyr Asn
65                  70                  75                  80
Gln Lys Phe Lys Asp Lys Ala Thr Leu Thr Ala Asp Lys Ser Ser Ser
                85                  90                  95
Thr Ala Phe Met Gln Leu Ser Ser Leu Thr Ser Glu Asp Ser Ala Val
                100                 105                 110
Tyr Tyr Cys Ala Ser Ser Arg Asp Ala Tyr His Gly Thr Tyr Trp Gly
                115                 120                 125
Gln Gly Thr Leu Val Thr Val Ser Ala
        130                 135
```

```
<210>  9
<211>  381
<212>  DNA
<213>  Mus musculus
<220>
<221>  V_region
<222>  (1)..(411)
<220>
<221>  CDS
<222>  (1)..(381)
<400>  9
atg gtg tcc act tct cag ctc ctt gga ctt ttg ctt ttc tgg act tca    48
Met Val Ser Thr Ser Gln Leu Leu Gly Leu Leu Leu Phe Trp Thr Ser
1               5                   10                  15
gcc tcc aga tgt gac att gtg atg act cag tct cca gcc acc ctg tct    96
Ala Ser Arg Cys Asp Ile Val Met Thr Gln Ser Pro Ala Thr Leu Ser
                20                  25                  30
gtg act cca gga gat aga gtc tct ctt tcc tgc agg gcc agt cag agt    144
Val Thr Pro Gly Asp Arg Val Ser Leu Ser Cys Arg Ala Ser Gln Ser
```

```
                35                40                45
att agc gac tac tta cac tgg tat caa cag aaa tca cat gag tct cca        192
Ile Ser Asp Tyr Leu His Trp Tyr Gln Gln Lys Ser His Glu Ser Pro
    50                55                60
agg ctt ctc atc aga tat gct tcc caa tcc atc tct ggg atc ccc tcc        240
Arg Leu Leu Ile Arg Tyr Ala Ser Gln Ser Ile Ser Gly Ile Pro Ser
65                70                75                80
agg ttc agt ggc agt gga tca ggg tca gat ttc act ctc agt atc aac        288
Arg Phe Ser Gly Ser Gly Ser Gly Ser Asp Phe Thr Leu Ser Ile Asn
                85                90                95
aat gta gaa cct gaa gat gtt gga gtg tat tac tgt caa aat ggt cac        336
Asn Val Glu Pro Glu Asp Val Gly Val Tyr Tyr Cys Gln Asn Gly His
            100               105               110

aac ttt ccg ctc acg ttc ggt gct ggg acc aag ctg gag ctg aaa           381
Asn Phe Pro Leu Thr Phe Gly Ala Gly Thr Lys Leu Glu Leu Lys
            115               120               125


<210>   10
<211>   127
<212>   PRT
<213>   Mus musculus
<400>   10
Met Val Ser Thr Ser Gln Leu Leu Gly Leu Leu Leu Phe Trp Thr Ser
1               5                 10                15
Ala Ser Arg Cys Asp Ile Val Met Thr Gln Ser Pro Ala Thr Leu Ser
                20                25                30
Val Thr Pro Gly Asp Arg Val Ser Leu Ser Cys Arg Ala Ser Gln Ser
                35                40                45
Ile Ser Asp Tyr Leu His Trp Tyr Gln Gln Lys Ser His Glu Ser Pro
    50                55                60
Arg Leu Leu Ile Arg Tyr Ala Ser Gln Ser Ile Ser Gly Ile Pro Ser
65                70                75                80
Arg Phe Ser Gly Ser Gly Ser Gly Ser Asp Phe Thr Leu Ser Ile Asn
                85                90                95
Asn Val Glu Pro Glu Asp Val Gly Val Tyr Tyr Cys Gln Asn Gly His
            100               105               110
Asn Phe Pro Leu Thr Phe Gly Ala Gly Thr Lys Leu Glu Leu Lys
            115               120               125


<210>   11
<211>   30
<212>   PRT
<213>   Mus musculus
<220>
<221>   MISC_FEATURE
<222>   (1)..(30)
<223>   IB23_VH_FR1
<400>   11
Gln Val Gln Leu Gln Gln Ser Gly Ala Glu Leu Ala Lys Pro Gly Ala
1               5                 10                15
Ser Val Lys Met Ser Cys Lys Ala Ser Gly Tyr Thr Phe Thr
                20                25                30


<210>   12
<211>   14
<212>   PRT
<213>   Mus musculus
<220>
<221>   MISC_FEATURE
<222>   (1)..(14)
<223>   IB23_VH_FR2
```

```
<400>  12
Trp Val Lys Gln Arg Pro Gly Gln Gly Leu Glu Trp Ile Gly
1               5                   10


<210>  13
<211>  32
<212>  PRT
<213>  Mus musculus
<220>
<221>  MISC_FEATURE
<222>  (1)..(32)
<223>  IB23_VH_FR3
<400>  13
Lys Ala Thr Leu Thr Ala Asp Lys Ser Ser Ser Thr Ala Phe Met Gln
1               5                   10                  15
Leu Ser Ser Leu Thr Ser Glu Asp Ser Ala Val Tyr Tyr Cys Ala Ser
            20                  25                  30


<210>  14
<211>  11
<212>  PRT
<213>  Mus musculus
<220>
<221>  MISC_FEATURE
<222>  (1)..(11)
<223>  IB23_VH_FR4
<400>  14
Trp Gly Gln Gly Thr Leu Val Thr Val Ser Ala
1               5                   10


<210>  15
<211>  5
<212>  PRT
<213>  Mus musculus
<220>
<221>  MISC_FEATURE
<222>  (1)..(5)
<223>  IB23_VH_CDR1
<400>  15
Thr Tyr Trp Met His
1               5


<210>  16
<211>  17
<212>  PRT
<213>  Mus musculus
<220>
<221>  MISC_FEATURE
<222>  (1)..(17)
<223>  IB23_VH_CDR2
<400>  16
Tyr Ile Asn Pro Asn Thr Asp Tyr Thr Glu Tyr Asn Gln Lys Phe Lys
1               5                   10                  15
Asp


<210>  17
<211>  9
<212>  PRT
<213>  Mus musculus
```

```
<220>
<221>  MISC_FEATURE
<222>  (1)..(9)
<223>  IB23_VH_CDR3
<400>  17
Ser Arg Asp Ala Tyr His Gly Thr Tyr
1               5


<210>  18
<211>  23
<212>  PRT
<213>  Mus musculus
<220>
<221>  MISC_FEATURE
<222>  (1)..(23)
<223>  IB23_VL_FR1
<400>  18

Asp Ile Val Met Thr Gln Ser Pro Ala Thr Leu Ser Val Thr Pro Gly
1               5                   10                  15
Asp Arg Val Ser Leu Ser Cys
                20


<210>  19
<211>  15
<212>  PRT
<213>  Mus musculus
<220>
<221>  MISC_FEATURE
<222>  (1)..(15)
<223>  IB23_VL_FR2
<400>  19
Trp Tyr Gln Gln Lys Ser His Glu Ser Pro Arg Leu Leu Ile Arg
1               5                   10                  15


<210>  20
<211>  32
<212>  PRT
<213>  Mus musculus
<220>
<221>  MISC_FEATURE
<222>  (1)..(32)
<223>  IB23_VL_FR3
<400>  20
Gly Ile Pro Ser Arg Phe Ser Gly Ser Gly Ser Gly Ser Asp Phe Thr
1               5                   10                  15
Leu Ser Ile Asn Asn Val Glu Pro Glu Asp Val Gly Val Tyr Tyr Cys
                20                  25                  30


<210>  21
<211>  10
<212>  PRT
<213>  Mus musculus
<220>
<221>  MISC_FEATURE
<222>  (1)..(10)
<223>  IB23_VL_FR4
<400>  21
Phe Gly Ala Gly Thr Lys Leu Glu Leu Lys
1               5                   10
```

```
<210>    22
<211>    11
<212>    PRT
<213>    Mus musculus
<220>
<221>    MISC_FEATURE
<222>    (1)..(11)
<223>    IB23_VL_CDR1
<400>    22
Arg Ala Ser Gln Ser Ile Ser Asp Tyr Leu His
1               5                   10


<210>    23
<211>    7
<212>    PRT
<213>    Mus musculus
<220>
<221>    MISC_FEATURE
<222>    (1)..(7)
<223>    IB23_VL_CDR2
<400>    23
Tyr Ala Ser Gln Ser Ile Ser
1               5


<210>    24
<211>    9
<212>    PRT
<213>    Mus musculus
<220>
<221>    MISC_FEATURE
<222>    (1)..(9)
<223>    IB23_VL_CDR3
<400>    24
Gln Asn Gly His Asn Phe Pro Leu Thr
1               5


<210>    25
<211>    996
<212>    DNA
<213>    Homo sapiens
<220>
<221>    C_region
<222>    (1)..(996)
<220>
<221>    CDS
<222>    (1)..(990)
<400>    25
gct agc acc aag ggc cca tcg gtc ttc ccc ctg gca ccc tcc tcc aag       48
Ala Ser Thr Lys Gly Pro Ser Val Phe Pro Leu Ala Pro Ser Ser Lys
1               5                   10                  15
agc acc tct ggg ggc aca gcg gcc ctg ggc tgc ctg gtc aag gac tac       96
Ser Thr Ser Gly Gly Thr Ala Ala Leu Gly Cys Leu Val Lys Asp Tyr
                20                  25                  30
ttc ccc gaa ccg gtg acg gtg tcg tgg aac tca ggc gcc ctg acc agc      144
Phe Pro Glu Pro Val Thr Val Ser Trp Asn Ser Gly Ala Leu Thr Ser
                35                  40                  45
ggc gtg cac acc ttc ccg gct gtc cta cag tcc tca gga ctc tac tcc      192
Gly Val His Thr Phe Pro Ala Val Leu Gln Ser Ser Gly Leu Tyr Ser
        50                  55                  60
ctc agc agc gtg gtg acc gtg ccc tcc agc agc ttg ggc acc cag acc      240
Leu Ser Ser Val Val Thr Val Pro Ser Ser Ser Leu Gly Thr Gln Thr
```

```
            65                      70                      75                      80
tac atc tgc aac gtg aat cac aag ccc agc aac acc aag gtg gac aag     288
Tyr Ile Cys Asn Val Asn His Lys Pro Ser Asn Thr Lys Val Asp Lys
                    85                      90                      95
aaa gtt gag ccc aaa tct tgt gac aaa act cac aca tgc cca ccg tgc     336
Lys Val Glu Pro Lys Ser Cys Asp Lys Thr His Thr Cys Pro Pro Cys
                100                     105                     110
cca gca cct gaa ctc ctg ggg gga ccg tca gtc ttc ctc ttc ccc cca     384
Pro Ala Pro Glu Leu Leu Gly Gly Pro Ser Val Phe Leu Phe Pro Pro
                115                     120                     125
aaa ccc aag gac acc ctc atg atc tcc cgg acc cct gag gtc aca tgc     432
Lys Pro Lys Asp Thr Leu Met Ile Ser Arg Thr Pro Glu Val Thr Cys
            130                     135                     140
gtg gtg gtg gac gtg agc cac gaa gac cct gag gtc aag ttc aac tgg     480
Val Val Val Asp Val Ser His Glu Asp Pro Glu Val Lys Phe Asn Trp
145                     150                     155                     160
tac gtg gac ggc gtg gag gtg cat aat gcc aag aca aag ccg cgg gag     528
Tyr Val Asp Gly Val Glu Val His Asn Ala Lys Thr Lys Pro Arg Glu
                    165                     170                     175
gag cag tac aac agc acg tac cgt gtg gtc agc gtc ctc acc gtc ctg     576
Glu Gln Tyr Asn Ser Thr Tyr Arg Val Val Ser Val Leu Thr Val Leu
                180                     185                     190
cac cag gac tgg ctg aat ggc aag gag tac aag tgc aag gtc tcc aac     624
His Gln Asp Trp Leu Asn Gly Lys Glu Tyr Lys Cys Lys Val Ser Asn
                195                     200                     205
aaa gcc ctc cca gcc ccc atc gag aaa acc atc tcc aaa gcc aaa ggg     672
Lys Ala Leu Pro Ala Pro Ile Glu Lys Thr Ile Ser Lys Ala Lys Gly
            210                     215                     220
cag ccc cga gaa cca cag gtg tac acc ctg ccc cca tcc cgg gat gag     720
Gln Pro Arg Glu Pro Gln Val Tyr Thr Leu Pro Pro Ser Arg Asp Glu
225                     230                     235                     240
ctg acc aag aac cag gtc agc ctg acc tgc ctg gtc aaa ggc ttc tat     768
Leu Thr Lys Asn Gln Val Ser Leu Thr Cys Leu Val Lys Gly Phe Tyr
                    245                     250                     255
ccc agc gac atc gcc gtg gag tgg gag agc aat ggg cag ccg gag aac     816
Pro Ser Asp Ile Ala Val Glu Trp Glu Ser Asn Gly Gln Pro Glu Asn
                260                     265                     270
aac tac aag acc acg cct ccc gtg ctg gac tcc gac ggc tcc ttc ttc     864
Asn Tyr Lys Thr Thr Pro Pro Val Leu Asp Ser Asp Gly Ser Phe Phe
                275                     280                     285
ctc tac agc aag ctc acc gtg gac aag agc agg tgg cag cag ggg aac     912
Leu Tyr Ser Lys Leu Thr Val Asp Lys Ser Arg Trp Gln Gln Gly Asn
            290                     295                     300
gtc ttc tca tgc tcc gtg atg cat gag gct ctg cac aac cac tac acg     960
Val Phe Ser Cys Ser Val Met His Glu Ala Leu His Asn His Tyr Thr
305                     310                     315                     320
cag aag agc ctc tcc ctg tct ccg ggt aaa tgataa                      996
Gln Lys Ser Leu Ser Leu Ser Pro Gly Lys
                325                     330
```

<210> 26
<211> 330
<212> PRT
<213> Homo sapiens
<400> 26

```
Ala Ser Thr Lys Gly Pro Ser Val Phe Pro Leu Ala Pro Ser Ser Lys
1               5                   10                  15
Ser Thr Ser Gly Gly Thr Ala Ala Leu Gly Cys Leu Val Lys Asp Tyr
            20                  25                  30
Phe Pro Glu Pro Val Thr Val Ser Trp Asn Ser Gly Ala Leu Thr Ser
            35                  40                  45
Gly Val His Thr Phe Pro Ala Val Leu Gln Ser Ser Gly Leu Tyr Ser
    50                  55                  60
Leu Ser Ser Val Val Thr Val Pro Ser Ser Ser Leu Gly Thr Gln Thr
```

```
            65                      70                      75                      80
Tyr Ile Cys Asn Val Asn His Lys Pro Ser Asn Thr Lys Val Asp Lys
                    85                      90                      95
Lys Val Glu Pro Lys Ser Cys Asp Lys Thr His Thr Cys Pro Pro Cys
                100                     105                     110
Pro Ala Pro Glu Leu Leu Gly Gly Pro Ser Val Phe Leu Phe Pro Pro
                115                     120                     125
Lys Pro Lys Asp Thr Leu Met Ile Ser Arg Thr Pro Glu Val Thr Cys
            130                     135                     140
Val Val Val Asp Val Ser His Glu Asp Pro Glu Val Lys Phe Asn Trp
145                     150                     155                     160
Tyr Val Asp Gly Val Glu Val His Asn Ala Lys Thr Lys Pro Arg Glu
                    165                     170                     175
Glu Gln Tyr Asn Ser Thr Tyr Arg Val Val Ser Val Leu Thr Val Leu
                180                     185                     190
His Gln Asp Trp Leu Asn Gly Lys Glu Tyr Lys Cys Lys Val Ser Asn
                195                     200                     205
Lys Ala Leu Pro Ala Pro Ile Glu Lys Thr Ile Ser Lys Ala Lys Gly
            210                     215                     220
Gln Pro Arg Glu Pro Gln Val Tyr Thr Leu Pro Pro Ser Arg Asp Glu
225                     230                     235                     240
Leu Thr Lys Asn Gln Val Ser Leu Thr Cys Leu Val Lys Gly Phe Tyr
                    245                     250                     255
Pro Ser Asp Ile Ala Val Glu Trp Glu Ser Asn Gly Gln Pro Glu Asn
                260                     265                     270
Asn Tyr Lys Thr Thr Pro Pro Val Leu Asp Ser Asp Gly Ser Phe Phe
                275                     280                     285
Leu Tyr Ser Lys Leu Thr Val Asp Lys Ser Arg Trp Gln Gln Gly Asn
            290                     295                     300
Val Phe Ser Cys Ser Val Met His Glu Ala Leu His Asn His Tyr Thr
305                     310                     315                     320
Gln Lys Ser Leu Ser Leu Ser Pro Gly Lys
                    325                     330
```

```
<210>  27
<211>  327
<212>  DNA
<213>  Homo sapiens
<220>
<221>  C_region
<222>  (1)..(327)
<220>
<221>  CDS
<222>  (1)..(321)
<400>  27
cgt acg gtg gct gca cca tct gtc ttc atc ttc ccg cca tct gat gag      48
Arg Thr Val Ala Ala Pro Ser Val Phe Ile Phe Pro Pro Ser Asp Glu
1               5                   10                  15
cag ttg aaa tct gga act gcc tct gtt gtg tgc ctg ctg aat aac ttc      96
Gln Leu Lys Ser Gly Thr Ala Ser Val Val Cys Leu Leu Asn Asn Phe
            20                  25                  30
tat ccc aga gag gcc aaa gta cag tgg aag gtg gat aac gcc ctc caa     144
Tyr Pro Arg Glu Ala Lys Val Gln Trp Lys Val Asp Asn Ala Leu Gln
        35                  40                  45
tcg ggt aac tcc cag gag agt gtc aca gag cag gac agc aag gac agc     192
Ser Gly Asn Ser Gln Glu Ser Val Thr Glu Gln Asp Ser Lys Asp Ser
    50                  55                  60
acc tac agc ctc agc agc acc ctg acg ctg agc aaa gca gac tac gag     240
Thr Tyr Ser Leu Ser Ser Thr Leu Thr Leu Ser Lys Ala Asp Tyr Glu
65                  70                  75                  80
aaa cac aaa gtc tac gcc tgc gaa gtc acc cat cag ggc ctg agc tcg     288
Lys His Lys Val Tyr Ala Cys Glu Val Thr His Gln Gly Leu Ser Ser
                85                  90                  95
ccc gtc aca aag agc ttc aac agg gga gag tgt tgataa                  327
Pro Val Thr Lys Ser Phe Asn Arg Gly Glu Cys
```

46

```
Pro Val Thr Lys Ser Phe Asn Arg Gly Glu Cys
        100                 105


<210>  28
<211>  107
<212>  PRT
<213>  Homo sapiens
<400>  28
Arg Thr Val Ala Ala Pro Ser Val Phe Ile Phe Pro Pro Ser Asp Glu
1               5                   10                  15
Gln Leu Lys Ser Gly Thr Ala Ser Val Val Cys Leu Leu Asn Asn Phe
            20                  25                  30
Tyr Pro Arg Glu Ala Lys Val Gln Trp Lys Val Asp Asn Ala Leu Gln
        35                  40                  45
Ser Gly Asn Ser Gln Glu Ser Val Thr Glu Gln Asp Ser Lys Asp Ser
    50                  55                  60
Thr Tyr Ser Leu Ser Ser Thr Leu Thr Leu Ser Lys Ala Asp Tyr Glu
65                  70                  75                  80
Lys His Lys Val Tyr Ala Cys Glu Val Thr His Gln Gly Leu Ser Ser
                85                  90                  95
Pro Val Thr Lys Ser Phe Asn Arg Gly Glu Cys
        100                 105


<210>  29
<211>  996
<212>  DNA
<213>  Homo sapiens
<220>
<221>  C_region
<222>  (1)..(996)
<220>
<221>  CDS
<222>  (1)..(990)
<400>  29
gct agc acc aag ggc cca tcg gtc ttc ccc ctg gca ccc tcc tcc aag      48
Ala Ser Thr Lys Gly Pro Ser Val Phe Pro Leu Ala Pro Ser Ser Lys
1               5                   10                  15
agc acc tct ggg ggc aca gcg gcc ctg ggc tgc ctg gtc aag gac tac      96
Ser Thr Ser Gly Gly Thr Ala Ala Leu Gly Cys Leu Val Lys Asp Tyr
            20                  25                  30
ttc ccc gaa ccg gtg acg gtg tcg tgg aac tca ggc gcc ctg acc agc     144
Phe Pro Glu Pro Val Thr Val Ser Trp Asn Ser Gly Ala Leu Thr Ser
            35                  40                  45
ggc gtg cac acc ttc ccg gct gtc cta cag tcc tca gga ctc tac tcc     192
Gly Val His Thr Phe Pro Ala Val Leu Gln Ser Ser Gly Leu Tyr Ser
    50                  55                  60
ctc agc agc gtg gtg acc gtg ccc tcc agc agc ttg ggc acc cag acc     240
Leu Ser Ser Val Val Thr Val Pro Ser Ser Ser Leu Gly Thr Gln Thr
65                  70                  75                  80
tac atc tgc aac gtg aat cac aag ccc agc aac acc aag gtg gac aag     288
Tyr Ile Cys Asn Val Asn His Lys Pro Ser Asn Thr Lys Val Asp Lys
                85                  90                  95
aaa gtt gag ccc aaa tct tgt gac aaa act cac aca tgc cca ccg tgc     336
Lys Val Glu Pro Lys Ser Cys Asp Lys Thr His Thr Cys Pro Pro Cys
            100                 105                 110
cca gca cct gaa ctc ctg ggg gga ccg tca gtc ttc ctc ttc ccc cca     384
Pro Ala Pro Glu Leu Leu Gly Gly Pro Ser Val Phe Leu Phe Pro Pro
            115                 120                 125
aaa ccc aag gac acc ctc atg atc tcc cgg acc cct gag gtc aca tgc     432
Lys Pro Lys Asp Thr Leu Met Ile Ser Arg Thr Pro Glu Val Thr Cys
            130                 135                 140
gtg gtg gtg gac gtg agc cac gaa gac cct gag gtc aag ttc aac tgg     480
Val Val Val Asp Val Ser His Glu Asp Pro Glu Val Lys Phe Asn Trp
```

```
145                    150                    155                    160
tac gtg gac ggc gtg gag gtg cat aat gcc aag aca aag ccg cgg gag     528
Tyr Val Asp Gly Val Glu Val His Asn Ala Lys Thr Lys Pro Arg Glu
            165                    170                    175
gag cag tac gcc agc acg tac cgt gtg gtc agc gtc ctc acc gtc ctg     576
Glu Gln Tyr Ala Ser Thr Tyr Arg Val Val Ser Val Leu Thr Val Leu
            180                    185                    190
cac cag gac tgg ctg aat ggc aag gag tac aag tgc aag gtc tcc aac     624
His Gln Asp Trp Leu Asn Gly Lys Glu Tyr Lys Cys Lys Val Ser Asn
            195                    200                    205
aaa gcc ctc cca gcc ccc atc gag aaa acc atc tcc aaa gcc aaa ggg     672
Lys Ala Leu Pro Ala Pro Ile Glu Lys Thr Ile Ser Lys Ala Lys Gly
    210                    215                    220
cag ccc cga gaa cca cag gtg tac acc ctg ccc cca tcc cgg gat gag     720
Gln Pro Arg Glu Pro Gln Val Tyr Thr Leu Pro Pro Ser Arg Asp Glu
225                    230                    235                    240
ctg acc aag aac cag gtc agc ctg acc tgc ctg gtc aaa ggc ttc tat     768
Leu Thr Lys Asn Gln Val Ser Leu Thr Cys Leu Val Lys Gly Phe Tyr
                245                    250                    255
ccc agc gac atc gcc gtg gag tgg gag agc aat ggg cag ccg gag aac     816
Pro Ser Asp Ile Ala Val Glu Trp Glu Ser Asn Gly Gln Pro Glu Asn
            260                    265                    270
aac tac aag acc acg cct ccc gtg ctg gac tcc gac ggc tcc ttc ttc     864
Asn Tyr Lys Thr Thr Pro Pro Val Leu Asp Ser Asp Gly Ser Phe Phe
            275                    280                    285
ctc tac agc aag ctc acc gtg gac aag agc agg tgg cag cag ggg aac     912
Leu Tyr Ser Lys Leu Thr Val Asp Lys Ser Arg Trp Gln Gln Gly Asn
    290                    295                    300
gtc ttc tca tgc tcc gtg atg cat gag gct ctg cac aac cac tac acg     960
Val Phe Ser Cys Ser Val Met His Glu Ala Leu His Asn His Tyr Thr
305                    310                    315                    320
cag aag agc ctc tcc ctg tct ccg ggt aaa tgataa                      996
Gln Lys Ser Leu Ser Leu Ser Pro Gly Lys
                325                    330
```

```
<210>   30
<211>   330
<212>   PRT
<213>   Homo sapiens
<400>   30
Ala Ser Thr Lys Gly Pro Ser Val Phe Pro Leu Ala Pro Ser Ser Lys
1               5                   10                  15
Ser Thr Ser Gly Gly Thr Ala Ala Leu Gly Cys Leu Val Lys Asp Tyr
            20                  25                  30
Phe Pro Glu Pro Val Thr Val Ser Trp Asn Ser Gly Ala Leu Thr Ser
            35                  40                  45
Gly Val His Thr Phe Pro Ala Val Leu Gln Ser Ser Gly Leu Tyr Ser
        50                  55                  60
Leu Ser Ser Val Val Thr Val Pro Ser Ser Ser Leu Gly Thr Gln Thr
65                  70                  75                  80
Tyr Ile Cys Asn Val Asn His Lys Pro Ser Asn Thr Lys Val Asp Lys
                85                  90                  95
Lys Val Glu Pro Lys Ser Cys Asp Lys Thr His Thr Cys Pro Pro Cys
            100                 105                 110
Pro Ala Pro Glu Leu Leu Gly Gly Pro Ser Val Phe Leu Phe Pro Pro
        115                 120                 125
Lys Pro Lys Asp Thr Leu Met Ile Ser Arg Thr Pro Glu Val Thr Cys
        130                 135                 140
Val Val Val Asp Val Ser His Glu Asp Pro Glu Val Lys Phe Asn Trp
145                 150                 155                 160
Tyr Val Asp Gly Val Glu Val His Asn Ala Lys Thr Lys Pro Arg Glu
                165                 170                 175
Glu Gln Tyr Ala Ser Thr Tyr Arg Val Val Ser Val Leu Thr Val Leu
            180                 185                 190
```

48

```
His Gln Asp Trp Leu Asn Gly Lys Glu Tyr Lys Cys Lys Val Ser Asn
        195                 200             205
Lys Ala Leu Pro Ala Pro Ile Glu Lys Thr Ile Ser Lys Ala Lys Gly
        210             215             220
Gln Pro Arg Glu Pro Gln Val Tyr Thr Leu Pro Pro Ser Arg Asp Glu
225             230             235             240
Leu Thr Lys Asn Gln Val Ser Leu Thr Cys Leu Val Lys Gly Phe Tyr
            245             250             255
Pro Ser Asp Ile Ala Val Glu Trp Glu Ser Asn Gly Gln Pro Glu Asn
        260             265             270
Asn Tyr Lys Thr Thr Pro Pro Val Leu Asp Ser Asp Gly Ser Phe Phe
        275             280             285
Leu Tyr Ser Lys Leu Thr Val Asp Lys Ser Arg Trp Gln Gln Gly Asn
    290             295             300
Val Phe Ser Cys Ser Val Met His Glu Ala Leu His Asn His Tyr Thr
305             310             315             320
Gln Lys Ser Leu Ser Leu Ser Pro Gly Lys
            325             330
```